(19) Europäisches Patentamt
European Patent Office
Office européen des brevets

(11) Numéro de publication : **0 349 424 B1**

(12) # FASCICULE DE BREVET EUROPEEN

(45) Date de publication du fascicule du brevet : **16.12.92 Bulletin 92/51**

(51) Int. Cl.[5] : **C07D 461/00,** A61K 31/435

(21) Numéro de dépôt : **89401850.6**

(22) Date de dépôt : **28.06.89**

(54) **Dérivés de la 20,21-dinoréburnaménine substitués en 15 par un groupement aminé et leurs sels, leur procédé de préparation et les intermédiaires ainsi obtenus, leur application comme médicaments et les compositions pharmaceutiques les renfermant.**

(30) Priorité : **28.06.88 FR 8808648**

(43) Date de publication de la demande : **03.01.90 Bulletin 90/01**

(45) Mention de la délivrance du brevet : **16.12.92 Bulletin 92/51**

(84) Etats contractants désignés : **AT BE CH DE ES FR GB GR IT LI LU NL SE**

(56) Documents cités :
**EP-A- 0 013 315**
**US-A- 4 123 535**
**US-A- 4 549 020**

(73) Titulaire : **ROUSSEL-UCLAF**
**35, Boulevard des Invalides**
**F-75007 Paris (FR)**

(72) Inventeur : **Clemence, François**
**2, rue Turgot**
**F-75009 Paris (FR)**
Inventeur : **Haesslein, Jean-Luc**
**18, Avenue du Président Kennedy**
**F-93110 Rosny sous Bois (FR)**
Inventeur : **Petit, Francis**
**119, Avenue Carnot**
**F-93140 Bondy (FR)**
Inventeur : **Degryse, Mauricette**
**9, rue Adélaide Lahaye**
**F-93170 Bagnolet (FR)**

(74) Mandataire : **Bourgouin, André et al**
**Département des Brevets ROUSSEL UCLAF**
**111, route de Noisy B.P. no 9**
**F-93230 Romainville (FR)**

**EP 0 349 424 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

L'invention concerne de nouveaux dérivés de la 20,21-dinorébumaménine substitués en 15 par un groupement aminé et leurs sels, leur procédé de préparation et les intermédiaires ainsi obtenus, leur application comme médicaments et les compositions pharmaceutiques les renfermant.

L'invention concerne des composés de formule (I) :

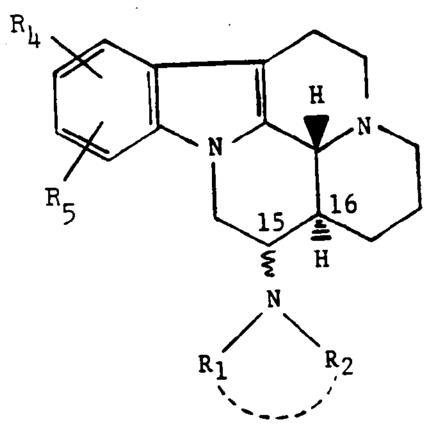

(I)

dans laquelle $R_1$ et $R_2$, identiques ou différents, représentent un atome d'hydrogène, un radical alkyle renfermant de 1 à 5 atomes de carbone, un radical $-(CH_2)_n-OH$, dans lequel n est compris entre 2 et 5, un radical aryle ou arylalkyle renfermant de 7 à 12 atomes de carbone, ces deux radicaux étant évent%uellement substitués par 1, 2 ou 3 radicaux choisis parmi le groupe formé par les atomes d'halogène, les radicaux méthyle, éthyle, méthoxy, éthoxy, trifluorométhyle, amino et nitro, un radical

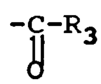

dans lequel $R_3$ est un radical alkyle renfermant de 1 à 5 atomes de carbone, un radical aryle ou arylalkyle renfermant de 7 à 12 atomes de carbone, ces deux radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, $R_1$ et $R_2$ ne pouvant toutefois pas représenter tous deux un radical aryle, ou $R_1$ et $R_2$ forment avec l'atome d'azote auquel ils sont liés un adical hétérocyclique saturé ou insaturé pouvant renfermer un second hétéroatome choisi parmi les atomes d'oxygène, de soufre et d'azote, ce dernier étant éventuellement substitué par un radical alkyle renfermant de 1 à 5 atomes de carbone, un radical aryle, un radical arylalkyle renfermant de 7 à 12 atomes de carbone, ces radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, $R_4$ et $R_5$, identiques ou différents, représentent un atome d'hydrogène, un atome d'halogène, un radical alkyle ou alcoxy renfermant de 1 à 5 atomes de carbone, un radical hydroxy, trifluorométhyle ou nitro, lesdits produits de formule (I) étant sous toutes les formes énantiomères et diastéréoisomères possibles et sous forme de sels d'addition avec les acides.

Le terme radical alkyle, représente de préférence, les radicaux méthyle, éthyle, n-propyle ou isopropyle, mais aussi radical n-butyle, isobutyle, n-pentyle. Le terme radical aryle désigne de préférence, un radical phényle ou naphtyle et le terme radical arylalkyle renfermant de 7 à 12 atomes de carbone désigne de préférence, un radical benzyle ou phénéthyle.

Lorsque $R_1$ et $R_2$ forment ensemble avec l'atome d'azote, un hétérocycle, il s'agit, par exemple, d'un cycle pyrrolidino, pipéridino, morpholino, pipérazinyle, méthylpipérazinyle, éthylpipérazinyle, propylpipérazinyle, phénylpipérazinyle, benzylpipérazinyle, lorsque $R_1$ et $R_2$ représentent ces deux derniers radicaux, les radicaux phényle et benzyle peuvent être éventuellement substitués par les substituants déjà mentionnés précédemment.

Lorsque $R_4$ et $R_5$ représentent un radical alkoxy, il s'agit de préférence d'un radical méthoxy ou éthoxy, mais ils peuvent aussi représenter un radical propoxy, isopropoxy, butoxy linéaire, secondaire ou tertiaire.

Lorsque $R_4$ et $R_5$ représentent un radical alkyle, il s'agit de préférence d'un radical méthyle ; il peut s'agir

aussi d'un radical éthyle, propyle, isopropyle, butyle linéaire ou ramifié.

Lorsque $R_4$ et $R_5$ représentent un atome d'halogène, il s'agit de préférence de l'atome de chlore, mais ils peuvent aussi représenter un atome de fluor, de brome ou d'iode.

Dans les produits de formule (I), l'atome d'azote lié au carbone en position 15 peut être sous la forme alpha ou béta.

L'invention concerne notamment les composés de formule (I) caractérisés en ce que $R_1$ et $R_2$, identiques ou différents, représentent un atome d'hydrogène, un radical méthyle ou éthyle, un radical

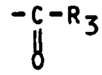

dans lequel $R_3$ est un radical méthyle ou éthyle, phényle, benzyle éventuellement substitué par un ou plusieurs atomes de chlore, un radical $-CH_2-CH_2-OH$ ou $R_1$ et $R_2$ forment avec l'atome d'azote un radical pyrrolidinyle, morpholinyle ou pipérazinyle dont l'atome d'azote est substitué par un radical méthyle ou phényle et les composés de formule (I) caractérisés en ce que $R_4$ et $R_5$ représentent un atome d'hydrogène ou $R_4$ représente un radical nitro en position 9 ou 11 et $R_5$ représente un atome d'hydrogène ainsi que leurs sels d'addition avec les acides.

L'invention concerne spécialement les produits dans lesquels $R_4$ et $R_5$ représentent chacun un atome d'hydrogène.

L'invention a tout particulièrement pour objet les produits décrits ci-après et spécialement :

- la [(±) (15alpha, 16alpha)] 14,15-dihydro N-méthyl 20,21- dinoréburnaménin-15-amine,
- la [(±) (15alpha, 16alpha)] 14,15-dihydro 20,21-dinoréburnaménin-15-amine,
- le [(±) (15alpha, 16alpha)] 2-[(14,15-dihydro 20,21-dinoréburnaménin-15-yl) méthylamino] éthanol,

ainsi que leurs sels d'addition avec les acides.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques, tel que l'acide méthanesulfonique, l'acide éthanesulfonique, l'acide propanesulfonique, les acides alcoylsdisulfoniques tels que l'acide méthanedisulfonique, l'acide alpha, béta-éthanedisulfonique, les acides arylmonosulfoniques, tel que l'acide benzène-sulfonique et les acides aryldisulfoniques.

L'invention a aussi pour objet un procédé de préparation des composés de formule (I), telle que définie précédemment, caractérisé en ce que l'on soumet un composé de formule (II) :

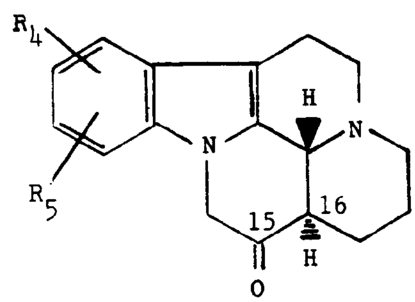

dans laquelle $R_4$ et $R_5$ ont la signification indiquée précédemment

- soit à l'action d'une amine aliphatique de formule ($III_A$) :

$$H_2NR'_1 \qquad (III_A)$$

dans laquelle $R'_1$ a la signification précédente de $R_1$, sauf hydrogène, pour obtenir un composé de formule (IV) :

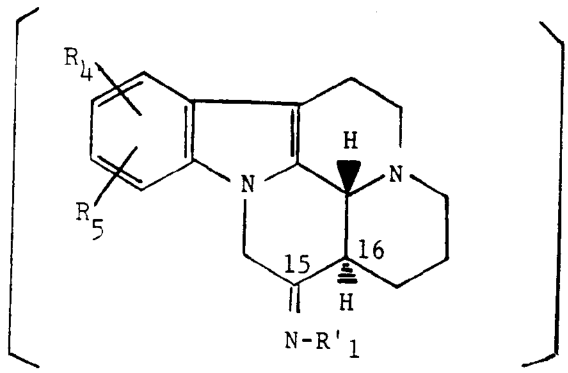

(IV)

que l'on réduit pour obtenir un composé de formule ($I_{A_1}$) :

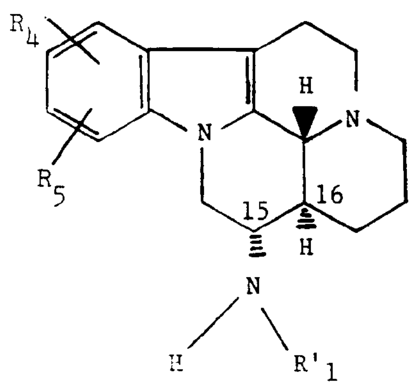

($I_{A_1}$)

dans laquelle $R'_1$, $R_3$ et $R_4$ ont la signification déjà indiquée,

- soit à l'action d'une amine hétérocyclique de formule ($III_B$) :

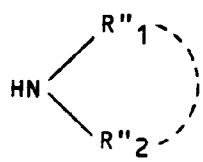

($III_B$)

dans laquelle $R''_1$ et $R''_2$ forment avec l'atome d'azote auquel ils sont liés les mêmes amines hétérocycliques que celles données précédemment pour $R_1$ et $R_2$, pour obtenir un composé de formule (V) :

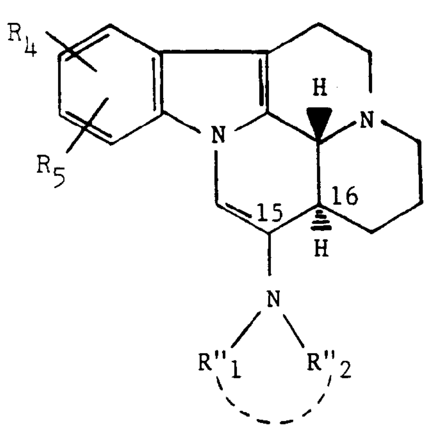

(V)

que l'on réduit pour obtenir un composé de formule ($I_{A_2}$) :

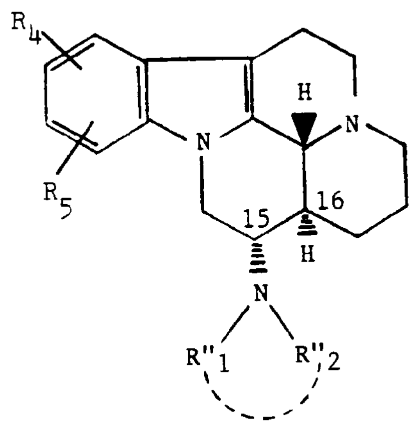

dans laquelle R"$_1$, R"$_2$, $R_4$ et $R_5$ ont les valeurs données précédemment

- soit à l'action d'un agent réducteur pour obtenir un composé de formule (VI) :

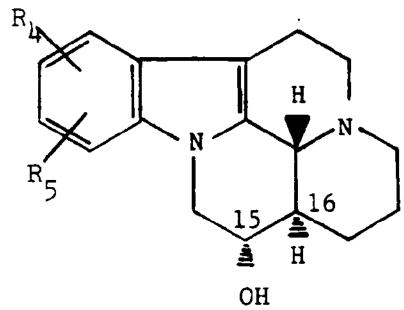

(VI)

dont on active la fonction hydroxyle et que l'on soumet

- soit à l'action d'une amine aliphatique ($III_A$) :

$$H_2N\text{-}R'_1 \qquad (III_A)$$

$R'_1$ ayant la signification précédente, pour obtenir un composé de formule ($I_{B_1}$) :

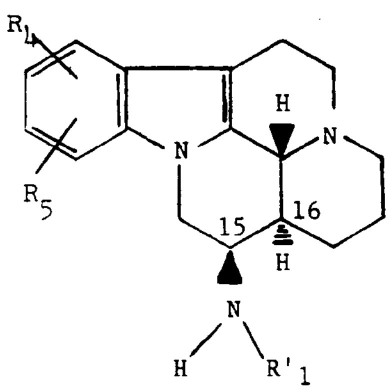

($I_{B_1}$)

$R'_1$, $R_4$ et $R_5$ ayant la signification précédente,

- soit à l'action d'une amine hétérocyclique de formule ($III_B$) :

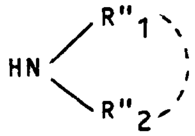

$(III_B)$

$R''_1$ et $R''_2$ ayant les significations précédentes, pour obtenir un composé de formule $(I_{B_2})$ :

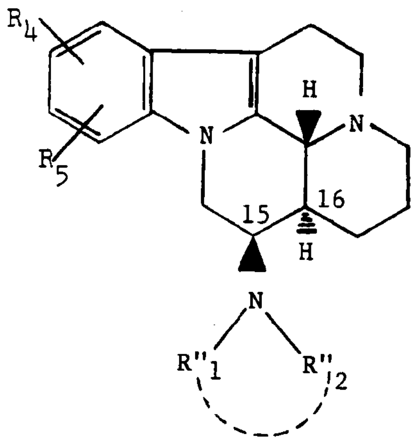

$(I_{B_2})$

dans laquelle $R''_1$ , $R''_2$, $R_4$ et $R_5$ ont les significations déjà indiquées et que, si désiré, on soumet les composés de formules $(I_{A_1})$ et $(I_{B_1})$ à l'action d'un réactif permettant de greffer sur l'amine en 15, un radical $R'_2$ ayant les mêmes valeurs données précédemment pour $R_2$, sauf hydrogène, pour obtenir les composés de formules $(I_{A_3})$ et $(I_{B_3})$ :

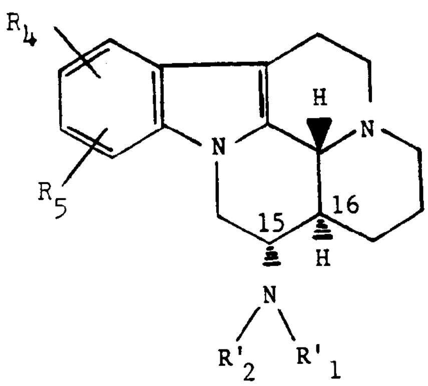

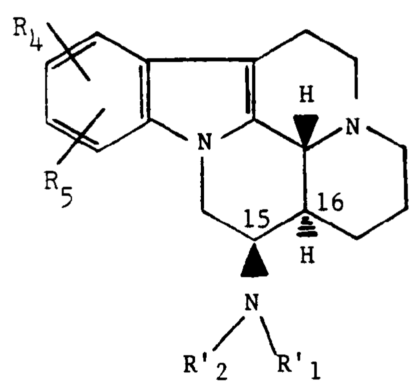

$(I_{A_3})$ $(I_{B_3})$

dans lesquelles $R'_1$, $R'_2$, $R_4$ et $R_5$ ont les significations précédentes et que, si désiré, dans le cas où le radical $R'_2$ à introduire est un radical méthyle, on soumet les composés de formules $(I_{A_1})$ et $(I_{B_1})$ à l'action d'un formiate de formule :

$$HCO_2R$$

dans laquelle R est un radical alkyle renfermant de 1 à 4 atomes de carbone pour obtenir un composé de formule (VII) :

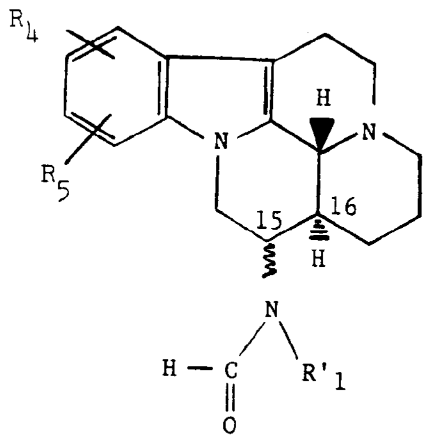

(VII)

que l'on réduit pour obtenir des composés de formules $(I_{A_3})$ et $(I_{B_3})$ dans lesquelles le radical $R'_2$ est un radical méthyle et que, si désiré, on réduit les composés de formules $(I_{A_1})$ et $(I_{B_1})$ pour lesquels $R'_1$ est un radical arylalkyle éventuellement substitué, pour obtenir les composés de formules $(I_{A_4})$ et $(I_{B_4})$ :

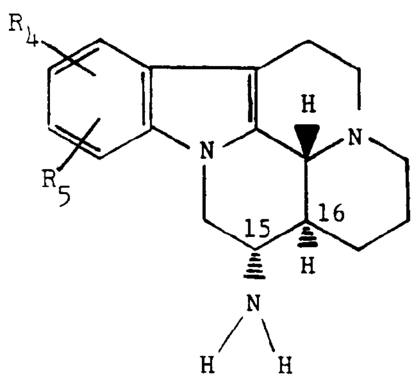

$(I_{A_4})$

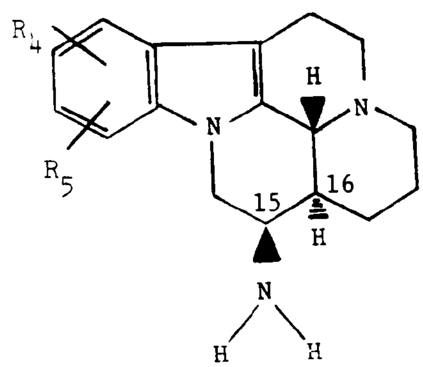

$(I_{B_4})$

dans lesquelles $R_4$ et $R_5$ ont les significations déjà indiquées, et que si désiré, l'on soumet les composés de formules $(I_{A_4})$ et $(I_{B_4})$,

- soit à l'action d'un réactif permettant de greffer les radicaux $R'_1$ et $R'_2$, pour obtenir les composés de formules $(I_{A_1})$, $(I_{B_1})$, $(I_{A_3})$ ou $(I_{B_3})$,
- soit à l'action d'un formiate $HCO_2R$ pour obtenir un composé de formule (VIII) :

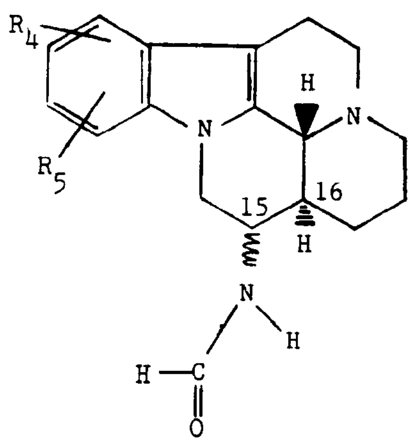

(VIII)

que l'on réduit pour obtenir un composé de formules ($I_{A_1}$) et ($I_{B_1}$) dans lesquelles le radical $R'_1$ est un radical méthyle, et traite, si désiré, tous les produits de formule (I) ainsi obtenus par un acide minéral ou organique.

Dans les conditions préférentielles de mise en oeuvre du procédé de l'invention :

- La réaction du composé de formule (II) avec les amines de formule ($III_A$) ou ($III_B$) s'effectue en présence d'un acide de Lewis qui est de préférence le tétrachlorure de titane.
- La réduction de l'imine de formule (IV) en composé de formule ($I_{A_1}$) et du composé de formule (V) en composé de formule ($I_{A_2}$) est réalisée par le borohydrure de sodium ou par le cyanoborohydrure de sodium.
- La réduction de l'imine de formule (IV) est réalisée à température ambiante et celle du composé de formule (V) au reflux d'un solvant tel que l'éthanol ou l'acide acétique.
- La réduction du composé de formule (II) en composé de formule (VI) s'effectue par le borohydrure de sodium en présence d'un alcool tel que le méthanol.
- L'activation de la fonction hydroxyle du composé de formule (VI) est réalisée par le chlorure de méthanesulfonyle.
- L'introduction d'un radical $R'_2$ sur l'amine en position 15 dans les composés de formules ($I_{A_1}$) et ($I_{B_1}$) pour obtenir les composés de formules ($I_{A_3}$) et ($I_{B_3}$) s'effectue grâce à un réactif du type X-$R'_2$ dans lequel X est un atome d'halogène.

Lorsque $R'_2$ est un radical alkyle, X est un atome d'iode.

Lorsque $R'_2$ est un radical

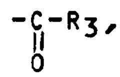

X est un atome de chlore.

Pour obtenir un composé de formule ($I_{A_3}$) ou ($I_{B_3}$), dans laquelle $R'_2$ est un radical méthyle, on fait réagir sur un composé de formules ($I_{A_1}$) et ($I_{B_1}$) le formiate d'éthyle pour obtenir un composé de formule (VII) que l'on réduit ensuite par un hydrure. On utilise pour cette réduction, notamment un hydrure mixte, tel que par exemple, l'hydrure mixte de lithium et d'aluminium, le diéthylhydrure de sodium et d'aluminium. On peut aussi utiliser le borane-diméthylamine.

Comme réactif permettant d'introduire un radical méthyle sur les composés de formule ($I_{A_1}$) ou ($I_{B_1}$), ou deux radicaux méthyle sur les composés de formule ($I_{A_4}$) ou ($I_{B_4}$), on peut aussi utiliser le mélange acide formique/aldéhyde formique.

- La méthode de réduction utilisée pour obtenir les composés de formules ($I_{A_4}$) et ($I_{B_4}$), à partir des composés de formules ($I_{A_1}$) et ($I_{B_1}$) pour lesquels $R'_1$ est un radical arylalkyle éventuellement substitué, est un hydrogénation catalytique, le catalyseur étant le platine ou le palladium.
- La formylation des composés de formules ($I_{A_4}$) et ($I_{B_4}$) en composés de formule (VIII) et la réduction des composés de formule (VIII) s'effectue dans les mêmes conditions que celles données ci-dessus pour les composés de formules ($I_{A_1}$) et ($I_{B_1}$).
- On peut transformer de façon usuelle dans le procédé de l'invention un radical

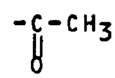

en radical éthyle par réduction par un hydrure et notamment par action de l'hydrure mixte de lithium et d'aluminium, ou par action du borane complexe diméthylsulfure.

- Les formes optiquement actives des produits de formule (I) peuvent être préparées par dédoublement des racémiques, selon les méthodes usuelles.

Les composés de formule (I) tels que définis ci-dessus ainsi que leurs sels d'addition avec les acides présentent d'intéressantes propriétés pharmacologiques. Ils présentent en particulier une très bonne activité analgésique.

Ces propriétés justifient leur application en thérapeutique et l'invention a également pour objet à titre de médicaments, des produits tels que définis par la formule (I) ci-dessus, lesdits produits de formule (I) étant sous toutes les formes isomères possibles racémique ou optiquement actives, ainsi que les sels d'addition avec les acides minéraux ou organiques pharmaceutiquement acceptables desdits produits de formule (I).

L'invention a plus particulièrement pour objet, à titre de médicament :

- la [(±) (15alpha, 16alpha)] 14,15-dihydro N-méthyl 20,21-dinoréburnaménin-15-amine,
- la [(±) (15alpha, 16alpha)] 14,15-dihydro 20,21-dinoréburnaménin-15-amine,
- le [(±) (15alpha, 16alpha)] 2-[(14,15-dihydro 20,21-dinoréburnaménin-15-yl) méthylamino] éthanol,

ainsi que leurs sels d'addition avec les acides, pharmaceutiquement acceptables.

Les médicaments, objet de l'invention, peuvent être utilisés dans le traitement des algies musculaires, articulaires ou nerveuses, les douleurs dentaires, les migraines, le zona et également à titre de traitement complémentaire dans les états infectieux et fébriles.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine, comme par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 20 mg à 2 g par jour chez l'adulte, par voie orale.

Le composé de formule (II) dans laquelle $R_4$ et $R_5$ représentent un atome d'hydrogène, utilisé comme produit de départ dans le procédé de l'invention est décrit dans le brevet européen n¤ 0 013 315.

Les produits de formule (II) dans laquelle l'un au moins des substituants $R_4$ et $R_5$ ne représente pas un atome d'hydrogène peuvent être préparés selon le procédé décrit dans le brevet cité précédemment à partir des tryptamines substituées correspondantes.

Un autre procédé de préparation des produits de formule (II) consiste à soumettre un produit de formule

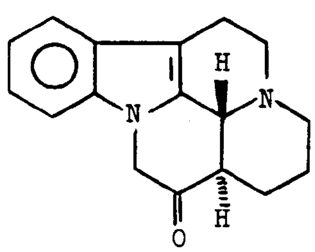

à une réaction de nitration pour obtenir un produit de formule ($II_A$) :

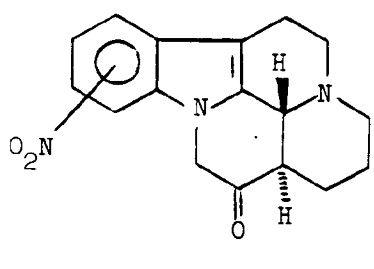

($II_A$)

que l'on réduit le cas échéant pour obtenir un produit de formule ($II_B$) :

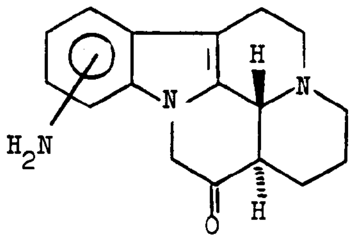

($II_B$)

que le cas échéant, soit l'on soumet à une réaction d'alkylation ou d'acylation, soit l'on transforme en sel de diazonium à partir duquel on prépare par les procédés connus les dérivés de formule ($II_C$) :

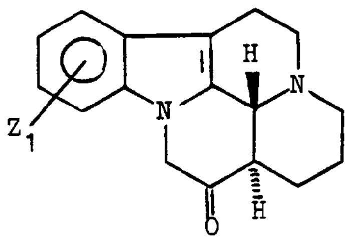

($II_C$)

dans laquelle $Z_1$ représente un radical hydroxy, trifluorométhyle ou un atome d'halogène que l'on transforme le cas échéant en dérivés correspondants dans lesquels $Z_1$ représente un radical alkoxyle ou alkyle.

Des exemples correspondants de préparation de produit de formule (II) sont donnés ci-après dans la partie expérimentale.

La présente invention a enfin pour objet à titre de produits industriels nouveaux, notamment à titre de produits intermédiaires nécessaires à la préparation des produits de formule (I), les produits de formule (IV), (V), (VII), (VIII) ainsi que les produits de formules (II) et (VI) dans lesquels $R_4$ et $R_5$ ne représentent pas chacun un atome d'hydrogène.

Les exemples suivants illustrent l'invention sans toutefois la limiter.

<u>Exemple 1</u> : Maléate de [(15béta, 16alpha) (±)] 14,15-dihydro N-(phényl méthyl) 20,21-dinoréburnaménin-15-amine.

<u>Stade A</u> : méthanesulfonate de [(15alpha, 16alpha) (±)] 14,15-dihydro 20,21-dinoréburnaménin-15-ol.

A une suspension de 3,15 g de [(±) (15alpha, 16alpha)] 14,15-dihydro 20,21-dinoréburnaménin-15-ol dans 20 $cm^3$ de pyridine on ajoute, à 20°C, 0,95 $cm^3$ de chlorure de mésyle. On agite 15 heures à température ambiante. On ajoute ensuite 200 $cm^3$ d'eau glacée, essore, lave à l'eau. On obtient 4 g de produit attendu F = 222°C.

<u>Stade B</u> : [(15béta, 16alpha) (±)] 14,15-dihydro N-(phényl méthyl) 20,21-dinoréburnaménin-15-amine.

On agite pendant 8 heures à 130°C un mélange de 6,8 g de produit obtenu comme au stade A et 100 $cm^3$ de benzylamine. La solution obtenue est amenée à sec. On reprend le résidu à l'éther isopropylique. On filtre l'insoluble et concentre les eaux mères à sec. On chromatographie le résidu sur silice en éluant avec un mélange chlorure de méthylène méthanol (88-2), on recueille ainsi 4,36 g de produit attendu.

<u>Stade C</u> : Maléate de [(15béta, 16alpha) (±)] 14,15-dihydro N-(phényl méthyl) 20,21-dinoréburnaménin-15-amine.

0,71 g de produit obtenu au stade B de l'exemple 1 sont mis en solution dans 40 $cm^3$ d'éthanol, on ajoute une solution de 461 mg d'acide maléique dans 20 $cm^3$ d'éthanol. On agite 3 heures à température ambiante. On essore, lave à l'éthanol puis à l'acétate d'éthyle. On obtient 720 mg de produit attendu F = 257°C.

| Analyse : pour $C_{28}H_{31}N_3O_4$ | | | |
|---|---|---|---|
| % calculés | C 71.01 | H 6.6 | N 8.87 |
| % trouvés | 70.8 | 6.5 | 8.8 |

Exemple 2 : [(15béta, 16alpha) (±)] 14,15-dihydro 20,21-dinoréburnaménin-15-amine.

On dissout 3 g de produit obtenu au stade B de l'exemple 1 dans 150 $cm^3$ d'éthanol, ajoute 600 mg de palladium à 10 % sur charbon actif, chauffe à 50°C sous 200 g de pression d'hydrogène et agite pendant 8 heures en maintenant ces conditions. On filtre le catalyseur et amène la solution à sec. On reprend le résidu avec du chlorure de méthylène, on sèche la solution organique avec du sulfate de sodium, filtre et amène à sec. Le résidu est chromatographié sur silice (éluant : acétate d'éthyle-méthanol-triéthylamine (8-1-1)).

On obtient 1,5 g de produit attendu F = 140°C.

Exemple 3 : Fumarate de [(15béta, 16alpha) (±)] 14,15-dihydro N-méthyl 20,21-dinoréburnaménin-15-amine.

Stade A : [(±) (15béta, 16alpha)] N-(14,15-dihydro 20,21-dinoréburnaménin-15-yl) formamide.

On agite au reflux pendant 5 heures un mélange de 1,37 g de produit obtenu à l'exemple 2 et 50 $cm^3$ de formiate d'éthyle. On amène à sec la solution obtenue. On obtient 1,5 g de produit attendu F = 125°C.

Stade B : [(15béta, 16alpha) (±)] 14,15-dihydro N-méthyl 20,21-dinoréburnaménin-15-amine.

A une suspension de 600 mg d'hydrure d'aluminium et de lithium dans 20 $cm^3$ de tétrahydrofuranne, on ajoute, sous agitation à température ambiante, une solution de 1,5 g de produit obtenu au stade A de l'exemple 3 dans 10 $cm^3$ de tétrahydrofuranne. Après 2 heures à température ambiante, on chauffe jusqu'au reflux que l'on maintient pendant 5 heures. On laisse revenir à température ambiante, ajoute une solution de tétrahydrofuranne à 10 % d'eau, puis extrait avec de l'acétate d'éthyle. On amène à sec et chromatographie le résidu sur silice, on obtient 720 mg de produit attendu F = 146°C.

Stade C : Fumarate de [(15béta, 16alpha) (±)] 14,15-dihydro N-méthyl 20,21-dinoréburnaménin-15-amine.

680 mg de produit obtenu au stade B sont dissous dans 50 $cm^3$ d'acétate d'éthyle, on ajoute sous agitation une solution de 561 mg d'acide fumarique dans 20 $cm^3$ d'éthanol. On agite pendant 5 heures à température ambiante, essore, lave à l'acétate d'éthyle. On obtient 1,1 g de produit attendu F = 222°C.

| Analyse : pour $C_{26}H_{31}N_3O_8$ | | | |
|---|---|---|---|
| % calculés | C 60.81 | H 6.08 | N 8.18 |
| % trouvés | 60.7 | 6.1 | 8.0 |

Exemple 4 : Fumarate de [(15béta, 16alpha) (±)] 14,15-dihydro N,N-diméthyl 20,21-dinoréburnaménin-15-amine.

Stade A : [(15béta, 16alpha) (±)] 14,15-dihydro N,N-diméthyl 20,21-dinoréburnaménin-15-amine.

On agite à 40°C pendant 2 heures 30 minutes une solution de 1,17 g de produit obtenu à l'exemple 2 dans 10 $cm^3$ d'aldéhyde formique (à 40 % dans l'eau) et 5 $cm^3$ d'acide formique. On ajoute ensuite 100 $cm^3$ d'eau et de l'ammoniaque concentré jusqu'à pH alcalin.

On extrait avec du chlorure de méthylène, lave à l'eau, concentre à sec. On chromatographie le résidu sur silice (éluant : acétate d'éthyle-triéthylamine (95-5)) ; on obtient 1,12 g de produit attendu F = 138°C.

Stade B : Fumarate de [(15béta, 16alpha) (±)] 14,15-dihydro N,N-diméthyl 20,21-dinoréburnaménin-15-amine.

A une solution de 1,1 g du produit obtenu au stade A dans 50 cm³ d'acétate d'éthyle, on ajoute une solution de 864 mg d'acide fumarique dans 50 cm³ d'acétate d'éthyle et 10 cm³ d'éthanol. On agite pendant 2 heures à température ambiante, essore, lave à l'acétate d'éthyle. On obtient 1,25 g de produit attendu F = 215°C.

Analyse : pour $C_{23}H_9N_3O_4$

| | | | |
|---|---|---|---|
| % calculés | C 67.13 | H 7.1 | N 10.21 |
| % trouvés | 67.3 | 7.0 | 10.2 |

**Exemple 5** : Maléate acide de [(15alpha, 16alpha) (±)] 14,15-dihydro N-méthyl 20,21-dinoréburnaménin-15-amine.

Stade A : [(15alpha, 16alpha) (±)] 14,15-dihydro N-méthyl 20,21-dinoréburnaménin-15-amine.

A une solution de 7 g de [(±) (16alpha)] 20,21 dinoréburnaménin-15 (14H)-one dans 140 cm³ d'éther éthylique et 70 cm³ de toluène on ajoute à -10°C par barbotage et jusqu'à saturation de la monométhylamine puis à -5°C du tétrachlorure de titane en solution dans un mélange 10 cm³ d'éther et 10 cm³ de toluène. On agite 30 minutes à 0°C puis 1 heure à température ambiante. On filtre l'insoluble, le lave à l'éther et amène à sec la solution organique. On dissout le résidu dans 150 cm³ d'éthanol et ajoute par fractions 1,5 g de borohydrure de sodium. On agite pendant 1 heure à température ambiante puis ajoute 200 cm³ d'eau. On essore et lave à l'eau. On obtient 6,08 g de produit attendu F = 170°C.

Stade B : Maléate acide de [(15alpha, 16alpha) (±)] 14,15-dihydro N-méthyl 20,21-dinoréburnaménin-15-amine.

6,08 g du produit obtenu au stade A sont dissous dans 200 cm³ d'acétate d'éthyle, on ajoute à 20°C, 5,01 g d'acide maléique en solution dans 100 cm³ d'éthanol. Après 1 heure à 25°C, on essore et lave à l'acétate d'éthyle. On recristallise dans 200 cm³ de méthanol.

On obtient 5,05 g de produit attendu F = 220°C.

Analyse : pour $C_{26}H_{31}N_3O_8$

| | | | |
|---|---|---|---|
| % calculés | C 60.81 | H 6.08 | N 8.18 |
| % trouvés | 60.6 | 6.0 | 8.1 |

**Exemple 6** : Maléate de [(15alpha, 16alpha) (±)] 14,15-dihydro N,N-diméthyl 20,21-dinoréburnaménin-15-amine.

Stade A : [(15alpha, 16alpha) (±)] N-(14,15-dihydro 20,21-dinoréburnaménin-15-yl) N-méthyl formamide.

On agite au reflux pendant 15 heures une suspension de 1,8 g de produit obtenu au stade A de l'exemple 5 et 36 cm³ de formiate d'éthyle. On amène à sec la solution obtenue. On obtient 1,95 g de produit attendu F = 216°C.

Stade B : [(15alpha, 16alpha) (±)] 14,15-dihydro N,N-diméthyl 20,21-dinoréburnaménin-15-amine.

A une suspension de 360 mg d'hydrure d'aluminium et de lithium dans 10 cm³ de tétrahydrofuranne, on ajoute à 0°C une solution de 1,95 g de produit obtenu au stade A dans 50 cm³ de tétrahydrofuranne. Après 1 heure d'agitation à 20°C on ajoute du tétrahydrofuranne à 10 % d'eau. On filtre l'insoluble et concentre à sec. On reprend le résidu avec de l'acétate d'éthyle, lave à l'eau, sèche et concentre à sec. On chromatographie le résidu obtenu en éluant avec : acétate d'éthyletriéthylamine (95-5). On obtient 1,31 g de produit attendu F = 134-35°C.

Stade C : Maléate de [(15alpha, 16alpha) (±)] 14,15-dihydro N,N-diméthyl 20,21-dinoréburnaménin-15-amine.

A une solution de 1,31 g de produit du stade B dans 100 $cm^3$ d'acétate d'éthyle, on ajoute 1,03 g d'acide maléique dissous dans 50 $cm^3$ d'éthanol. Après 2 heures à température ambiante, on essore et lave à l'acétate d'éthyle. On obtient 2,3 g de produit attendu F = 172°C ; par recristallisation dans l'éthanol on obtient 1,68 g de produit purifié F = 175-8°C.

Analyse : pour $C_{27}H_{33}N_3O_8$

| | | | |
|---|---|---|---|
| % calculés | C 61.47 | H 6.3 | N 7.96 |
| % trouvés | 61.2 | 6.5 | 7.8 |

**Exemple 7** : Fumarate de [(15alpha, 16alpha) (±)] N-(14,15-dihydro 20,21-dinoréburnaménin-15-yl) N-méthyl acétamide.

Stade A : [(15alpha, 16alpha) (±)] N-(14,15-dihydro 20,21-dinoréburnaménin-15-yl) N-méthyl acétamide.

A une solution de 0,8 g de produit obtenu au stade A de l'exemple 5 dans 0,8 $cm^3$ de triéthylamine et 15 $cm^3$ de tétrahydrofuranne, on ajoute à 0°C, 0,2 $cm^3$ de chlorure d'acétyle en solution dans 5 $cm^3$ de tétrahydrofuranne. On agite 30 minutes à température ambiante, filtre l'insoluble et amène à sec. On obtient 750 mg de produit attendu F = 196°C.

Stade B : Fumarate de [(15alpha, 16alpha) (±)] N-(14,15-dihydro 20,21-dinoréburnaménin-15-yl) N-méthyl acétamide.

A une solution de 750 mg de produit obtenu au stade A dans l'acétate d'éthyle on ajoute une solution de 400 mg d'acide fumarique dans l'acétate d'éthyle. Après 5 heures à 20°C on essore et obtient : 920 mg de produit attendu F > 260°C.

Analyse : pour $C_{24}H_{29}N_3O_5$

| | | | |
|---|---|---|---|
| % calculés | C 65.59 | H 6.65 | N 9.56 |
| % trouvés | 65.5 | 6.8 | 9.4 |

**Exemple 8** : Maléate de [(15alpha, 16alpha) (±)] 14,15-dihydro N-éthyl N-méthyl 20,21-dinoréburnaménin-15-amine.

Stade A : [(15alpha, 16alpha) (±)] 14,15-dihydro N-éthyl N-méthyl 20,21-dinoréburnaménin-15-amine.

A une solution de 810 mg de produit obtenu comme au stade A de l'exemple 7 dans 20 $cm^3$ de tétrahydrofuranne on ajoute 1 $cm^3$ du complexe borane-diméthyl sulfure ($BH_3$, $Me_2S$). On agite pendant 2 heures au reflux. On refroidit à + 10°C et ajoute 5 $cm^3$ d'acide chlorydrique 2N puis on porte 30 minutes au reflux.
On ramène à température ambiante, ajoute 50 $cm^3$ d'eau et alcalinise avec de l'ammoniaque concentré. On extrait à l'acétate d'éthyle, amène à sec et chromatographie le résidu obtenu, sur silice (éluant : chlorure de méthylène-méthanol (97-3)).
On obtient 690 mg de produit attendu F = 100°C.

Stade B : Maléate de [(15alpha, 16alpha) (±)] 14,15-dihydro N-éthyl N-méthyl 20,21-dinoréburnaménin-15-amine.

A une solution de 690 mg de produit obtenu au stade A, on ajoute à 20°C une solution de 518 mg d'acide maléique dans 30 $cm^3$ d'éthanol. Après 2 heures d'agitation à 20°C on essore, lave à l'éthanol et obtient 1,03 g de produit attendu F = 192°C.

| Analyse : pour $C_{28}H_{35}N_3O_8$ | | | |
|---|---|---|---|
| % calculés | C 62.09 | H 6.51 | N 7.76 |
| % trouvés | 62.0 | 6.7 | 7.7 |

**Exemple 9** : Fumarate de [(15alpha, 16alpha) (±)] 3,4-dichloro N-(14,15-dihydro 20,21-dinoréburnaménin-15-yl) N-méthyl benzèneacétamide.

Stade A : [(15alpha, 16alpha) (±)] 3,4-dichloro N-(14,15-dihydro 20,21-dinoréburnaménin-15-yl) N-méthyl benzèneacétamide.

A une solution de 800 mg de produit obtenu comme au stade A de l'exemple 5 dans 25 $cm^3$ de tétrahydrofuranne et 0,8 $cm^3$ de triéthylamine, on ajoute 1 g de chlorure de l'acide 3,4-dichloro phénylacétique en solution dans 2 $cm^3$ de tétrahydrofuranne. On agite 1 heure, filtre l'insoluble, amène à sec la solution organique. On chromatographie le résidu sur silice (éluant chlorure de méthylène-méthanol (95-5)) ; après avoir amené à sec, le résidu est empâté dans 50 $cm^3$ d'éther isopropylique, on obtient 1 g de produit attendu F = 166°C.

Stade B : Fumarate de [(15alpha, 16alpha) (±)] 3,4-dichloro N-(14,15-dihydro 20,21-dinoréburnaménin-15-yl) N-méthyl benzèneacétamide.

1 g de produit obtenu au stade A est dissous dans un mélange de 10 $cm^3$ d'éthanol et 50 $cm^3$ d'acétate d'éthyle, on ajoute à cette solution 248 mg d'acide fumarique dissous dans 20 $cm^3$ d'éthanol. On agite 4 heures à température ambiante, essore, lave à l'éthanol puis à l'acétate d'éthyle. On obtient 970 mg de produit attendu F = 257°C.

| Analyse : pour $C_{30}H_{31}N_3O_5Cl_2$ | | | | |
|---|---|---|---|---|
| % calculés | C 61.65 | H 5.34 | N 7.19 | Cl 12.13 |
| % trouvés | 61.7 | 5.5 | 6.9 | 11.8 |

**Exemple 10** : Maléate de [(15alpha, 16alpha) (±)] 14,15-dihydro 15-(1-pyrrolidinyl) 20,21-dinoréburnaménine.

Stade A : [(16alpha) (±)] 15-(1-pyrrolidinyl) 20,21-dinoréburnaménine.

A une solution de 3 g de [(16alpha) (±)] 20,21-dinoréburnaménin-15 (14H)-one dans un mélange de 150 $cm^3$ d'éther éthylique et 150 $cm^3$ de toluène on ajoute à 0°C, 0,72 $cm^3$ de tétrachlorure de titane en solution, dans un mélange de 20 $cm^3$ d'éther éthylique et 20 $cm^3$ de toluène. On agite à 0°C pendant 30 minutes puis 15 heures à température ambiante. On filtre l'insoluble et amène à sec. On obtient 3,5 g de produit attendu F = 180°C.

Stade B : [(15alpha, 16alpha) (±)] 14,15-dihydro 15-(1-pyrrolidinyl) 20,21-dinoréburnaménine.

A une solution de 3,5 g de produit obtenu au stade A dans 200 $cm^3$ d'éthanol, on ajoute par fraction 2,08 g de borohydrure de sodium puis on chauffe au reflux que l'on maintient pendant 4 heures.
On laisse revenir à température ambiante et ajoute 200 $cm^3$ d'eau glacée, essore le précipité, lave à l'eau. On chromatographie sur silice, le produit brut obtenu (éluant : chlorure de méthylène-méthanol (95-5)) puis recristallise l'extrait sec dans 180 $cm^3$ d'éther isopropylique. On obtient 1,7 g de produit attendu F = 156°C.

Stade C : Maléate de [(15alpha, 16alpha) (±)] 14,15-dihydro 15-(1-pyrrolidinyl) 20,21-dinoréburnaménine.

A une solution de 1,7 g de produit obtenu au stade B dans un mélange de 50 $cm^3$ d'acétate d'éthyle et 50 $cm^3$ d'éthanol, on ajoute une solution de 1,23 g d'acide maléique dans 50 $cm^3$ d'éthanol. On agite pendant 15 heures à température ambiante, essore, lave à l'acétate d'éthyle.
On obtient 2,6 g de produit attendu F = 232°C.

| Analyse : pour $C_{29}H_{35}N_3O_8$ | | | |
|---|---|---|---|
| % calculés | C 62.92 | H 6.37 | N 7.59 |
| % trouvés | 63.2 | 6.5 | 7.4 |

**Exemple 11** : Maléate de [(15alpha, 16alpha) (±)] 14,15-dihydro 15-(4-morpholinyl) 20,21-dinoréburnaménine.

Stade A : [(16alpha) (±)] 15-(4-morpholinyl) 20,21-dinoréburnaménine.

A une solution de 2,13 g de [(16alpha) (±)] 20,21-dinoréburnaménin-15(14H)-one dans un mélange de 40 $cm^3$ d'éther et 40 $cm^3$ de toluène on ajoute 3,6 $cm^3$ de morpholine. On refroidit à - 5°C, puis on ajoute sans dépasser 0°C, 0,5 $cm^3$ de chlorure de titane en solution dans un mélange de 10 $cm^3$ d'éther éthylique et 10 $cm^3$ de toluène. On agite pendant 1 heure à 0°C, puis 15 heures à température ambiante. On filtre l'insoluble et concentre à sec la solution organique. On obtient 1,6 g de produit attendu F = 176°C.

Stade B : [(15alpha, 16alpha) (±)] 14,15-dihydro 15-(4-morpholinyl) 20,21-dinoréburnaménine.

A une solution chauffée à 60°C de 1,6 g de produit obtenu au stade A dans 50 $cm^3$ d'acide acétique on ajoute 2 g de borohydrure de sodium, on agite pendant 20 minutes à 60°C puis laisse revenir à température ambiante. On ajoute 200 $cm^3$ d'eau et alcalinise le milieu avec de l'ammoniaque concentré.
On essore le précipité formé et le lave à l'eau. On purifie le produit brut par chromatographie sur silice (éluant : acétate d'éthyle à 1 % de triéthylamine). On obtient 1,25 g de produit attendu F = 222°C.

Stade C : Maléate de [(15alpha, 16alpha) (±)] 14,15-dihydro 15-(4-morpholinyl) 20,21-dinoréburnaménine.

On dissout 1 g de la base obtenue au stade B dans un mélange de 100 $cm^3$ d'acétate d'éthyle et 50 $cm^3$ d'éthanol, on ajoute à 20°C une solution de 692 mg d'acide maléique dans 20 $cm^3$ d'éthanol. On agite 2 heures et demie à 20°C puis 30 minutes à 0°C. On essore et lave à l'acétate d'éthyle. On obtient 1,07 g de produit attendu F = 250°C.

| Analyse : pour $C_{25}H_{31}N_3O_5$ | | | |
|---|---|---|---|
| % calculés | C 66.21 | H 6.89 | N 9.26 |
| % trouvés | 66.5 | 6.9 | 9.2 |

**Exemple 12** : Maléate de [(15alpha, 16alpha) (±)] 14,15-dihydro N-(2-phényl éthyl) 20,21-dinoréburnaménin-15-amine.

Stade A : [(15alpha, 16alpha) (±)] 14,15-dihydro N-(2-phényl éthyl) 20,21-dinoréburnaménin-15-amine.

A une solution de 1,6 g de [(16alpha) (±)] 20,21-dinoréburnaménin-15 (14H)-one dans un mélange éther 60 $cm^3$, toluène 60 $cm^3$, on ajoute 3,8 $cm^3$ de phénéthylamine. On refroidit à 0°C + 5°C et on ajoute 0,4 $cm^3$ de tétrachlorure de titane en solution dans un mélange d'éther 10 $cm^3$ et toluène 10 $cm^3$. On agite pendant 30 minutes à 0°C puis 2 heures à température ambiante. On filtre l'insoluble et concentre à sec. On dissout le résidu dans 75 $cm^3$ d'éthanol et ajoute à température ambiante 912 mg de borohydrure de sodium. On ajoute ensuite 100 $cm^3$ d'eau glacée puis extrait avec de l'acétate d'éthyle et concentre à sec. On chromatographie le résidu sur silice (éluant : chlorure de méthylène-méthanol (95-5)). On obtient 1,2 g de produit attendu F = 127°C.

Stade B : Maléate de [(15alpha, 16alpha) (±)] 14,15-dihydro N-(2-phényl éthyl) 20,21-dinoréburnaménin-15-amine.

On dissout 1,07 g du produit obtenu au stade A dans 100 $cm^3$ d'acétate d'éthyle et ajoute à cette solution 668 mg d'acide maléique en solution dans 50 $cm^3$ d'éthanol. Après 5 heures de contact on essore et lave à l'acétate d'éthyle, on obtient 1,3 g de produit attendu F = 235°C.

| Analyse : pour $C_{33}H_{37}N_3O_8$ | | | |
|---|---|---|---|
| % calculés | C 65.66 | H 6.18 | N 6.96 |
| % trouvés | 65.6 | 6.3 | 6.8 |

Exemple 13 : Oxalate acide de [(15alpha, 16alpha) (±)] 14,15-dihydro 15-(4-phényl 1-pipérazinyl) 20,21-dinoréburnaménine.

Stade A : (16alpha) (±) 15-(4-phényl 1-pipérazinyl) 20,21-dinoréburnaménine.

On opère comme au stade A de l'exemple 11 en utilisant 533 mg de [(16alpha) (±)] 20,21-dinoréburnaménine 15-(14H)-one et 1,5 cm3 de N-phénylpipérazine. On obtient, après purification par empâtage dans l'hexane, 610 mg de produit attendu. F = 202¤C.

Stade B : [(15alpha, 16alpha) (±)] 14,15-dihydro 15-(4-phényl 1-pipérazinyl) 20,21-dinoréburnaménine.

On opère comme au stade B de l'exemple 11 en utilisant 3,8 g de produit préparé comme au stade A. Après chromatographie sur silice (éluant : acétate d'éthyle), on obtient 1,8 g de produit attendu. F = 120¤C.

Stade C : Oxalate acide de [(15alpha, 16alpha) (±)] 14,15-dihydro 15-(4-phényl 1-pipérazinyl) 20,21-dinoréburnaménine.

On dissout 1,03 g de produit obtenu au stade B dans 50 cm3 d'acétate d'éthyle et ajoute 553 mg d'acide oxalique dissous à chaud dans 50 cm3 d'acétate d'éthyle. On agite 3 heures à température ambiante puis 15 minutes à 0¤C. On essore, sèche sous pression réduite et recueille 1,15 g de produit attendu. F > 260¤C.

| Analyse : | $C_{29}H_{34}N_4O_4$ | | |
|---|---|---|---|
| Calculé : | C% 69,3 | H% 6,82 | N% 11,15 |
| Trouvé : | 69,4 | 6,8 | 11,0 |

Exemple 14 : Oxalate acide de [(15alpha, 16alpha) (±)] 14,15-dihydro 15-(4-méthyl 1-pipérazinyl) 20,21-dinoréburnaménine.

Stade A : [(16alpha) (±)] 15-(4-méthyl 1-pipérazinyl) 20,21-dinoréburnaménine.

On opère comme au stade A de l'exemple 11 en utilisant 3 g de [(16alpha) (±)] 20,21-dinoréburnaménin-15-(14H)-one et 5,6 cm3 de N-méthylpipérazine. On obtient 3,9 g de produit attendu utilisé tel quel pour le stade suivant.

Stade B : [(15alpha, 16alpha) (±)] 14,15-dihydro 15-(4-méthyl 1-pipérazinyl) 20,21-dinoréburnaménine.

On opère comme au stade B de l'exemple 11 en utilisant 3 g de produit obtenu au stade A et 1,5 g de cyano borohydrure de sodium. Après chromatographie sur silice (éluant : acétate d'éthyle-méthanol-triéthylamine 95-5-5), on obtient 1,86 g de produit attendu.

Stade C : Oxalate acide de [(15alpha, 16alpha) (±)] 14,15-dihydro 15-(4-méthyl 1-pipérazinyl) 20,21-dinoréburnaménine.

On opère comme au stade C de l'exemple 11 en utilisant 1 g de la base obtenue au stade B et 771 mg d'acide oxalique. On obtient 1,45 g de produit attendu. F > 260¤C.

| Analyse | : | $C_{26}H_{34}N_4O_8$ | | |
|---|---|---|---|---|
| Calculé | : | C% 58,86 | H% 6,46 | N% 10,56 |
| Trouvé | : | 59,1 | 6,5 | 10,5 |

**Exemple 15** : Maléate de [(15alpha, 16alpha) (±)] 14,15-dihydro 20,21-dinoréburnaménin-15-amine.

Stade A : [(15alpha, 16alpha) (±)] 14,15-dihydro N-(phényl-méthyl) 20,21-dinoréburnaménin-15-amine.

On opère comme au stade A de l'exemple 12 en utilisant 2,13 g de [(16alpha) (±)] 20,21-dinoréburnaménin-15-(14H)-one, 4,4 cm3 de benzylamine et 0,5 cm3 de tétrachlorure de titane. Après extraction à l'acétate d'éthyle et concentration à sec, on reprend le résidu dans l'éther isopropylique, essore et sèche sous pression réduite à 50¤C. On récupère 2,07 g de produit attendu. F =166¤C.

Stade B : [(15alpha, 16alpha) (±)] 14,15-dihydro 20,21-dinoréburnaménin-15-amine.

On hydrogène pendant 6 heures à 50¤C sous une pression de 600 g, 1,5 g de produit obtenu au stade A dans 100 cm3 d'éthanol en présence de 300 mg de charbon actif à 10% de palladium. On filtre le catalyseur, concentre à sec la solution organique et récupère 1,03 g de produit attendu. F = 160¤C.

Stade C : Maléate de [(15alpha, 16alpha) (±)] 14,15-dihydro 20,21-dinoréburnaménin-15-amine.

On dissout 1,18 g de la base obtenue au stade B dans 50 cm3 d'éthanol et 100 cm3 d'acétate d'éthyle et ajoute 1,02 g d'acide maléïque dissous préalablement dans 50 cm3 d'éthanol. On agite 3 heures, essore le précipité, le lave à l'éthanol, le sèche à 50¤C sous pression réduite et recueille après recristallisation dans l'éthanol 1,2 g de produit attendu. F = 230¤C.

| Analyse | : | $C_{25}H_{29}N_3O_8$ | | |
|---|---|---|---|---|
| Calculé | : | C% 60,11 | H% 5,85 | N% 8,41 |
| Trouvé | : | 60,2 | 5,6 | 8,4 |

**Exemple 16** : Fumarate de [(15alpha, 16alpha) (±)] 2-[(14,15-dihydro 20,21-dinoréburnaménin-15-yl) méthylamino] éthanol.

Stade A : [(15alpha, 16alpha) (±)] [(14,15-dihydro 20,21-dinoréburnaménin-15-yl) méthylamino] acétate d'éthyle.

On dissout, sous atmosphère inerte, 2 g de [(15alpha, 16alpha) (±)] 14,15-dihydro N-méthyl 20,21-dinoréburnaménin-15-amine dans un mélange comprenant 3 cm3 de triéthylamine et 100 cm3 de tétrahydrofuranne et ajoute 0,83 cm3 d'iodo acétate d'éthyle, et de nouveau après 2 heures, puis 7 heures d'agitation, on effectue l'addition de 0,83 cm3 d'iodo acétate d'éthyle supplémentaire. On maintient encore 8 heures sous agitation, filtre, concentre à sec la phase organique, chromatographie le résidu sur silice (éluant : chlorure de méthylène-méthanol 95-5) et recueille 1,8 g de produit attendu.

Stade B : [(15alpha, 16alpha) (±)] 2-[(14,15-dihydro 20,21-dinoréburnaménin-15-yl) méthylamino] éthanol.

On ajoute sous atmosphère inerte 1,8 cm3 de complexe borane-disulfone dans 1,8 g de produit obtenu au stade A en solution dans 50 cm3 de tétrahydrofuranne puis chauffe 1 heure et demie au reflux. On laisse revenir à température ambiante, ajoute 50 cm3 d'acide chlorhydrique 0,1N, agite 15 minutes à 40¤C, ajoute 100 cm3 d'eau glacée, alcalinise à l'aide d'ammoniaque, essore le précipité, le lave à l'eau, le sèche à 80¤C sous pression réduite et obtient 0,8 g de produit attendu après purification par empâtage dans l'acétone. F = 200¤C.

Stade C : Fumarate de [(15alpha, 16alpha) (±)] 2-[(14,15-dihydro 20,21-dinoréburnaménin-15-yl) méthylamino] éthanol.

On dissout 457 mg de la base obtenue au stade B dans 100 cm3 d'acétate d'éthyle et 10 cm3 d'éthanol et ajoute 326 mg d'acide fumarique dissous dans 10 cm3 d'éthanol. On agite 2 heures à 0¤C, essore, sèche sous pression réduite à 70¤C et obtient 460 mg de produit attendu. F = 208¤C.

| | | | | |
|---|---|---|---|---|
| Analyse | : | $C_{29}H_{31}N_3O_5$ | | |
| Calculé | : | C% 65,29 | H% 7,07 | N% 9,52 |
| Trouvé | : | 65,3 | 7,1 | 9,6 |

**Exemple 17** : Maléate acide de [(3alpha, 15béta) 14,15-dihydro 20,21-dinoréburnaménin-15-amine.

Stade A : : [3alpha, 15béta (S)] 14,15-dihydro N-(1-phényl-éthyl) 20,21-dinoréburnaménin-15-amine et l'isomère [15alpha (S) 16alpha] correspondant.

On ajoute 4,1 cm3 de L(-) alpha-phénéthylamine à une suspension de 2,13 g de [(16alpha (±) ] 20,21-dinoréburnaménin-15-(14H)-one en suspension dans 50 cm3 d'éther et 50 cm3 de toluène. On refroidit à 0¤C/+5¤C et on ajoute 0,5 cm3 de tétrachlorure de titane en solution dans un mélange éther 10 cm3 et toluène 10 cm3. On agite pendant 30 minutes à 0¤C puis 1 heure à température ambiante. On ajoute 75 cm3 d'éthanol, ajoute à température ambiante 1,5 g de borohydrure de sodium et agite 1 heure et demie à température ambiante. On ajoute ensuite 200 cm3 d'eau glacée puis extrait avec de l'acétate d'éthyle et concentre à sec. On chromatographie le résidu sur silice (éluant : chlorure de méthylène-méthanol 95-5). On obtient 790 mg d'isomère A (isomère 3alpha) F = 166¤C [$alpha_D$] = +53,5¤ ± 1,5¤ (c = 1% $CHCl_3$) et 460 mg d'isomère B (isomère 16alpha) F = 182¤C
[$alpha_D$] = 206¤ ± 3¤ (c = 1% $CHCl_3$).

Stade B : [(3alpha, 15béta) 14,15-dihydro 20,21-dinoréburnaménin-15-amine.

On hydrogène pendant 16 heures à 60¤C sous une pression de 500 g, 500 mg d'isomère A préparé au stade A en solution dans 20 cm3 d'éthanol, en présence de 100 mg de charbon actif à 10% de palladium. On filtre le catalyseur, évapore à sec la phase organique, chromatographie le résidu sur silice (éluant : acétate d'éthyle-triéthylamine-méthanol 8-1-1) et recueille 290 mg de produit attendu. F = 135¤C.
[$alpha_D$] = -29¤ ± 2¤ (c = 0,5% éthanol).

Stade C : Maléate acide de [(3alpha, 15béta) 14,15-dihydro 20,21-dinoréburnaménin-15-amine.

On dissout 0,8 g de produit préparé comme au stade B dans 50 cm3 d'acétate d'éthyle et 50 cm3 d'éthanol et ajoute 694 mg d'acide maléïque dissous dans 50 cm3 d'éthanol. On agite pendant 2 heures à température ambiante, essore le précipité et le sèche à 80¤C sous pression réduite. On récupère 1,2 g de produit attendu. F = 240¤C.

| | | | | |
|---|---|---|---|---|
| Analyse | : | $C_{17}H_{21}N_3$, 2 $C_4H_4O_4$ | | |
| Calculé | : | C% 60,11 | H% 5,85 | N% 8,41 |
| Trouvé | : | 60,1 | 5,8 | 8,1 |

**Exemple 18** : Maléate acide de [(15alpha, 16alpha)] 14,15-dihydro 20,21-dinoréburnaménin-15-amine.

Stade A : [(15alpha, 16alpha)] 14,15-dihydro 20,21-dinoréburnaménin-15-amine.

On opère comme au stade B de l'exemple 17 en utilisant au départ 3,1 g de l'isomère B préparé comme au stade A de l'exemple 17. On obtient 1,15 g de produit attendu. F = 135¤C.
[$alpha_D$] = + 25¤ ± 2¤ (c = 0,5% éthanol).

Stade B : Maléate acide de [(15alpha, 16alpha)] 14,15-dihydro 20,21-dinoréburnaménin-15-amine.

On opère comme au stade C de l'exemple 17 en utilisant 550 mg de produit préparé au stade A et 477 mg d'acide maléïque. On obtient 940 mg de produit attendu. F = 238¤C.

| | | | | |
|---|---|---|---|---|
| Analyse | : $C_{17}H_{21}N_3$, 2 $C_4H_4O_4$ | | | |
| Calculé | : | C% 60,11 | H% 5,85 | N% 8,41 |
| Trouvé | : | 59,9 | 5,7 | 8,5 |

**Exemple 19** : Maléate acide de [(3alpha, 15béta)] 14,15-dihydro N-méthyl 20,21-dinoréburnaménin-15-amine.

Stade A : [(3alpha, 15béta)] N-[14,15-dihydro 20,21-dinoréburnaménin-15-yl] formamide.

On chauffe au reflux pendant 6 heures 1 g de la base obtenue au stade B de l'exemple 17 en solution dans un mélange comprenant 30 cm3 de formiate d'éthyle et 30 cm3 de tétrahydrofuranne. On filtre, concentre à sec et obtient 1,13 g de produit attendu. F = 211¤C.
[$alpha_D$] = + 53¤ (c = 1% $CHCl_3$).

Stade B : [(3alpha, 15béta)] 14,15-dihydro N-méthyl 20,21-dinoréburnaménine-15-amine.

On dissout 1,1 g de produit obtenu au stade A dans 50 cm3 de tétrahydrofuranne puis ajoute lentement 1,1 cm3 de borane complexe disulfure puis chauffe au reflux pendant 1 heure et demie. On laisse revenir à température ambiante puis acidifie à l'aide d'acide chlorhydrique 0,1N. On agite 15 heures à température ambiante, alcalinise par addition d'ammoniaque et extrait à l'acétate d'éthyle. On lave à l'eau la phase organique, la sèche et concentre à sec et chromatographie le résidu sur silice (éluant : acétate d'éthyle-triéthylamine-méthanol 8-1-1). On obtient 470 mg de produit attendu. F = 138¤C.
[$alpha_D$] = + 9,5¤ ± 1¤ (c = 1% $CHCl_3$).

Stade C : Maléate acide de [(3alpha, 15béta] 14,15-dihydro N-méthyl 20,21-dinoréburnaménine-15-amine.

On dissout 690 mg de base obtenue comme au stade B dans 100 cm3 d'acétate d'éthyle chaud, ajoute 567 mg d'acide maléïque en solution dans un mélange comprenant 50 cm3 d'acétate d'éthyle et 10 cm3 d'éthanol, agite 2 heures à température ambiante puis 30 minutes à 0¤C. On essore le précipité, le lave à l'acétate d'éthyle, le sèche à 60¤C sous pression réduite et recueille 1,06 g de produit attendu. F = 165¤C.

| | | | | |
|---|---|---|---|---|
| Analyse | : $C_{18}H_{23}N_3$, 2 $C_4H_4O_4$ | | | |
| Calculé | : | C% 60,81 | H% 6,08 | N% 8,18 |
| Trouvé | : | 60,6 | 6,2 | 8,1 |

**Exemple 20** : Maléate acide de [(15alpha, 16alpha)] N-méthyl 20,21-dinoréburnaménin-15-amine.

Stade A : [15alpha, 16alpha] N-[14,15-dihydro 20,21-dinoréburnaménin-15-yl] formamide.

On opère comme au stade A de l'exemple 19 en utilisant 560 mg de la base obtenue au stade A de l'exemple 18 et 10 cm3 de formiate d'éthyle. Le produit obtenu est chromatographié sur silice (éluant : acétate d'éthyle-triéthylamine-méthanol 8-1-1). On obtient 522 mg de produit attendu. F = 210¤C.
[$alpha_D$] = - 57,5¤ ± 1,5¤ (c = 1% $CHCl_3$).

Stade B : [(15alpha, 16alpha)] N-méthyl 20,21-dinoréburnaménin-15-amine.

On opère comme au stade B de l'exemple 19 en utilisant 490 mg du produit obtenu au stade A et 0,5 cm3 de borane complexe disulfure. Après chromatographie sur silice (éluant : chlorure de méthylène-méthanol-ammoniaque 93-6,5-0,5), on obtient 220 mg de produit attendu. F = 135¤C.

Stade C : Maléate acide de [(15alpha, 16alpha)] N-méthyl 20,21-dinoréburnaménin-15-amine.

On opère comme au stade C de l'exemple 15 en utilisant 524 mg de base préparée comme au stade B et 432 mg d'acide maléïque. On obtient 750 mg de produit que l'on purifie par recristallisation dans l'éthanol et recueille 660 mg de produit attendu. F = 170¤C.

| | | | |
|---|---|---|---|
| Analyse : | $C_{18}H_{23}N_3$, 2 $C_4H_4O_4$ | | |
| Calculé : | C% 60,81 | H% 6,08 | N% 8,18 |
| Trouvé : | 60,8 | 6,2 | 8,1 |

**Exemple 21** : Maléate neutre de [(15alpha, 16alpha) (±)] 14,15-dihydro N-méthyl 9-nitro 20,21-dinoréburnaménin-15-amine.

Stade A : [(15alpha, 16alpha) (±)] 14,15-dihydro N-méthyl 9-nitro 20,21-dinoréburnaménin-15-amine.

On refroidit à 0¤C 1,5 g de (16alpha) (±) 9-nitro 20,21-dinoréburnaménin-15-(14H)-one en suspension dans un mélange comprenant 100 cm3 d'éther et 100 cm3 de toluène et fait barboter de la monométhylamine jusqu'à saturation. On ajoute lentement à 0¤C 0,35 cm3 de tétrachlorure de titane en solution dans un mélange comprenant 5 cm3 d'éther et 5 cm3 de toluène et agite 1 heure et demie à 20¤C. On ajoute 100 cm3 d'éthanol puis 535 mg de borohydrure de sodium, agite 2 heures, ajoute 300 cm3 d'eau glacée, et extrait à l'acétate d'éthyle. On lave la phase organique à l'eau, la sèche, concentre à sec et chromatographie le résidu sur silice (éluant : acétate d'éthyle-méthanol-triéthylamine 8-1-1). On obtient 1 g de produit attendu. F = 168¤C.

Stade B : Maléate neutre de [(15alpha, 16alpha) (±)] 14,15-dihydro N-méthyl 9-nitro 20,21-dinoréburnaménin-15-amine.

On opère comme au stade C de l'exemple 15 en utilisant 930 mg de la base préparée au stade B et 661 mg d'acide maléïque. On obtient 1,12 g de produit attendu. F > 260¤C.

| | | | |
|---|---|---|---|
| Analyse : | $C_{18}H_{22}N_4O_2$, $C_4H_4O_4$ | | |
| Calculé : | C% 59,71 | H% 5,92 | N% 12,66 |
| Trouvé : | 59,6 | 5,9 | 12,6 |

Préparation de la [(16alpha) (±)] 9-nitro 20,21-dinoréburnaménin-15-(14H)-one et dérivé 11-nitro correspondant.

On mélange à -25¤C 5 cm3 d'acide nitrique dans 300 cm3 de chloroforme et 75 cm3 d'acide acétique puis ajoute goutte à goutte 15 g de [(16alpha) (±)] 20,21-dinoréburnaménin-15-(14H)-one en solution dans 100 cm3 de chloroforme. On maintient sous agitation pendant 30 minutes, verse le milieu réactionnel dans 500 cm3 d'eau glacée puis alcalinise à l'aide d'ammoniaque concentrée. On sépare la phase organique, la lave à l'eau, la sèche et concentre à sec. On chromatographie le résidu sur silice (éluant : acétate d'éthyle) et obtient 5,3 g de dérivé 9-nitro F = 230¤C et 5,62 g de dérivé 11-nitro F = 250¤C.

**Exemple 22** : Maléate neutre de [(15alpha, 16alpha) (±)] 14,15-dihydro N-méthyl 11-nitro 20,21-dinoréburnaménin-15-amine.

Stade A : [(15alpha, 16alpha) (±)] 14,15-dihydro N-méthyl 11-nitro 20,21-dinoréburnaménin-15-amine.

On opère comme au stade A de l'exemple 21 en utilisant 1,5 g de dérivé 11-nitro obtenu comme indiqué dans la préparation ci-dessus. On obtient 1 g de produit attendu. F = 168¤C.

Stade B : Maléate neutre de [(15alpha, 16alpha) (±)] 14,15-dihydro N-méthyl 11-nitro 20,21-dinoréburnaménin-15-amine.

On opère comme au stade C de l'exemple 15 en utilisant 930 mg de la base préparée au stade A. On obtient

1,12 g de produit attendu. F > 260¤C.

| | | | | |
|---|---|---|---|---|
| Analyse | : $C_{18}H_{22}N_4O_2$, $C_4H_4O_4$ | | | |
| Calculé | : | C% 59,71 | H% 5,92 | N% 12,66 |
| Trouvé | : | 59,6 | 5,9 | 12,6 |

**Exemple 23** :

On a préparé des comprimés répondant à la formule suivante :
- Produit de l'exemple 5 50 mg
-Excipient q.s.p. 350 mg
(Détail de l'excipient : lactose, talc, amidon; stéarate de magnésium).

Activité analgésique :

1) Test de la plaque chaude.

Des souris femelles pesant 22 à 24 g sont placées une par une sur une plaque de cuivre maintenue à 56°C : la réaction à la douleur se manifeste par le léchage des pattes avant de l'animal ; le temps de cette réaction est noté et l'on ne retient que les souris réagissant en moins de 8 secondes.
Les animaux sont répartis par groupes homogènes et traités par le produit à étudier administré par voie orale, un groupe ne recevant que le véhicule. Le temps de réaction à la douleur est de nouveau mesuré 30 minutes après le traitement. La dose active ou $DA_{100}$ est la dose qui augmente le temps de réaction de 100 %, 30 minutes après le traitement, compte tenu des variations du temps de réaction des animaux témoins.
Pour le produit de l'exemple 5 la $DA_{100}$ est de 100 mg/kg.

2) Test des étirements à l'acide acétique.

Le test employé est basé sur le fait signalé par R. KOSTER et Coll., (Fed, Proc., 1959, 1B, 412) selon lequel l'injection intrapéritonéale d'acide acétique provoque, chez la souris, des mouvements répétés d'étirement et de torsion pouvant persister pendant plus de six heures. Les analgésiques préviennent ou diminuent ce syndrome qui peut être considéré comme l'extériorisation d'une douleur abdominale diffuse. On utilise une solution d'acide acétique à 1 % dans l'eau. La dose déclenchant le syndrome est dans ces conditions de 0,01 $cm^3/g$, soit 100 mg/kg d'acide acétique.
Le produit étudié est administré par voie buccale une demi-heure avant l'injection d'acide acétique, les souris étant à jeun depuis la veille de l'expérience.
Les étirements sont observés et comptés pour chaque souris, pendant une période d'observation de quinze minutes commençant 5 minutes après l'injection d'acide acétique.
Les résultats sont exprimés au moyen de la $DA_{50}$, c'est-à-dire la dose qui permet d'obtenir une diminution de 50 % du nombre des étirements par rapport aux animaux témoins.
La $DA_{50}$ du produit de l'exemple 5 est de 70 mg/kg.

**Revendications**

**Revendications pour les Etats contractants suivants : AT, BE, CH, DE, FR, GB, IT, LI, LU, NL, SE**

**1.** Composés de formule (I) :

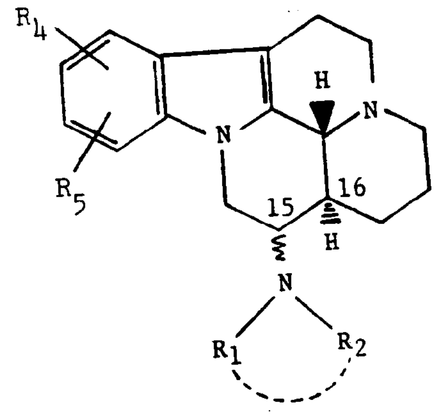

(I)

dans laquelle $R_1$ et $R_2$, identiques ou différents, représentent un atome d'hydrogène, un radical alkyle renfermant de 1 à 5 atomes de carbone, un radical $-(CH_2)_n-OH$, dans lequel n est compris entre 2 et 5, un radical aryle ou arylalkyle renfermant de 7 à 12 atomes de carbone, ces deux radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, un radical

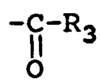

dans lequel $R_3$ est un radical alkyle renfermant de 1 à 5 atomes de carbone, un radical aryle ou arylalkyle renfermant de 7 à 12 atomes de carbone, ces deux radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, $R_1$ et $R_2$ ne pouvant toutefois pas représenter tous deux un radical aryle, ou $R_1$ et $R_2$ forment avec l'atome d'azote auquel ils sont liés un radical hétérocyclique saturé ou insaturé pouvant renfermer un second hétéroatome choisi parmi les atomes d'oxygène, de soufre et d'azote, ce dernier étant éventuellement substitué par un radical alkyle renfermant de 1 à 5 atomes de carbone, un radical aryle, un radical arylalkyle renfermant de 7 à 12 atomes de carbone, ces radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, $R_4$et $R_5$, identiques ou différents, représentent un atome d'hydrogène, un atome d'halogène, un radical alkyle ou alcoxy renfermant de 1 à 5 atomes de carbone, un radical hydroxy, trifluorométhyle ou nitro, lesdits produits de formule (I) étant sous toutes les formes énantiomères et diastéréoisomères possibles et sous forme de sels d'addition avec les acides.

2. Composés de formule (I), tels que définis à la revendication 1, caractérisés en ce que $R_1$ et $R_2$, identiques ou différents, représentent un atome d'hydrogène, un radical méthyle ou éthyle, un radical

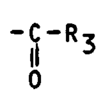

dans lequel $R_3$ est un radical méthyle ou éthyle, phényle, benzyle éventuellement substitué par un ou plusieurs atomes de chlore, un radical $-CH_2-CH_2-OH$ ou $R_1$ et $R_2$ forment avec l'atome d'azote un radical pyrrolidinyle, morpholinyle ou pipérazinyle dont l'atome d'azote est substitué par un radical méthyle ou phényle, $R_4$ et $R_5$ représentent un atome d'hydrogène ou $R_4$ représente un radical nitro en position 9 ou 11 et $R_5$ représente un atome d'hydrogène et leurs sels d'addition avec les acides.

3. La [(±) (15alpha, 16alpha)] 14,15-dihydro N-méthyl 20,21- dinoréburnaménin-15-amine et ses sels d'addition avec les acides.

4. La [(±) (15alpha, 16alpha)] 14,15-dihydro 20,21-dinoréburnaménin-15-amine et ses sels d'addition avec les acides.

**5.** Le [(±) (15alpha, 16alpha)] 2-[(14,15-dihydro 20,21-dinoréburnaménin-15-yl) méthylamino] éthanol et ses sels d'addition avec les acides.

**6.** Procédé de préparation des composés de formule (I), telle que définie à la revendication 1, caractérisé en ce que l'on soumet un composé de formule (II) :

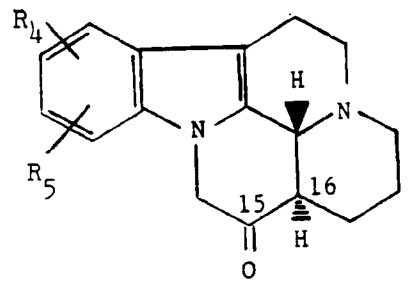

(II)

dans laquelle $R_4$ et $R_5$ ont la signification indiquée précédemment

- soit à l'action d'une amine aliphatique de formule ($III_A$) :

$H_2NR'_1$ ($III_A$)

dans laquelle $R'_1$ a la signification précédente de $R_1$, sauf hydrogène, pour obtenir un composé de formule (IV) :

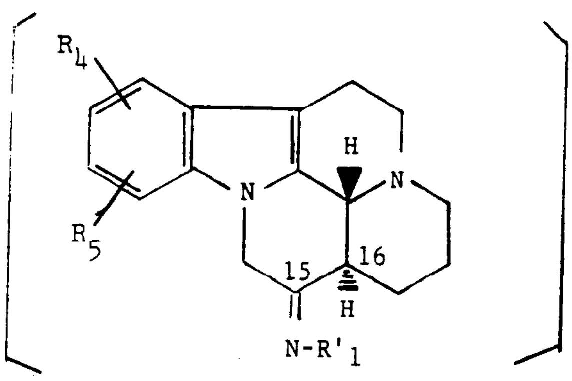

(IV)

que l'on réduit pour obtenir un composé de formule ($I_{A_1}$) :

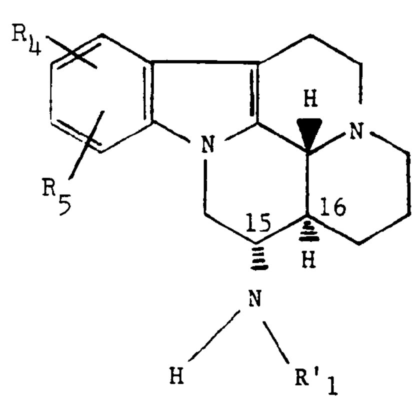

($I_{A_1}$)

dans laquelle $R'_1$ , $R_4$ et $R_5$ ont la signification déjà indiquée,

- soit à l'action d'une amine hétérocyclique de formule ($III_B$) :

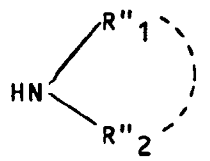

(III$_B$)

dans laquelle R″$_1$ et R″$_2$ forment avec l'atome d'azote auquel ils sont liés les mêmes amines hétérocycliques que celles données précédemment pour R$_1$ et R$_2$, pour obtenir un composé de formule (V) :

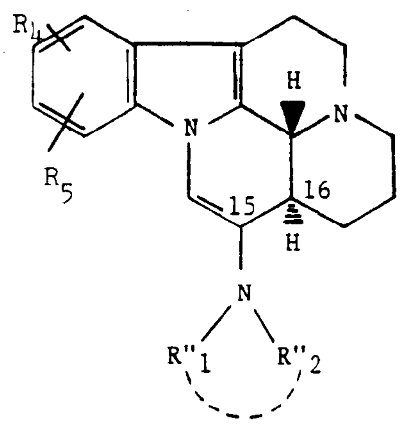

(V)

que l'on réduit pour obtenir un composé de formule ($I_{A_2}$) :

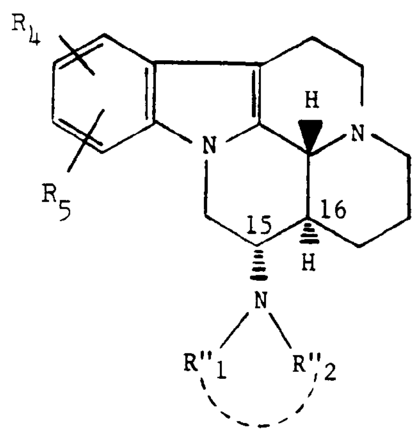

($I_{A_2}$)

dans laquelle R″$_1$ , R″$_2$, R$_4$ et R$_5$ ont les valeurs données précédemment
- soit à l'action d'un agent réducteur pour obtenir un composé de formule (VI) :

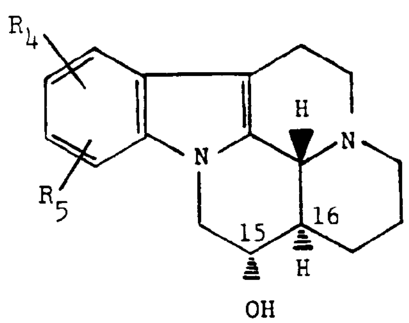

(VI)

dont on active la fonction hydroxyle et que l'on soumet
- soit à l'action d'une amine aliphatique (III$_A$) :

$H_2N\text{-}R'_1$ $(III_A)$

$R'_1$ ayant la signification précédente, pour obtenir un composé de formule $(I_{B_1})$ :

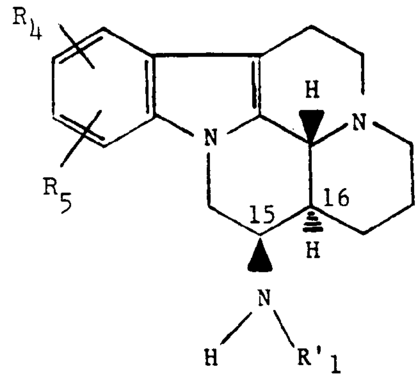

$(I_{B_1})$

$R'_1$, $R_4$ et $R_5$ ayant la signification précédente,

- soit à l'action d'une amine hétérocyclique de formule $(III_B)$ :

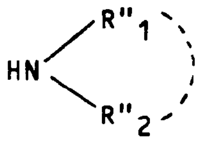

$(III_B)$

$R''_1$ et $R''_2$ ayant les significations précédentes, pour obtenir un composé de formule $(I_{B_2})$ :

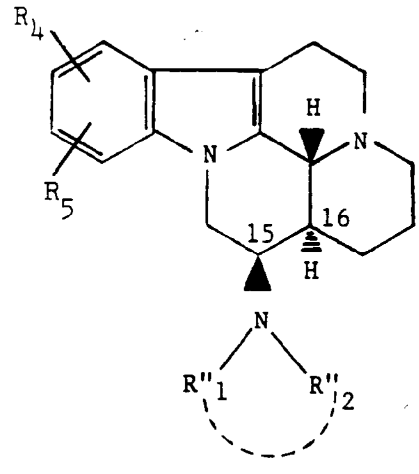

$(I_{B_2})$

dans laquelle $R''_1$ , $R''_2$, $R_4$ et $R_5$ ont les significations déjà indiquées et que, si désiré, on soumet les composés de formules $(I_{A_1})$ et $(I_{B_1})$ à l'action d'un réactif permettant de greffer sur l'amine en 15, un radical $R'_2$ ayant les mêmes valeurs données précédemment pour $R_2$, sauf hydrogène, pour obtenir les composés de formules $(I_{A_3})$ et $(I_{B_3})$ :

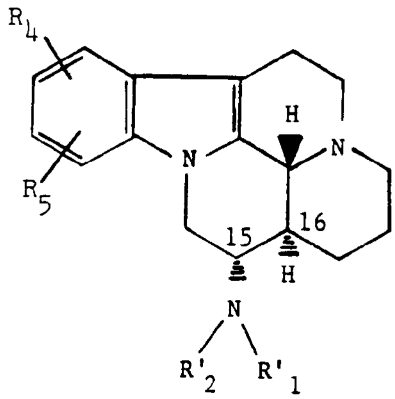

$(I_{A_3})$

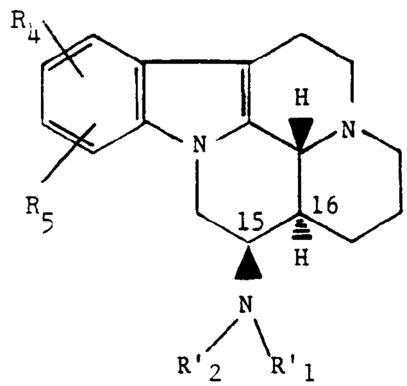

$(I_{B_3})$

dans lesquelles $R'_1$, $R'_2$, $R_4$ et $R_5$ ont les significations précédentes et que, si désiré, dans le cas où le radical $R'_2$ à introduire est un radical méthyle, on soumet les composés de formules $(I_{A_1})$ et $(I_{B_1})$ à l'action d'un formiate de formule :

$$HCO_2R$$

dans laquelle R est un radical alkyle renfermant de 1 à 4 atomes de carbone pour obtenir un composé de formule (VII) :

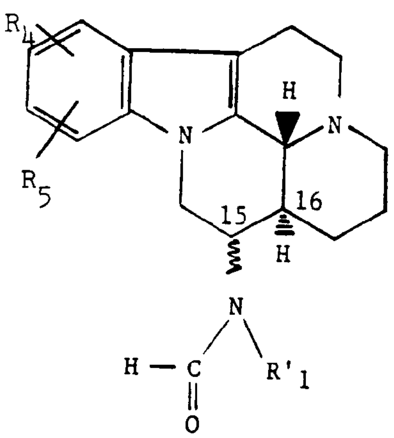

(VII)

que l'on réduit pour obtenir des composés de formules $(I_{A_3})$ et $(I_{B_3})$ dans lesquelles le radical $R'_2$ est un radical méthyle et que, si désiré, on réduit les composés de formules $(I_{A_1})$ et $(I_{B_1})$ pour lesquels $R'_1$ est un radical arylalkyle éventuellement substitué, pour obtenir les composés de formules $(I_{A_4})$ et $(I_{B_4})$ :

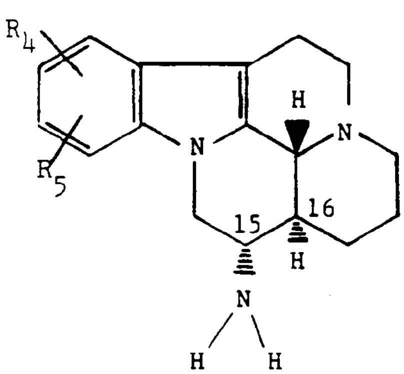

$(I_{A_4})$

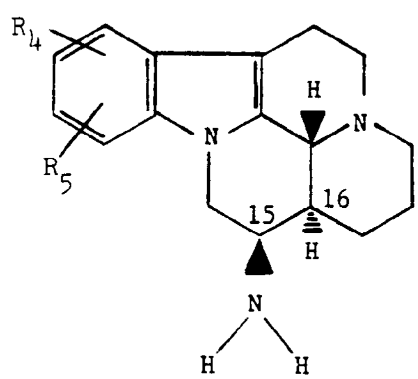

$(I_{B_4})$

dans lesquelles $R_4$ et $R_5$ ont les signification déjà indiquées, et que, si désiré, l'on soumet les composés de formules ($I_{A_4}$) et ($I_{B_4}$),

- soit à l'action d'un réactif permettant de greffer les radicaux $R'_1$ et $R'_2$, pour obtenir les composés de formules ($I_{A_1}$), ($I_{B_1}$), ($I_{A_3}$) ou ($I_{B_3}$),
- soit à l'action d'un formiate $HCO_2R$ pour obtenir un composé de formule (VIII) :

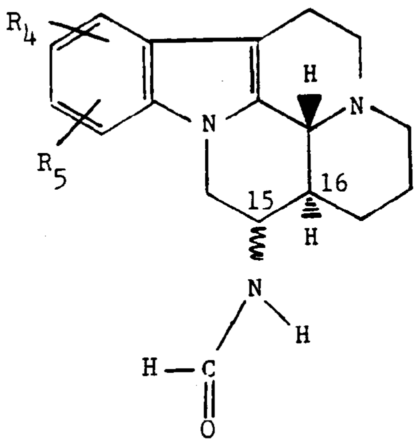

(VIII)

que l'on réduit pour obtenir un composé de formules ($I_{A_1}$) et ($I_{B_1}$) dans lesquelles le radical $R'_1$ est un radical méthyle, et traite, si désiré, tous les produits de formule (I) ainsi obtenus par un acide minéral ou organique, pour en former les sels.

7. A titre de médicaments, les composés tels que définis par la formule (I) de la revendication 1, lesdits produits de formule (I) étant sous toutes les formes isomères possibles racémiques ou optiquement actives, ainsi que les sels d'addition avec les acides minéraux ou organiques pharmaceutiquement acceptables.

8. A titre de médicaments, les composés tels que définis à la revendication 2.

9. A titre de médicaments, l'un quelconque des composés des revendications 3, 4 et 5.

10. Les compositions pharmaceutiques contenant à titre de principe actif, l'un au moins des médicaments tels que définis à l'une quelconque des revendications 7 à 9.

11. A titre de produits industriels nouveaux, les composés de formules (IV), (V), (VII), (VIII) et ceux de formules (II) et (VI) dans lesquelles $R_4$ et $R_5$ ne représentent pas chacun un atome d'hydrogène.

**Revendications pour l'Etat contractant suivant : ES**

1. Procédé pour préparer les composés de formule (I):

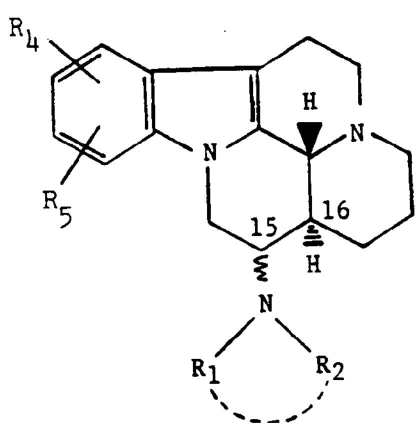

(I)

dans laquelle $R_1$ et $R_2$, identiques ou différents, représentent un atome d'hydrogène, un radical alkyle renfermant de 1 à 5 atomes de carbone, un radical $-(CH_2)_n-OH$, dans lequel n est compris entre 2 et 5, un radical aryle ou arylalkyle renfermant de 7 à 12 atomes de carbone, ces deux radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, un radical

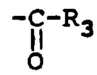

dans lequel $R_3$ est un radical alkyle renfermant de 1 à 5 atomes de carbone, un radical aryle ou arylalkyle renfermant de 7 à 12 atomes de carbone, ces deux radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, $R_1$ et $R_2$ ne pouvant toutefois pas représenter tous deux un radical aryle, ou $R_1$ et $R_2$ forment avec l'atome d'azote auquel ils sont liés un radical hétérocyclique saturé ou insaturé pouvant renfermer un second hétéroatome choisi parmi les atomes d'oxygène, de soufre et d'azote, ce dernier étant éventuellement substitué par un radical alkyle renfermant de 1 à 5 atomes de carbone, un radical aryle, un radical arylalkyle renfermant de 7 à 12 atomes de carbone, ces radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, $R_4$ et $R_5$, identiques ou différents, représentent un atome d'hydrogène, un atome d'halogène, un radical alkyle ou alcoxy renfermant de 1 à 5 atomes de carbone, un radical hydroxy, trifluorométhyle ou nitro, lesdits produits de formule (I) étant sous toutes les formes énantiomères et diastéréoisomères possibles et sous forme de sels d'addition avec les acides, caractérisé en ce que l'on soumet un composé de formule (II) :

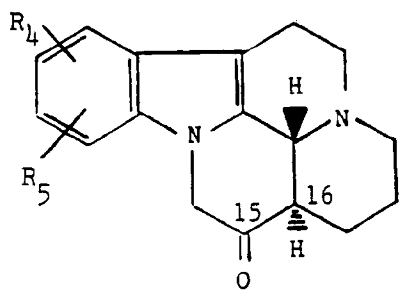

(II)

dans laquelle $R_4$ et $R_5$ ont la signification indiquée précédemment

- soit à l'action d'une amine aliphatique de formule ($III_A$) :

$H_2NR'_1$ ($III_A$)

dans laquelle $R'_1$ a la signification précédente de $R_1$, sauf hydrogène, pour obtenir un composé de formule (IV) :

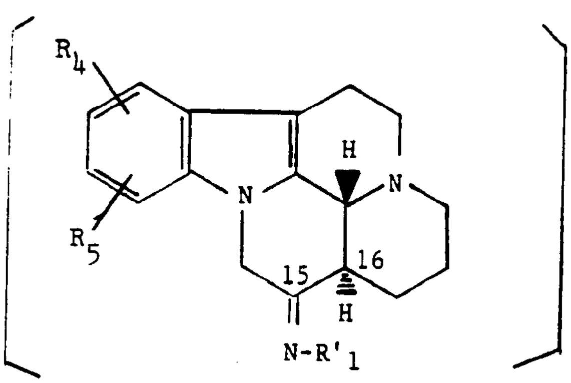

(IV)

que l'on réduit pour obtenir un composé de formule ($I_{A_1}$) :

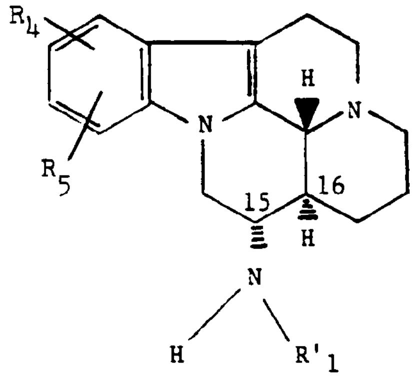

(I$_{A_1}$)

dans laquelle R'$_1$, $R_4$ et $R_5$ ont la signification déjà indiquée,
- soit à l'action d'une amine hétérocyclique de formule ($III_B$) :

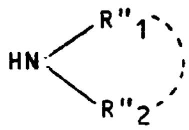

($III_B$)

dans laquelle R"$_1$ et R"$_2$ forment avec l'atome d'azote auquel ils sont liés les mêmes amines hétérocycliques que celles données précédemment pour $R_1$ et $R_2$, pour obtenir un composé de formule (V) :

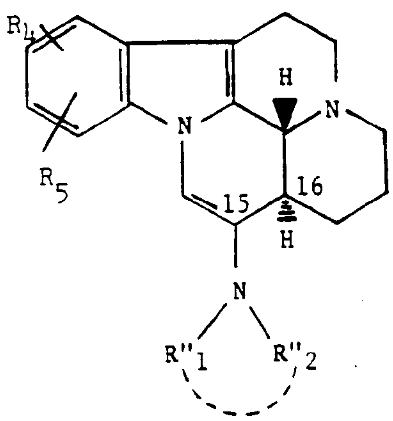

(V)

que l'on réduit pour obtenir un composé de formule ($I_{A_2}$) :

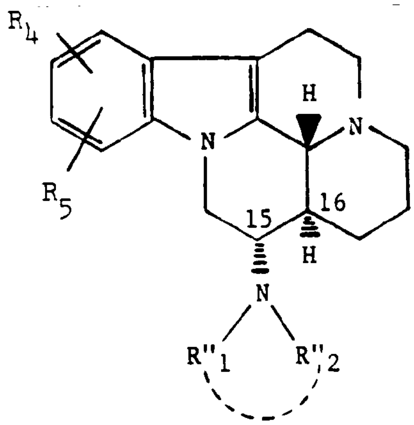

($I_{A_2}$)

dans laquelle R″$_1$, R″$_2$, $R_4$ et $R_5$ ont les valeurs données précédemment
- soit à l'action d'un agent réducteur pour obtenir un composé de formule (VI) :

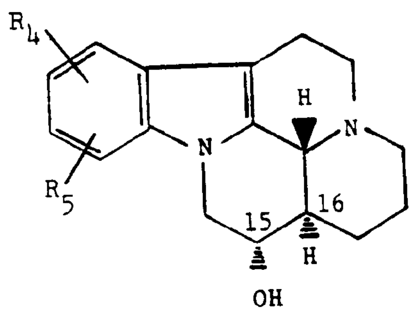

(VI)

dont on active la fonction hydroxyle et que l'on soumet
- soit à l'action d'une amine aliphatique ($III_A$) :

$H_2N\text{-}R'_1$ ($III_A$)

$R'_1$ ayant la signification précédente, pour obtenir un composé de formule ($I_{B_1}$) :

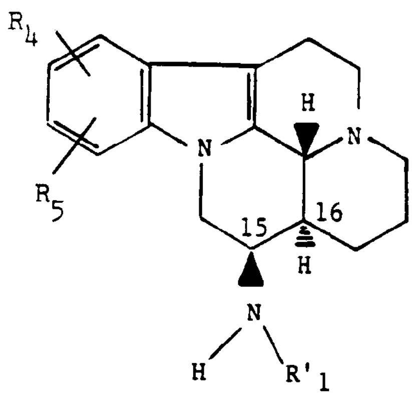

($I_{B_1}$)

$R'_1$, $R_4$ et $R_5$ ayant la signification précédente,
- soit à l'action d'une amine hétérocyclique de formule ($III_B$) :

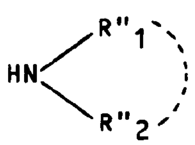

($III_B$)

R″$_1$ et R″$_2$ ayant les significations précédentes, pour obtenir un composé de formule ($I_{B_2}$) :

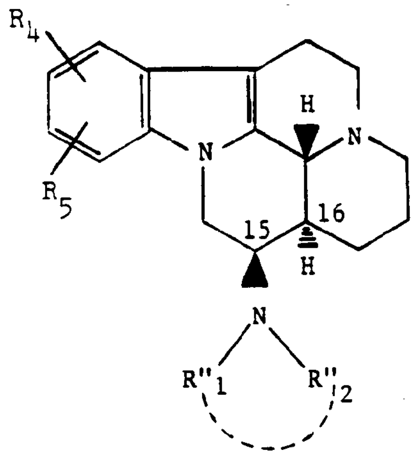

$(I_{B_2})$

dans laquelle $R''_1$, $R''_2$, $R_4$ et $R_5$ ont les significations déjà indiquées et que, si désiré, on soumet les composés de formules $(I_{A_1})$ et $(I_{B_1})$ à l'action d'un réactif permettant de greffer sur l'amine en 15, un radical $R'_2$ ayant les mêmes valeurs données précédemment pour $R_2$, sauf hydrogène, pour obtenir les composés de formules $(I_{A_3})$ et $(I_{B_3})$ :

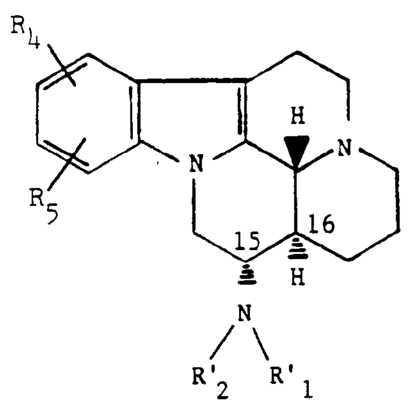

$(I_{A_3})$

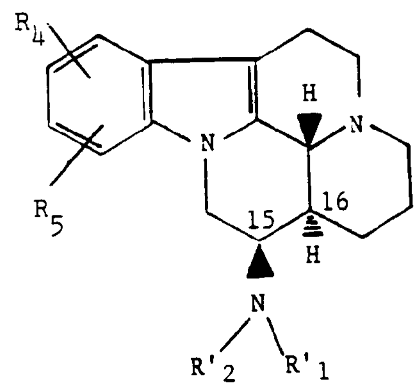

$(I_{B_3})$

dans lesquelles $R'_1$, $R'_2$, $R_4$ et $R_5$ ont les significations précédentes et que, si désiré, dans le cas où le radical $R'_2$ à introduire est un radical méthyle, on soumet les composés de formules $(I_{A_1})$ et $(I_{B_1})$ à l'action d'un formiate de formule :

$$HCO_2R$$

dans laquelle R est un radical alkyle renfermant de 1 à 4 atomes de carbone pour obtenir un composé de formule (VII) :

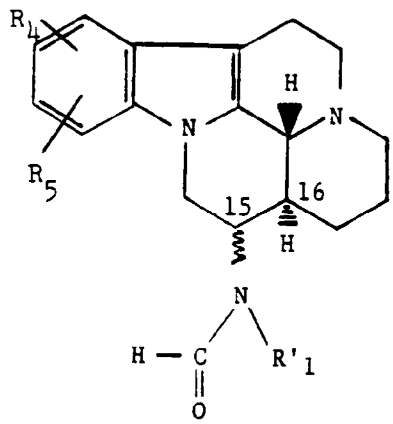

(VII)

que l'on réduit pour obtenir des composés de formules $(I_{A_3})$ et $(I_{B_3})$ dans lesquelles le radical $R'_2$ est un radical méthyle et que, si désiré, on réduit les composés de formules $(I_{A_1})$ et $(I_{B_1})$ pour lesquels $R'_1$ est un radical arylalkyle éventuellement substitué, pour obtenir les composés de formules $(I_{A_4})$ et $(I_{B_4})$ :

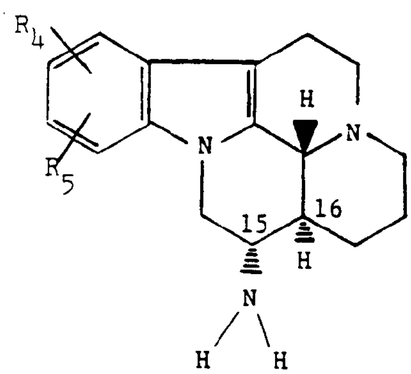

$(I_{A_4})$

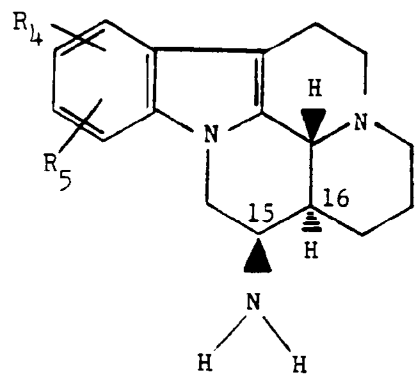

$(I_{B_4})$

dans lesquelles $R_4$ et $R_5$ ont les signification déjà indiquées, et que, si désiré, l'on soumet les composés de formules $(I_{A_4})$ et $(I_{B_4})$,

- soit à l'action d'un réactif permettant de greffer les radicaux $R'_1$ et $R'_2$, pour obtenir les composés de formules $(I_{A_1})$, $(I_{B_1})$, $(I_{A_3})$ ou $(I_{B_3})$,
- soit à l'action d'un formiate $HCO_2R$ pour obtenir un composé de formule (VIII) :

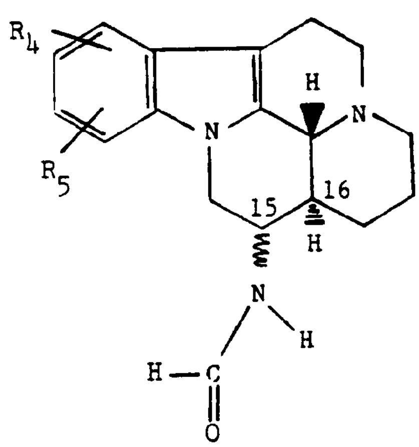

(VIII)

que l'on réduit pour obtenir un composé de formules $(I_{A_1})$ et $(I_{B_1})$ dans lesquelles le radical $R'_1$ est un

radical méthyle, et traite, si désiré, tous les produits de formule (I) ainsi obtenus par un acide minéral ou organique, pour en former les sels.

2. Procédé selon la revendication 1, caractérisé en ce que le produit de départ de formule ($III_A$) ou ($III_B$) et les réactifs que l'on fait réagir sur les composés de formule ($I_{A_1}$), ($I_{B_1}$), ($I_{A_4}$), ($I_{B_4}$) sont tels que l'on obtient un produit de formule (I) dans laquelle $R_1$ et $R_2$ indentiques ou différents représentent un radical méthyle ou éthyle, un radical

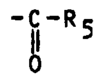

dans lequel $R_5$ est un radical méthyle ou éthyle, phényle, benzyle éventuellement substitué par un ou plusieurs atomes de chlore, un radical $-CH_2-CH_2-OH$ ou $R_1$ et $R_2$ forment avec l'atome d'azote un radical pyrrolidinyle, morpholinyle ou pipérazinyle dont l'atome d'azote est substitué par un radical méthyle ou phényle, $R_4$ et $R_5$ identiques ou différents représentent un atome d'hydrogène ou $R_4$ représente un radical nitro en position 9 ou 11 et $R_5$ représente un atome d'hydrogène.

3. Procédé selon la revendication 1, caractérisé en ce que l'on prépare :
- la [(±) (15alpha, 16alpha)] 14,15-dihydro N-méthyl 20,21- dinoréburnaménin-15-amine,
- la [(±) (15alpha, 16alpha)] 14,15-dihydro 20,21-dinoréburnaménin-15-amine,
- le [(±) (15alpha, 16alpha)] 2-[(14,15-dihydro 20,21-dinoréburnaménin-15-yl) méthylamino] éthanol,
et leurs sels d'addition avec les acides.

**Revendications pour l'Etat contractant suivant : GR**

1. Procédé pour préparer les composés de formule (I):

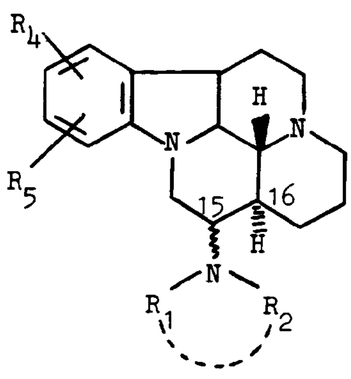

(I)

dans laquelle $R_1$ et $R_2$, identiques ou différents, représentent un atome d'hydrogène, un radical alkyle renfermant de 1 à 5 atomes de carbone, un radical $-(CH_2)_n-OH$, dans lequel n est compris entre 2 et 5, un radical aryle ou arylalkyle renfermant de 7 à 12 atomes de carbone, ces deux radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, un radical

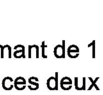

dans lequel $R_3$ est un radical alkyle renfermant de 1 à 5 atomes de carbone, un radical aryle ou arylalkyle renfermant de 7 à 12 atomes de carbone, ces deux radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, $R_1$ et $R_2$ ne pouvant toutefois pas représenter tous deux un radical aryle, ou $R_1$ et $R_2$ forment avec l'atome d'azote auquel ils sont liés un radical hétérocyclique saturé ou insaturé pouvant renfermer un second hétéroatome choisi parmi les atomes d'oxygène, de soufre et

d'azote, ce dernier étant éventuellement substitué par un radical alkyle renfermant de 1 à 5 atomes de carbone, un radical aryle, un radical arylalkyle renfermant de 7 à 12 atomes de carbone, ces radicaux étant éventuellement substitués par 1, 2 ou 3 radicaux choisis parmi le groupe formé par les atomes d'halogène, les radicaux méthyle, éthyle, méthoxy, éthoxy, trifluorométhyle, amino et nitro, $R_4$ et $R_5$, identiques ou différents, représentent un atome d'hydrogène, un atome d'halogène, un radical alkyle ou alcoxy renfermant de 1 à 5 atomes de carbone, un radical hydroxy, trifluorométhyle ou nitro, lesdits produits de formule (I) étant sous toutes les formes énantiomères et diastéréoisomères possibles et sous forme de sels d'addition avec les acides, caractérisé en ce que l'on soumet un composé de formule (II) :

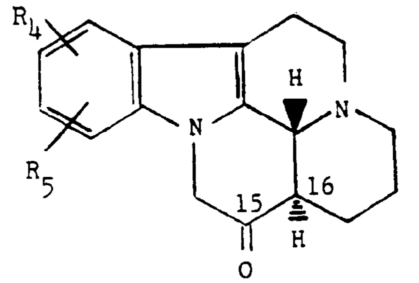

(II)

dans laquelle $R_4$ et $R_5$ ont la signification indiquée précédemment

- soit à l'action d'une amine aliphatique de formule ($III_A$) ;

$H_2NR'_1$ ($III_A$)

dans laquelle $R'_1$ a la signification précédente de $R_1$, sauf hydrogène, pour obtenir un composé de formule (IV) :

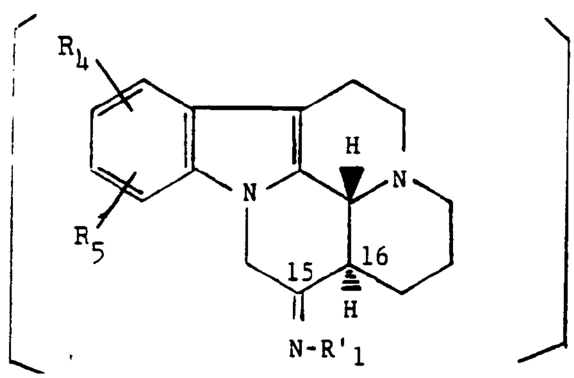

(IV)

que l'on réduit pour obtenir un composé de formule ($I_{A_1}$) :

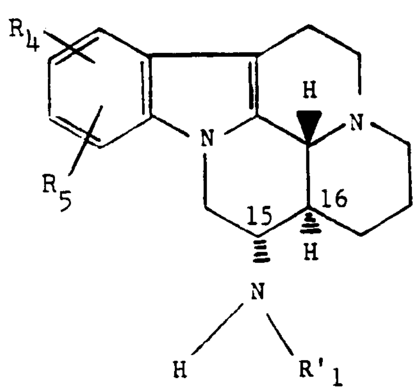

($I_{A_1}$)

dans laquelle $R'_1$, $R_4$ et $R_5$ ont la signification déjà indiquée,

- soit à l'action d'une amine hétérocyclique de formule ($III_B$) :

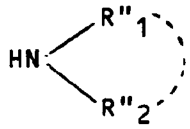

(III$_B$)

dans laquelle R″$_1$ et R″$_2$ forment avec l'atome d'azote auquel ils sont liés les mêmes amines hétérocycliques que celles données précédemment pour $R_1$ et $R_2$, pour obtenir un composé de formule (V) :

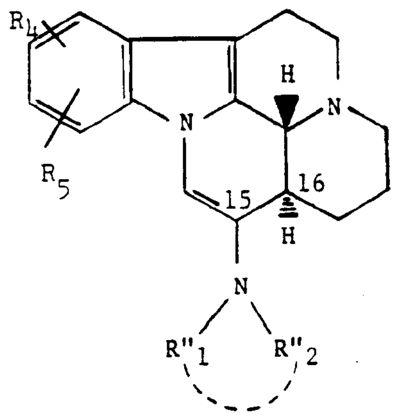

(V)

que l'on réduit pour obtenir un composé de formule ($I_{A_2}$) :

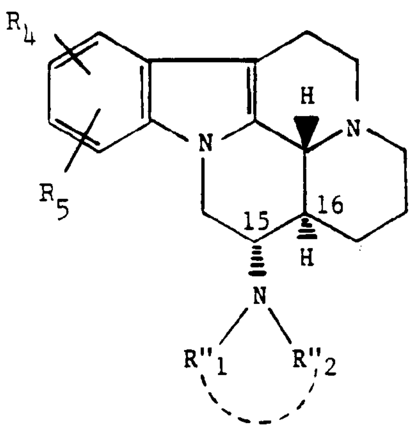

($I_{A_2}$)

dans laquelle R″$_1$, R″$_2$, $R_4$ et $R_5$ ont les valeurs données précédemment
- soit à l'action d'un agent réducteur pour obtenir un composé de formule (VI) :

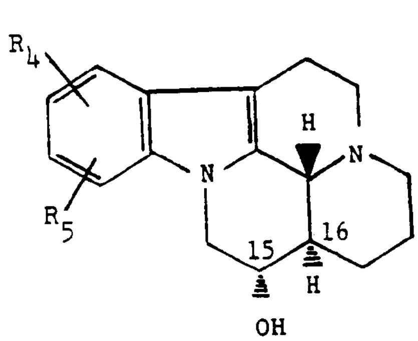

(VI)

dont on active la fonction hydroxyle et que l'on soumet
- soit à l'action d'une amine aliphatique (III$_A$) :

$H_2N\text{-}R'_1$ $(III_A)$

$R'_1$ ayant la signification précédente, pour obtenir un composé de formule $(I_{B_1})$:

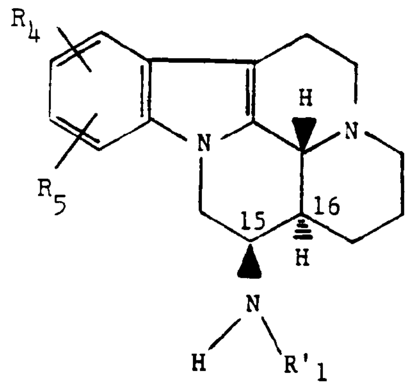

$(I_{B_1})$

$R'_1$, $R_4$ et $R_5$ ayant la signification précédente,

- soit à l'action d'une amine hétérocyclique de formule $(III_B)$ :

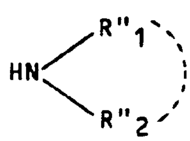

$(III_B)$

$R''_1$ et $R''_2$ ayant les significations précédentes, pour obtenir un composé de formule $(I_{B_2})$ :

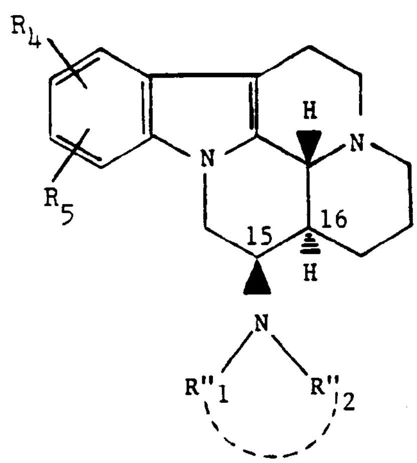

$(I_{B_2})$

dans laquelle $R''_1$, $R''_2$, $R_4$ et $R_5$ ont les significations déjà indiquées et que, si désiré, on soumet les composés de formules $(I_{A_1})$ et $(I_{B_1})$ à l'action d'un réactif permettant de greffer sur l'amine en 15, un radical $R'_2$ ayant les mêmes valeurs données précédemment pour $R_2$, sauf hydrogène, pour obtenir les composés de formules $(I_{A_3})$ et $(I_{B_3})$ :

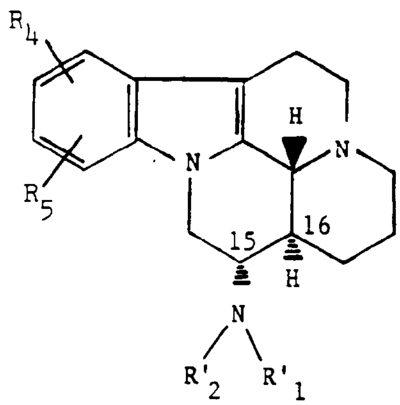

$(I_{A_3})$

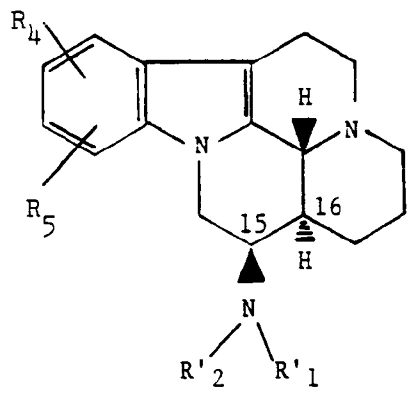

$(I_{B_3})$

dans lesquelles $R'_1$, $R'_2$, $R_4$ et $R_5$ ont les significations précédentes et que, si désiré, dans le cas où le radical $R'_2$ à introduire est un radical méthyle, on soumet les composés de formules $(I_{A_1})$ et $(I_{B_1})$ à l'action d'un formiate de formule :

$$HCO_2R$$

dans laquelle R est un radical alkyle renfermant de 1 à 4 atomes de carbone pour obtenir un composé de formule (VII):

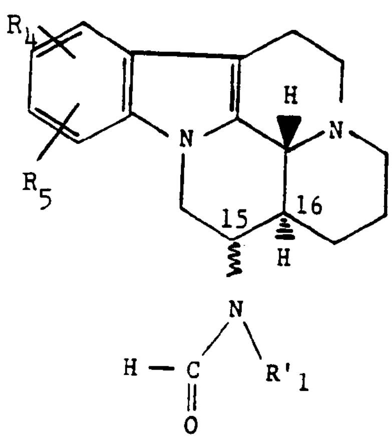

(VII)

que l'on réduit pour obtenir des composés de formules $(I_{A_3})$ et $(I_{B_3})$ dans lesquelles le radical $R'_2$ est un radical méthyle et que, si désiré, on réduit les composés de formules $(I_{A_1})$ et $(I_{B_1})$ pour lesquels $R'_1$ est un radical arylalkyle éventuellement substitué, pour obtenir les composés de formules $(I_{A_4})$ et $(I_{B_4})$ :

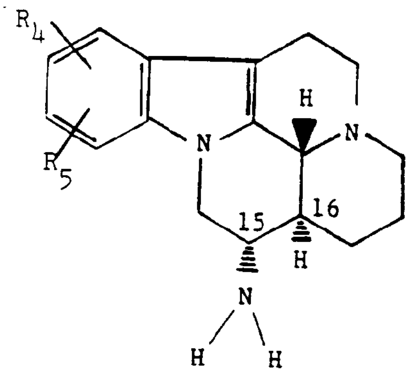

$(I_{A_4})$

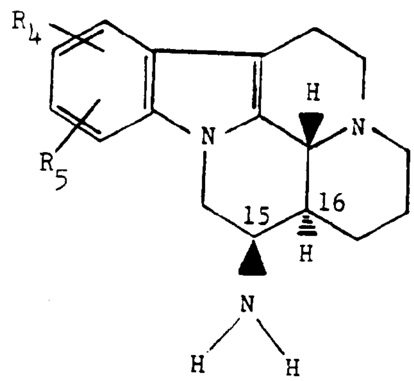

$(I_{B_4})$

dans lesquelles $R_4$ et $R_5$ ont les signification déjà indiquées, et que, si désiré, l'on soumet les composés de formules $(I_{A_4})$ et $(I_{B_4})$,

- soit à l'action d'un réactif permettant de greffer les radicaux $R'_1$ et $R'_2$, pour obtenir les composés de formules $(I_{A_1})$, $(I_{B_1})$, $(I_{A_3})$ ou $(I_{B_3})$,
- soit à l'action d'un formiate $HCO_2R$ pour obtenir un composé de formule (VIII) :

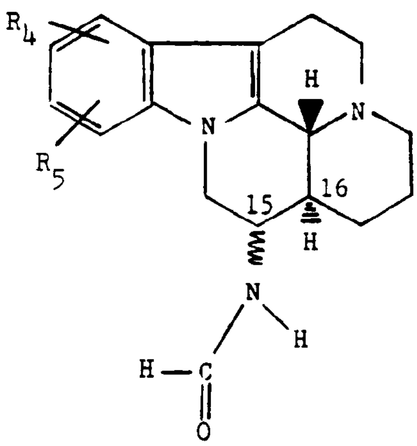

(VIII)

que l'on réduit pour obtenir un composé de formules $(I_{A_1})$ et $(I_{B_1})$ dans lesquelles le radical $R'_1$ est un radical méthyle, et traite, si désiré, tous les produits de formule (I) ainsi obtenus par un acide minéral ou organique, pour en former les sels.

**2.** Procédé selon la revendication 1, caractérisé en ce que le produit de départ de formule $(III_A)$ ou $(III_B)$ et les réactifs que l'on fait réagir sur les composés de formule $(I_{A_1})$, $(I_{B_1})$, $(I_{A_4})$, $(I_{B_4})$ sont tels que l'on obtient un produit de formule (I) dans laquelle $R_1$ et $R_2$ indentiques ou différents représentent un radical méthyle ou éthyle, un radical

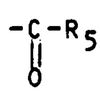

dans lequel $R_5$ est un radical méthyle ou éthyle, phényle, benzyle éventuellement substitué par un ou plusieurs atomes de chlore, un radical $-CH_2-CH_2-OH$ ou $R_1$ et $R_2$ forment avec l'atome d'azote un radical pyrrolidinyle, morpholinyle ou pipérazinyle dont l'atome d'azote est substitué par un radical méthyle ou phényle, $R_4$ et $R_5$ identiques ou différents représentent un atome d'hydrogène ou $R_4$ représente un radical nitro en position 9 ou 11 et $R_5$ représente un atome d'hydrogène.

3. Procédé selon la revendication 1, caractérisé en ce que l'on prépare :
   - la [(±) (15alpha, 16alpha)] 14,15-dihydro N-méthyl 20,21- dinoréburnaménin-15-amine,
   - la [(±) (15alpha, 16alpha)] 14,15-dihydro 20,21-dinoréburnaménin-15-amine,
   - le [(±) (15alpha, 16alpha)] 2-[(14,15-dihydro 20,21-dinoréburnaménin-15-yl) méthylamino] éthanol,

   et leurs sels d'addition avec les acides.

4. Procédé de prépartion de compositions pharmaceutiques, caractérisé en ce que l'on met à titre de principe actif, l'un au moins des composés de formule (I) tels que définis à la revendication 1 ou l'un au moins de leurs sels d'addition avec les acides minéraux ou organiques pharmaceutiquement acceptables, sous une forme destinée à cet usage.

5. Procédé selon la revendication 4, caractérisé en ce que le composé de formule (I) est choisi parmi :
   - la [(±) (15alpha, 16alpha)] 14,15-dihydro N-méthyl 20,21- dinoréburnaménin-15-amine,
   - la [(±) (15alpha, 16alpha)] 14,15-dihydro 20,21-dinoréburnaménin-15-amine,
   - le [(±) (15alpha, 16alpha)] 2-[(14,15-dihydro 20,21-dinoréburnaménin-15-yl) méthylamino] éthanol,

   et leurs sels d'addition avec les acides.

6. A titre de produits industriels nouveaux, les composés de formules (IV), (V), (VII), (VIII) et ceux de formules (II) et (VI) dans lesquelles $R_4$ et $R_5$ ne représentent pas chacun un atome d'hydrogène.

## Patentansprüche

### Patentansprüche für folgende Vertragsstaaten : AT, BE, CH, DE, FR, GB, IT, LI, LU, NL, SE

1. Verbindungen der Formel (I)

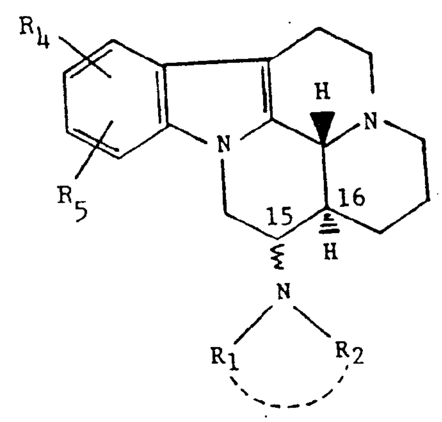

(I)

worin $R_1$ und $R_2$ die identisch oder verschieden sind, bedeuten ein Wasserstoffatom, einen Alkylrest mit 1 bis 5 Kohlenstoffatomen, einen Rest $-(CH_2)_n-OH$, wobei n zwischen 2 und 5 beträgt, einen Aryl- oder Arylalkylrest mit 7 bis 12 Kohlenstoffatomen, wobei diese beiden Reste gegebenenfalls durch 1, 2 oder 3 Reste substituiert sein können, ausgewählt aus der Gruppe bestehend aus Wasserstoffatomen, Methyl-, Ethyl-, Methoxy-, Ethoxy-, Trifluormethyl-, Amino- und Nitroresten, einen Rest

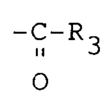

worin $R_3$ ein Alkylrest mit 1 bis 5 Kohlenstoffatomen , ein Aryl- oder Arylalkylrest mit 7 bis 12 Kohlenstoffatomen ist, wobei diese beiden Reste gegebenenfalls substituiert sind durch 1, 2 oder 3 Reste, ausgewählt aus der Gruppe gebildet von den Halogenatomen, Methyl-, Ethyl-, Methoxy-, Ethoxy-, Trifluormethyl-, Amino- und Nitroresten, wobei $R_1$ und $R_2$ nicht beide einen Arylrest bedeuten können, oder
$R_1$ und $R_2$ bilden mit dem Stickstoffatom, an das sie gebunden sind, einen gesättigten oder ungesättigten heterocyclischen Rest, der ein zweites Heteroatom enthalten kann, ausgewählt aus den Sauerstoff-, Schwefel- und Stickstoffatomen, wobei letzterer gegebenenfalls substituiert sein kann durch einen Alkylrest mit 1 bis 5 Kohlenstoffatomen, einen Arylrest, einen Arylalkylrest mit 7 bis 12 Kohlenstoffatomen, und diese Reste gegebenenfalls substituiert sind durch 1, 2 oder 3 Reste, ausgewählt aus der Gruppe gebildet von den Halogenatomen, den Methyl-, Ethyl-, Methoxy-, Ethoxy-, Trifluormethyl-, Amino- und Nitroresten, $R_4$ und $R_5$, die identisch oder verschieden sind, bedeuten ein Wasserstoffatom, ein Halogenatom, einen Alkyl- oder Alkoxyrest mit 1 bis 5 Kohlenstoffatomen, einen Hydroxy-, Trifluormethyl- oder Nitrorest, wobei diese Produkte der Formel (I) unter allen möglichen enantiomeren und diastereoisomeren Formen und in Form der Additionssalze mit Säuren vorliegen.

2. Verbindungen der Formel (I), wie sie in Anspruch 1 definiert sind, dadurch gekennzeichnet, daß $R_1$ und $R_2$, die identisch oder verschieden sind, ein Wasserstoffatom, einen Methyl- oder Ethylrest, einen Rest

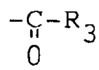

bedeuten, worin $R_3$ einen Methyl- oder Ethylrest, Phenyl, Benzyl, gegebenenfalls substituiert durch ein oder mehrere Chloratome, einen $-CH_2-CH_2-OH$-Rest, oder
$R_1$ und $R_2$ bilden zusammen mit dem Stickstoffatom einen Pyrrolidinyl-, Morpholinyl- oder Piperazinylrest, dessen Stickstoffatom durch einen Methyl- oder Phenylrest, substituiert ist,
$R_4$ und $R_5$ bedeuten ein Wasserstoffatom oder $R_4$ bedeutet einen Nitrorest in 9- oder 11-Stellung und $R_5$ bedeutet ein Wasserstoffatom,
und ihre Additionssalze mit Säuren.

3. [(±)(15alpha,16alpha)]-14,15-Dihydro-N-methyl-20,21-dinoreburnamenin-15-amin und dessen Additionssalze mit Säuren.

4. [(±)(15alpha,16alpha)]-14,15-Dihydro- 20,21-dinoreburnamenin-15-amin und dessen Additionssalze mit Säuren.

5. [(±)(15alpha,16alpha)]-2-(14,15-Dihydro-20-21-dinoreburnamenin-15-yl)-methylamino ethanol und dessen Additionssalze mit Säuren

6. Verfahren zur Herstellung der Verbindungen der Formel (I), wie sie in Anspruch 1 definiert ist, dadurch gekennzeichnet, daß man eine Verbindung der Formel (II)

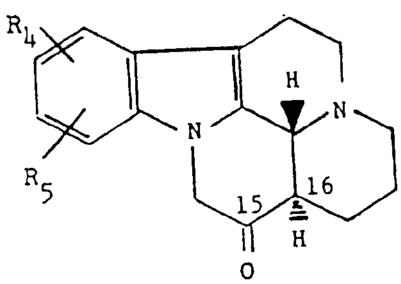

(II)

worin $R_4$ und $R_5$ die vorstehend angegebene Bedeutung haben,
- entweder der Einwirkung eines aliphatischen Amins der Formel ($III_A$):

$$H_2NR'_1 \qquad (III_A)$$

unterwirft, worin $R'_1$ die vorstehende Bedeutung von $R_1$ hat, mit Ausnahme von Wasserstoff, um eine Verbindung der Formel (IV)

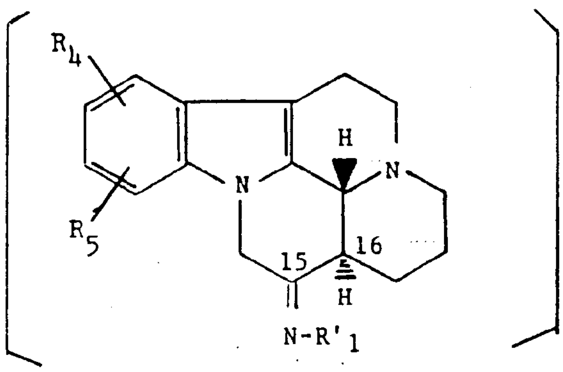

(IV)

zu erhalten, die man reduziert, um eine Verbindung der Formel ($I_{A_1}$)

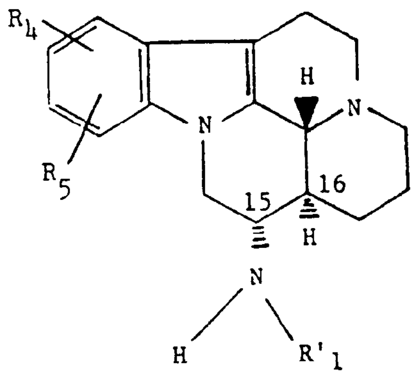

($I_{A_1}$)

zu erhalten, worin $R'_1$, $R_4$ und $R_5$ die angegebene Bedeutung haben,
- oder der Einwirkung eines heterocyclischen Amins der Formel ($III_B$):

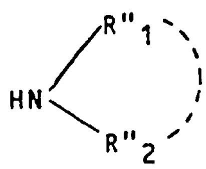

($III_B$)

unterwirft, worin $R''_1$ und $R''_2$ mit dem Stickstoffatom, an das sie gebunden sind, die gleichen heterocyclischen Amine bilden wie diejenigen wie vorstehend für $R_1$ und $R_2$ genannt sind, um eine Verbindung der Formel (V)

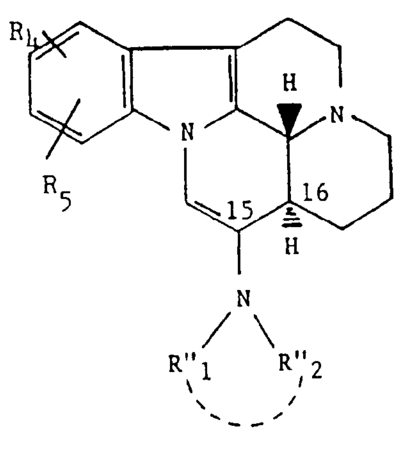

(V)

zu erhalten, die man reduziert, um eine Verbindung der Formel ($I_{A_2}$) zu erhalten:

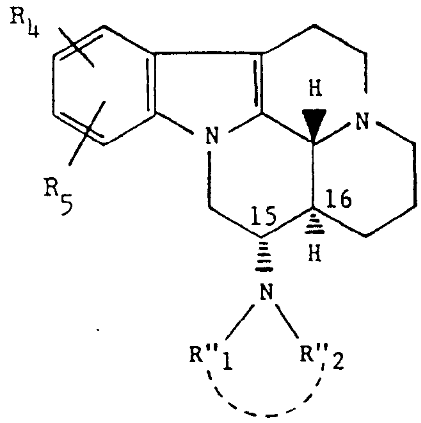

($I_{A_2}$)

worin $R''_1$, $R''_2$, $R_4$ und $R_5$ die vorstehend angegebenen Werte haben,

- oder der Einwirkung eines Reduktionsmittels unterwirft, um eine Verbindung der Formel (VI) zu erhalten,

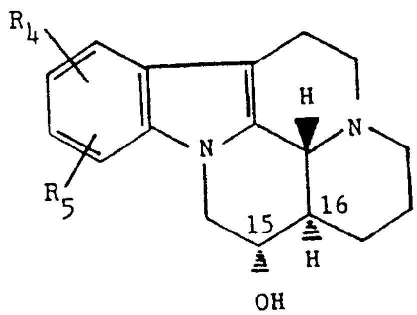

(VI)

von der man die Hydroxylfunktion aktiviert, und die man

- entweder der Einwirkung eines aliphatischen Amins ($III_A$)

$H_2N$-$R'_1$ ($III_A$)

unterwirft, wobei $R'_1$ die vorstehende Bedeutung hat, um eine Verbindung der Formel ($I_{B_1}$)

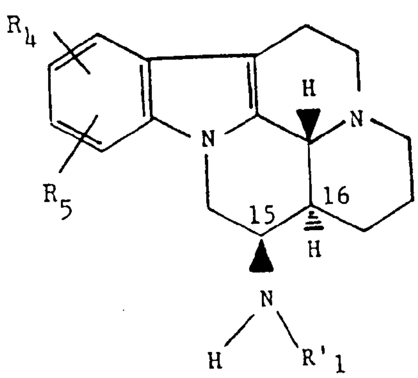

($I_{B_1}$)

zu erhalten, wobei $R'_1$, $R_4$ und $R_5$ die vorstehende Bedeutung haben,

- oder der Einwirkung eines heterocyclischen Amins der Formel ($III_B$)

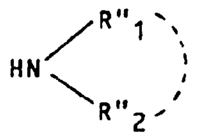

$(III_B)$

unterwirft, wobei $R''_1$ und $R''_2$ die vorstehenden Bedeutungen haben, um eine Verbindung der Formel $(I_{B_2})$

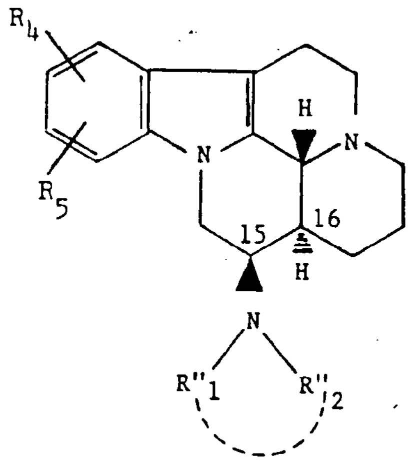

$(I_{B_2})$

zu erhalten, worin $R''_1$, $R''_2$, $R_4$ und $R_5$ die bereits angegebenen Bedeutungen haben, und daß man gewünschtenfalls die Verbindungen der Formeln $(I_{A_1})$ und $(I_{B_1})$ der Einwirkung eines Reagens unterwirft, das es ermöglicht, an dem Amin in 15-Stellung einen Rest $R'_2$ aufzupfropfen, der die gleichen Werte hat wie sie vorstehend für $R_2$ angegeben wurden, ausgenommen Wasserstoff, um die Verbindungen der Formeln $(I_{A_3})$ und $(I_{B_3})$

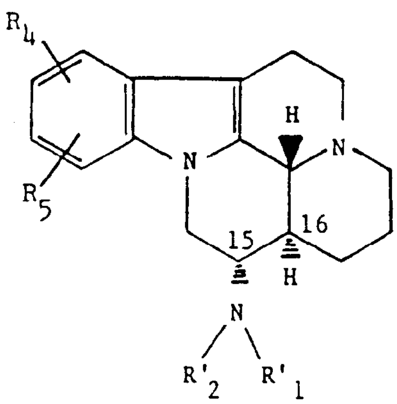

$(I_{A_3})$

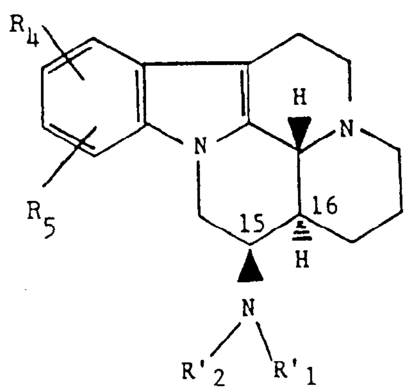

$(I_{B_3})$

zu erhalten, worin die Reste $R'_1$, $R'_2$, $R_4$ und $R_5$ die vorstehenden Bedeutungen haben, und daß man gewünschtenfalls für den Fall, daß der einzuführende Rest $R'_2$ ein Methylrest ist, die Verbindungen der Formel $(I_{A_1})$ und $(I_{B_1})$ der Einwirkung eines Formiats der Formel

$$HCO_2R$$

unterwirft, worin R ein Alkylrest mit 1 bis 4 Kohlenstoffatomen ist, um eine Verbindung der Formel (VII)

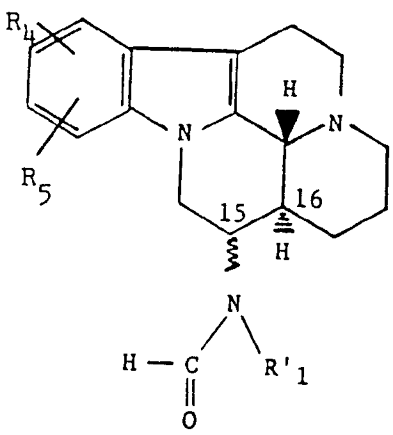

(VII)

zu erhalten, die man reduziert, um Verbindungen der Formeln ($I_{A_3}$) und ($I_{B_3}$) zu erhalten, worin der Rest $R'_2$ ein Methylrest ist, und daß man gewünschtenfalls die Verbindungen der Formeln ($I_{A_1}$) und ($I_{B_1}$), für die $R'_1$ ein gegebenenfalls substituierter Arylalkylrest ist, reduziert, um die Verbindungen der Formeln ($I_{A_4}$) und ($I_{B_4}$)

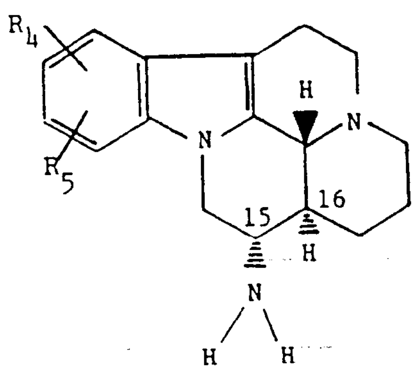

($I_{A_4}$)

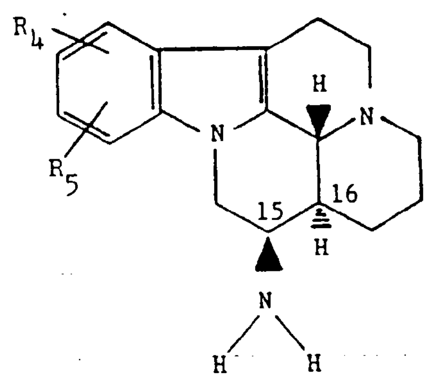

($I_{B_4}$)

worin $R_4$ und $R_5$, die bereits angegebenen Bedeutungen haben, und daß man gewünschtenfalls die Verbindungen der Formel ($I_{A_4}$) und ($I_{B_4}$)

- entweder der Einwirkung eines Reagens unterwirft, das es erlaubt, die Reste $R'_1$ und $R'_2$ aufzupfropfen, um die Verbindungen der Formeln ($I_{A_1}$) ($I_{B_1}$), ($I_{A_3}$) oder ($I_{B_3}$) zu erhalten,
- oder der Einwirkung eines Formiats $HCO_2R$ unterwirft, um eine Verbindung der Formel (VIII)

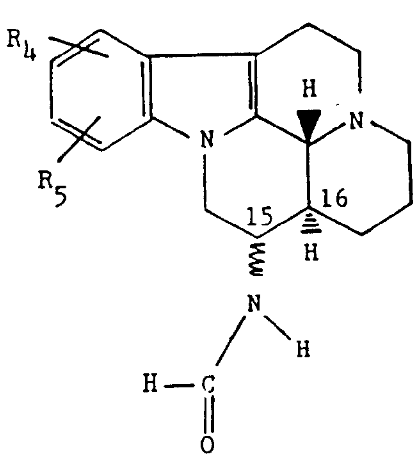

(VIII)

zu erhalten, die man reduziert, um eine Verbindung der Formeln ($I_{A_1}$) und ($I_{B_1}$) zu erhalten, worin der Rest $R'_1$ ein Methylrest ist, und daß man gewünschtenfalls alle so erhaltenen Produkte der Formel (I) mit einer Mineral- oder organischen Säure behandelt, um daraus die Salze zu bilden.

**7.** Als Arzneimittel die Verbindungen, wie sie durch Formel (I) des Anspruchs 1 definiert sind, wobei diese Produkte der Formel (I) in allen möglichen isomeren, racemischen oder optisch aktiven Formen sein können, sowie die Additionssalze mit pharmazeutisch annehmbaren Mineral- oder organischen Säuren.

**8.** Als Arzneimittel die Verbindungen, wie sie in Anspruch 2 definiert sind.

**9.** Als Arzneimittel eine der Verbindungen der Ansprüche 3, 4 und 5.

**10.** Pharmazeutische Zusammensetzungen, die als Wirkstoff mindestens eines der Arzneimittel enthalten, wie sie in einem der Ansprüche 7 bis 9 definiert sind.

**11.** Als neue industrielle Produkte die Verbindungen der Formeln (IV), (V), (VII), (VIII) und diejenigen der Formel (II) und (VI), worin $R_4$ und $R_5$ jedes nicht ein Wasserstoffatom bedeutet.

**Patentansprüche für folgenden Vertragsstaat: ES**

**1.** Verfahren zur Herstellung der Verbindungen der Formel (I)

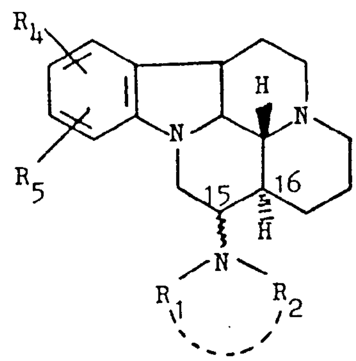

(I)

worin $R_1$ und $R_2$, die identisch oder verschieden sind, bedeuten ein Wasserstoffatom, einen Alkylrest mit 1 bis 5 Kohlenstoffatomen, einen Rest $-(CH_2)_n-OH$, wobei n zwischen 2 und 5 beträgt, einen Aryl- oder Arylalkylrest mit 7 bis 12 Kohlenstoffatomen, wobei diese beiden Reste gegebenenfalls durch 1, 2 oder 3 Reste substituiert sein können, ausgewählt aus der Gruppe gebildet durch die Halogenatome, die Methyl-, Ethyl-, Methoxy-, Ethoxy-, Trifluormethyl-, Amino- und Nitroreste, einen Rest

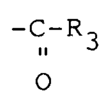

worin $R_3$ ein Alkylrest mit 1 bis 5 Kohlenstoffatomen, ein Aryl- oder Arylalkylrest mit 7 bis 12 Kohlenstoffatomen ist, wobei diese beiden Reste gegebenenfalls substituiert sind durch 1, 2 oder 3 Reste, ausgewählt aus der Gruppe gebildet von den Halogenatomen, Methyl-, Ethyl-, Methoxy-, Ethoxy-, Trifluormethyl-, Amino- und Nitroresten, wobei $R_1$ und $R_2$ nicht beide einen Arylrest bedeuten können, oder
$R_1$ und $R_2$ bilden mit dem Stickstoffatom, an das sie gebunden sind, einen gesättigten oder ungesättigten heterocyclischen Rest, der ein zweites Heteroatom enthalten kann, ausgewählt aus den Sauerstoff-, Schwefel- und Stickstoffatomen, wobei letzterer gegebenenfalls substituiert sein kann durch einen Alkylrest mit 1 bis 5 Kohlenstoffatomen, einen Arylrest, einen Arylalkylrest mit 7 bis 12 Kohlenstoffatomen, und diese Reste gegebenenfalls substituiert sind durch 1, 2 oder 3 Reste, ausgewählt aus der Gruppe gebildet von den Halogenatomen, den Methyl-, Ethyl-, Methoxy-, Ethoxy-, Trifluormethyl-, Amino- und Nitroresten,
$R_4$ und $R_5$, die identisch oder verschieden sind, bedeuten ein Wasserstoffatom, ein Halogenatom, einen Alkyl- oder Alkoxyrest mit 1 bis 5 Kohlenstoffatomen, einen Hydroxy-, Trifluormethyl- oder Nitrorest, wobei diese Produkte der Formel (I) unter allen möglichen enantiomeren und diastereoisomeren Formen

und in Form der Additionssalze mit Säuren vorliegen, dadurch gekennzeichnet, daß man eine Verbindung der Formel (II)

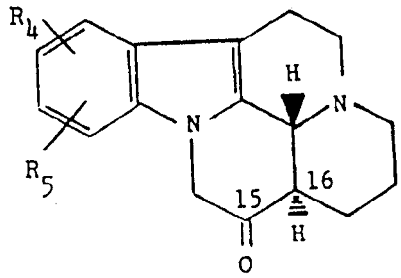

(II)

worin $R_4$ und $R_5$ die vorstehend angegebene Bedeutung haben,

- entweder der Einwirkung eines aliphatischen Amins der Formel ($III_A$):

$$H_2NR'_1 \qquad (III_A)$$

unterwirft, worin $R'_1$ die vorstehende Bedeutung von $R_1$ hat, mit Ausnahme von Wasserstoff, um eine Verbindung der Formel (IV)

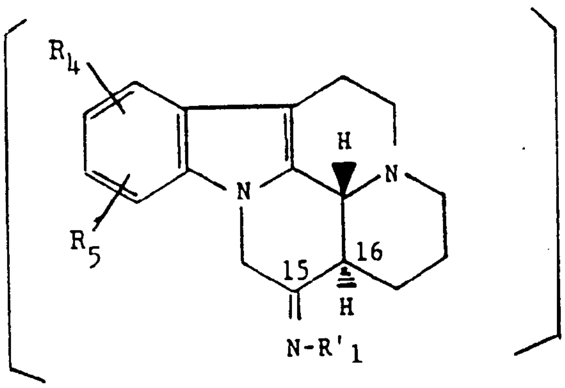

(IV)

zu erhalten, die man reduziert, um eine Verbindung der Formel ($I_{A_1}$)

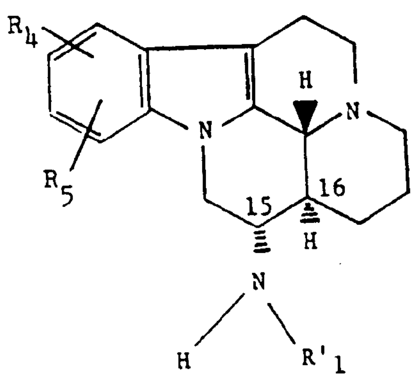

($I_{A_1}$)

zu erhalten, worin $R'_1$, $R_4$ und $R_5$ die angegebene Bedeutung haben,

- oder der Einwirkung eines heterocyclischen Amins der Formel ($III_B$):

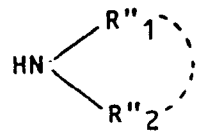

(III$_B$)

unterwirft, worin $R''_1$ und $R''_2$ mit dem Stickstoffatom, an das sie gebunden sind, die gleichen heterocyclischen Amine bilden wie diejenigen wie vorstehend für $R_1$ und $R_2$ genannt sind, um eine Verbindung der Formel (V)

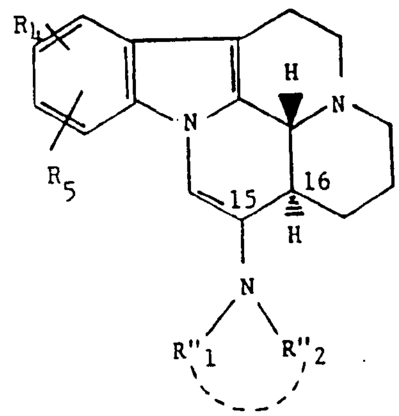

(V)

zu erhalten, die man reduziert, um eine Verbindung der Formel ($I_{A_2}$) zu erhalten:

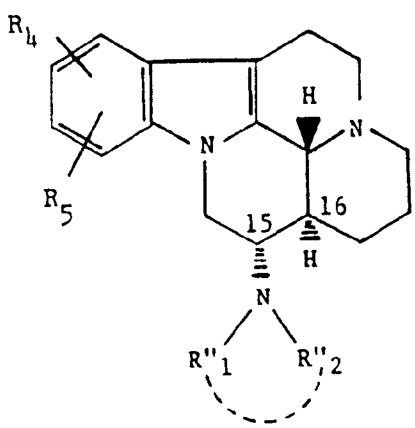

($I_{A_2}$)

worin $R''_1$, $R''_2$, $R_4$ und $R_5$ die vorstehend angegebenen Werte haben,
- oder der Einwirkung eines Reduktionsmittels unterwirft, um eine Verbindung der Formel (VI) zu erhalten,

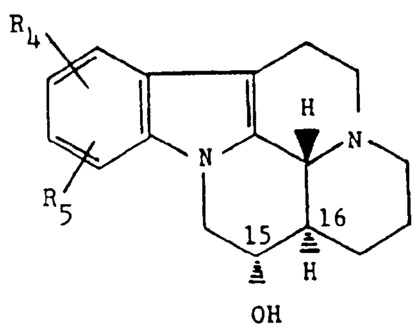

(VI)

von der man die Hydroxylfunktion aktiviert, und die man
- entweder der Einwirkung eines aliphatischen Amins ($III_A$)

$$H_2N\text{-}R'_1 \qquad (III_A)$$

unterwirft, wobei $R'_1$ die vorstehende Bedeutung hat, um eine Verbindung der Formel ($I_{B_1}$)

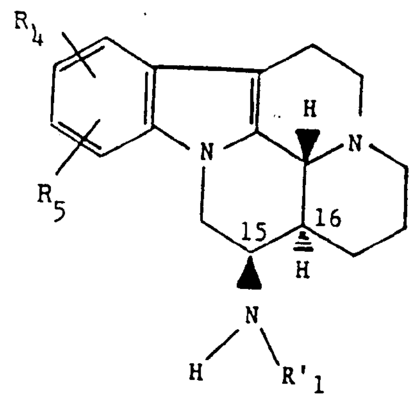

($I_{B_1}$)

zu erhalten, wobei $R'_1$, $R_4$ und $R_5$ die vorstehende Bedeutung haben,
- oder der Einwirkung eines heterocyclischen Amins der Formel ($III_B$)

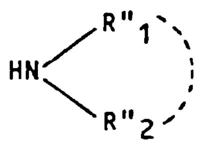

($III_B$)

unterwirft, wobei $R''_1$ und $R''_2$ die vorstehenden Bedeutungen haben, um eine Verbindung der Formel ($I_{B_2}$)

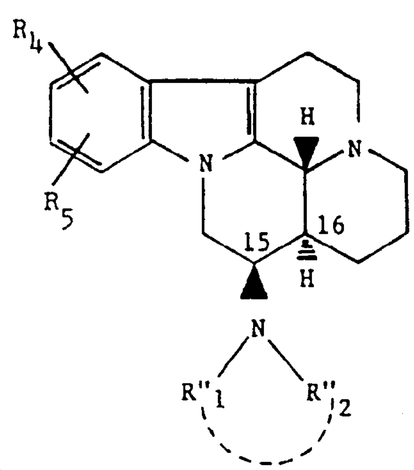

($I_{B_2}$)

zu erhalten, worin $R''_1$, $R''_2$, $R_4$ und $R_5$ die bereits angegebenen Bedeutungen haben, und daß man gewünschtenfalls die Verbindungen der Formeln ($I_{A_1}$) und ($I_{B_1}$) der Einwirkung eines Reagens unterwirft, das es ermöglicht, auf das Amin in 15-Stellung einen Rest $R'_2$ aufzupfropfen, der die gleichen Werte hat wie sie vorstehend für $R_2$ angegeben wurden, ausgenommen Wasserstoff, um die Verbindungen der Formeln ($I_{A_3}$) und ($I_{B_3}$)

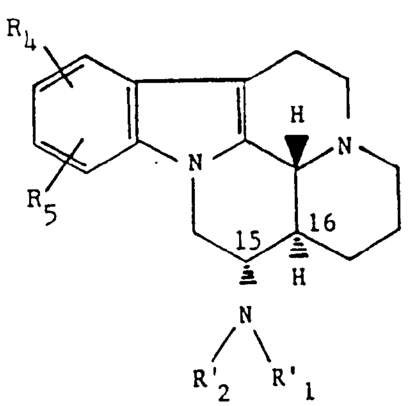

$(I_{A_3})$

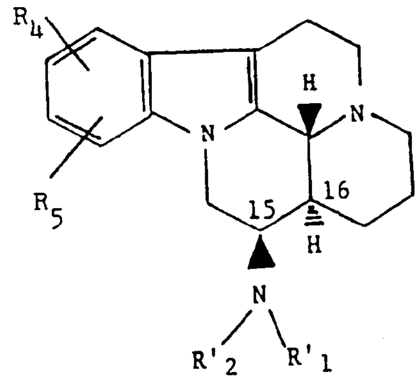

$(I_{B_3})$

zu erhalten, worin die Reste $R'_1$, $R'_2$, $R_4$ und $R_5$ die vorstehenden Bedeutungen haben, und daß man gewünschtenfalls für den Fall, daß der einzuführende Rest $R'_2$ ein Methylrest ist, die Verbindungen der Formel $(I_{A_1})$ und $(I_{B_1})$ der Einwirkung eines Formiats der Formel

$$HCO_2R$$

unterwirft, worin R einen Alkylrest mit 1 bis 4 Kohlenstoffatomen bedeutet, um eine Verbindung der Formel (VII)

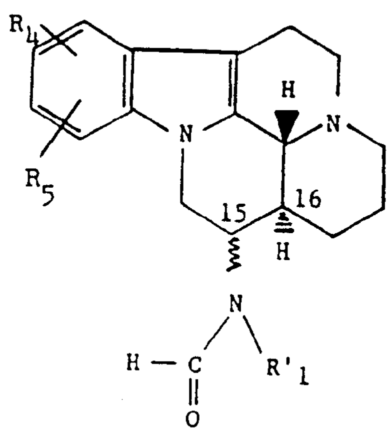

(VII)

zu erhalten, die man reduziert, um Verbindungen der Formeln $(I_{A_3})$ und $(I_{B_3})$ zu erhalten, worin der Rest $R'_2$ ein Methylrest ist, und daß man gewünschtenfalls die Verbindungen der Formeln $(I_{A_1})$ und $(I_{B_1})$, für die $R'_1$ ein gegebenenfalls substituierter Arylalkylrest ist, reduziert, um die Verbindungen der Formeln $(I_{A_4})$ und $(I_{B_4})$

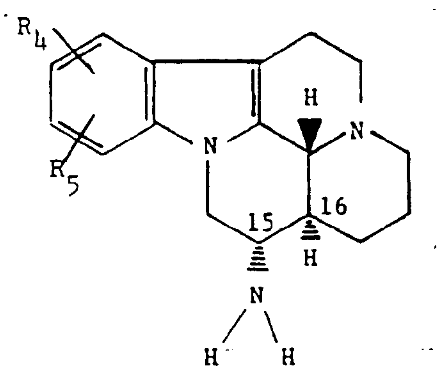

$(I_{A_4})$

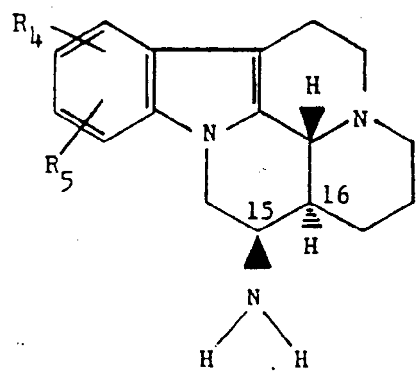

$(I_{B_4})$

zu erhalten, worin $R_4$ und $R_5$, die bereits angegebenen Bedeutungen haben, und daß man gewünschtenfalls die Verbindungen der Formel $(I_{A_4})$ und $(I_{B_4})$

- entweder der Einwirkung eines Reagens unterwirft, das es erlaubt, die Reste $R'_1$ und $R'_2$ aufzupfropfen, um die Verbindungen der Formeln $(I_{A_1})$ $(I_{B_1})$, $(I_{A_3})$ oder $(I_{B_3})$ zu erhalten,
- oder der Einwirkung eines Formiats $HCO_2R$ unterwirft, um eine Verbindung der Formel (VIII)

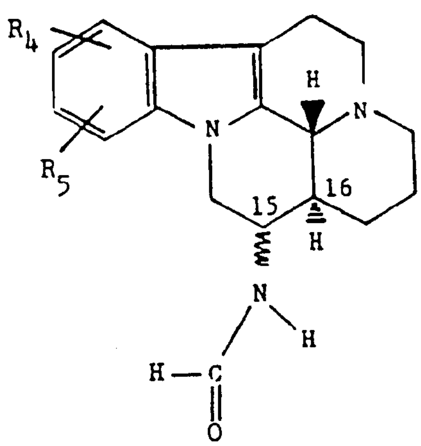

(VIII)

zu erhalten, die man reduziert, um eine Verbindung der Formeln $(I_{A_1})$ und $(I_{B_1})$ zu erhalten, worin der Rest $R'_1$ ein Methylrest ist, und
daß man gewünschtenfalls alle so erhaltenen Produkte der Formel (I) mit einer Mineral- oder organischen Säure behandelt, um daraus die Salze zu bilden.

2. Verfahren gemäß Anspruch 1, dadurch gekennzeichnet, daß das Ausgangsprodukt der Formel $(III_A)$ oder $(III_B)$ und die Reagentien, die man auf die Verbindungen der Formeln $(I_{A_1})$ und $(I_{B_1})$, $(I_{A_4})$, $(I_{B_4})$ zur Reaktion bringt, derart sind, daß man ein Produkt der Formel (I) erhält, worin $R_1$ und $R_2$, die identisch oder verschieden sind, einen Methyl- oder Ethylrest bedeuten, einen Rest

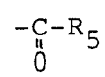

bedeuten, worin $R_5$ ein Methyl- oder Ethylrest, Phenyl, Benzyl, gegebenenfalls substituiert durch ein oder mehrere Chloratome, einen Rest $-CH_2-CH_2-OH$ oder
$R_1$ und $R_2$ bilden zusammen mit dem Stickstoffatom einen Pyrrolidinyl-, Morpholinyl- oder Piperazinylrest, dessen Stickstoffatom durch einen Methyl- oder phenylrest substituiert ist,
$R_4$ und $R_5$, die identisch oder verschieden sind, bedeuten ein Wasserstoffatom oder $R_4$ bedeutet einen

Nitrorest in 9- oder 11-Stellung und $R_5$ bedeutet ein Wasserstoffatom.

**3.** Verfahren gemäß Anspruch 1, dadurch gekennzeichnet, daß man herstellt:
[(±)(15alpha,16alpha]-14,15-Dihydro-N-methyl-20,21-dinoreburnamenin-15-amin,
[(±)(15alpha,16alpha)]-14,15-Dihydro -20,21-dinoreburnamenin-15-amin,
[(±)(15alpha,16alpha)]-2-[14,15-Dihydro-20-21-dinoreburnamenin-15-yl)-methylaminoethanol
und deren Additionssalze mit Säuren.

**Patentansprüche für folgenden Vertragsstaat : GR**

**1.** Verfahren zur Herstellung der Verbindungen der Formel (I)

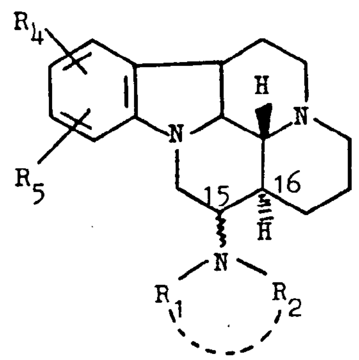

(I)

worin $R_1$ und $R_2$, die identisch oder verschieden sind, bedeuten ein Wasserstoffatom, einen Alkylrest mit 1 bis 5 Kohlenstoffatomen, einen Rest $-(CH_2)_n-OH$, wobei n zwischen 2 und 5 beträgt, einen Aryl- oder Arylalkylrest mit 7 bis 12 Kohlenstoffatomen, wobei diese beiden Reste gegebenenfalls durch 1, 2 oder 3 Reste substituiert sein können, ausgewählt aus der Gruppe gebildet durch die Halogenatome, die Methyl-, Ethyl-, Methoxy-, Ethoxy-, Trifluormethyl-, Amino- und Nitroreste, einen Rest

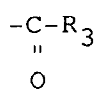

worin $R_3$ ein Alkylrest mit 1 bis 5 Kohlenstoffatomen, ein Aryl- oder Arylalkylrest mit 7 bis 12 Kohlenstoffatomen ist, wobei diese beiden Reste gegebenenfalls substituiert sind durch 1, 2 oder 3 Reste, ausgewählt aus der Gruppe gebildet von den Halogenatomen, Methyl-, Ethyl-, Methoxy-, Ethoxy-, Trifluormethyl-, Amino- und Nitroresten, wobei $R_1$ und $R_2$ nicht beide einen Arylrest bedeuten können, oder
$R_1$ und $R_2$ bilden mit dem Stickstoffatom, an das sie gebunden sind, einen gesättigten oder ungesättigten heterocyclischen Rest, der ein zweites Heteroatom enthalten kann, ausgewählt aus den Sauerstoff-, Schwefel- und Stickstoffatomen, wobei letzterer gegebenenfalls substituiert sein kann durch einen Alkylrest mit 1 bis 5 Kohlenstoffatomen, einen Arylrest, einen Arylalkylrest mit 7 bis 12 Kohlenstoffatomen, und diese Reste gegebenenfalls substituiert sind durch 1, 2 oder 3 Reste, ausgewählt aus der Gruppe gebildet von den Halogenatomen, den Methyl-, Ethyl-, Methoxy-, Ethoxy-, Trifluormethyl-, Amino- und Nitroresten,
$R_4$ und $R_5$, die identisch oder verschieden sind, bedeuten ein Wasserstoffatom, ein Halogenatom, einen Alkyl- oder Alkoxyrest mit 1 bis 5 Kohlenstoffatomen, einen Hydroxy-, Trifluormethyl- oder Nitrorest,
wobei diese Produkte der Formel (I) unter allen möglichen enantiomeren und diastereoisomeren Formen und in Form der Additionssalze mit Säuren vorliegen, dadurch gekennzeichnet, daß man eine Verbindung der Formel (II)

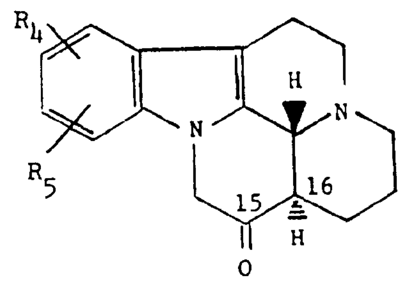

(II)

worin $R_4$ und $R_5$ die vorstehend angegebene Bedeutung haben,

- entweder der Einwirkung eines aliphatischen Amins der Formel ($III_A$):

$$H_2NR'_1 \qquad (III_A)$$

unterwirft, worin $R'_1$ die vorstehende Bedeutung von $R_1$ hat, mit Ausnahme vor Wasserstoff, um eine Verbindung der Formel (IV)

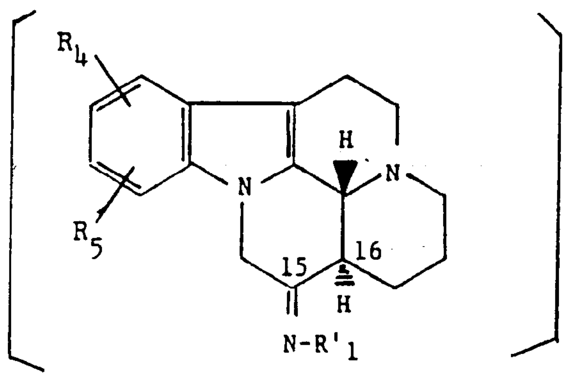

(IV)

zu erhalten, die man reduziert, um eine Verbindung der Formel ($I_{A_1}$)

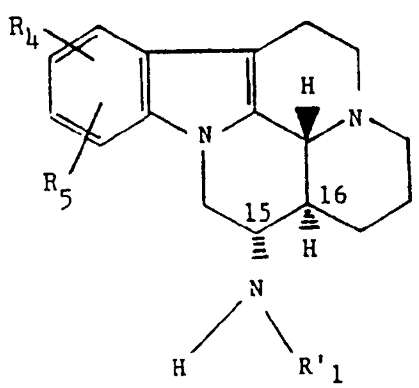

($I_{A_1}$)

zu erhalten, worin $R'_1$, $R_4$ und $R_5$ die angegebene Bedeutung haben,

- oder der Einwirkung eines heterocyclischen Amins der Formel ($III_B$):

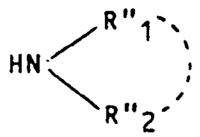

($III_B$)

unterwirft, worin $R''_1$ und $R''_2$ mit dem Stickstoffatom, an das sie gebunden sind, die gleichen heterocyclischen Amine bilden wie diejenigen wie vorstehend für $R_1$ und $R_2$ genannt sind, um eine Verbindung der Formel (V)

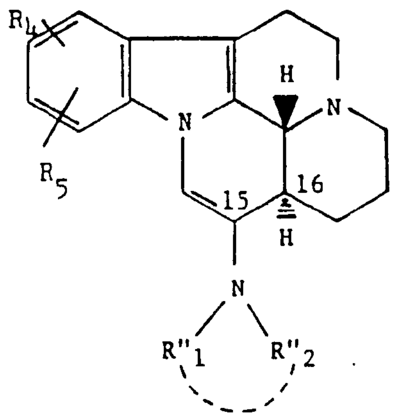

(V)

zu erhalten, die man reduziert, um eine Verbindung der Formel ($I_{A_2}$) zu erhalten:

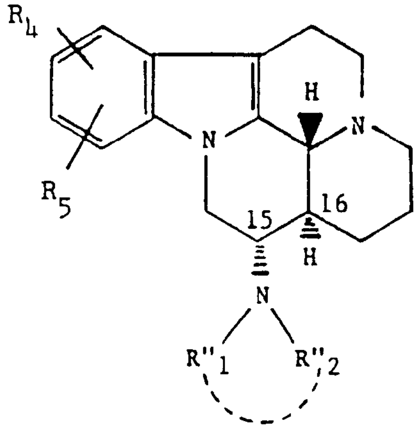

($I_{A_2}$)

worin $R''_1$, $R''_2$, $R_4$ und $R_5$ die vorstehend angegebenen Werte haben,
- oder der Einwirkung eines Reduktionsmittels unterwirft, um eine Verbindung der Formel (VI) zu erhalten,

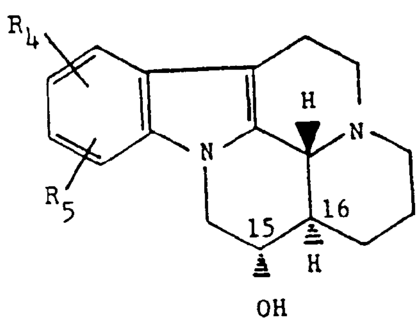

(VI)

von der man die Hydroxylfunktion aktiviert, und die man
- entweder der Einwirkung eines aliphatischen Amins ($III_A$)

$$H_2N\text{-}R'_1 \qquad (III_A)$$

unterwirft, wobei $R'_1$ die vorstehende Bedeutung hat, um eine Verbindung der Formel ($I_{B_1}$)

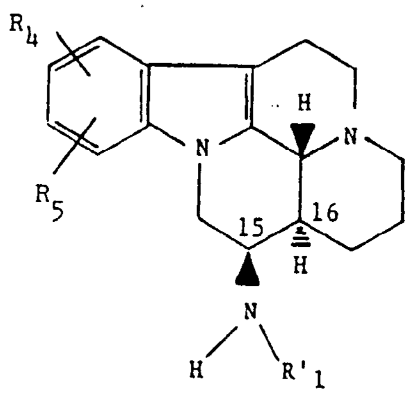

$(I_{B_1})$

zu erhalten, wobei $R'_1$, $R_4$ und $R_5$ die vorstehende Bedeutung haben,
- oder der Einwirkung eines heterocyclischen Amins der Formel $(III_B)$

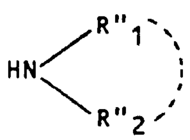

$(III_B)$

unterwirft, wobei $R''_1$ und $R''_2$ die vorstehenden Bedeutungen haben, um eine Verbindung der Formel $(I_{B_2})$

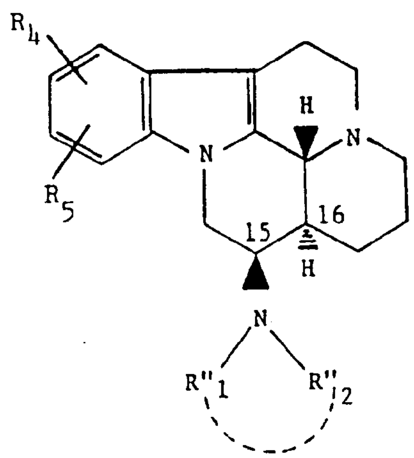

$(I_{B_2})$

zu erhalten, worin $R''_1$, $R''_2$, $R_4$ und $R_5$ die bereits angegebenen Bedeutungen haben, und daß man gewünschtenfalls die Verbindungen der Formeln $(I_{A_1})$ und $(I_{B_1})$ der Einwirkung eines Reagens unterwirft, das es ermöglicht, auf das Amin in 15-Stellung einen Rest $R'_2$ aufzupfropfen, der die gleichen Werte hat wie sie vorstehend für $R_2$ angegeben wurden, ausgenommen Wasserstoff, um die Verbindungen der Formeln $(I_{A_3})$ und $(I_{B_3})$

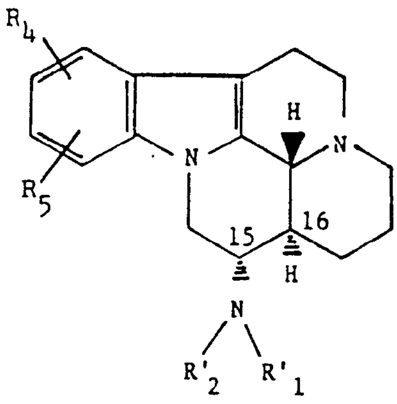

$(I_{A_3})$

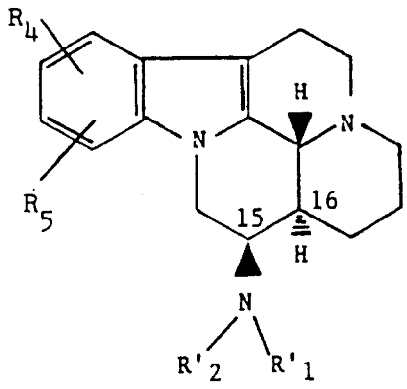

$(I_{B_3})$

zu erhalten, worin die Reste $R'_1$, $R'_2$, $R_4$ und $R_5$ die vorstehenden Bedeutungen haben, und daß man gewünschtenfalls für den Fall, daß der einzuführende Rest $R'_2$ ein Methylrest ist, die Verbindungen der Formel $(I_{A_1})$ und $(I_{B_1})$ der Einwirkung eines Formiats der Formel

$$HCO_2R$$

unterwirft, worin R einen Alkylrest mit 1 bis 4 Kohlenstoffatomen bedeutet, um eine Verbindung der Formel (VII)

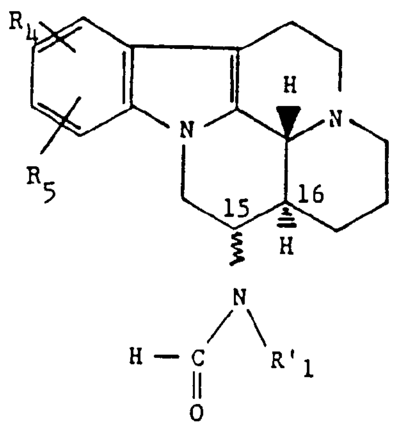

(VII)

zu erhalten, die man reduziert, um Verbindungen der Formeln $(I_{A_3})$ und $(I_{B_3})$ zu erhalten, worin der Rest $R'_2$ ein Methylrest ist, und daß man gewünschtenfalls die Verbindungen der Formeln $(I_{A_1})$ und $(I_{B_1})$, für die $R'_1$ ein gegebenenfalls substituierter Arylalkylrest ist, reduziert, um die Verbindungen der Formeln $(I_{A_4})$ und $(I_{B_4})$

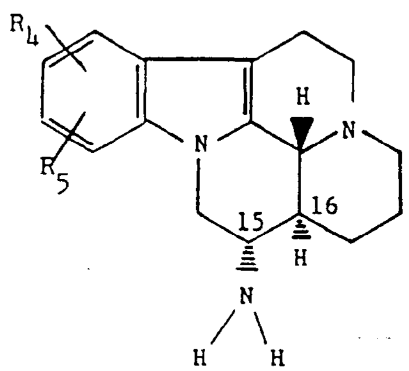

$(I_{A_4})$

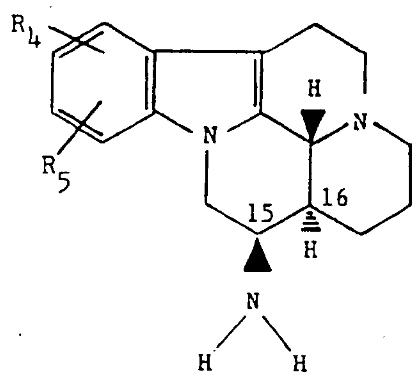

$(I_{B_4})$

zu erhalten, worin $R_4$ und $R_5$, die bereits angegebenen Bedeutungen haben, und daß man gewünschtenfalls die Verbindungen der Formel $(I_{A_4})$ und $(I_{B_4})$

- entweder der Einwirkung eines Reagens unterwirft, das es erlaubt, die Reste $R'_1$ und $R'_2$ aufzupfropfen, um die Verbindungen der Formeln $(I_{A_1})$ $(I_{B_1})$, $(I_{A_3})$ oder $(I_{B_3})$ zu erhalten,
- oder der Einwirkung eines Formiats $HCO_2R$ unterwirft, um eine Verbindung der Formel (VIII)

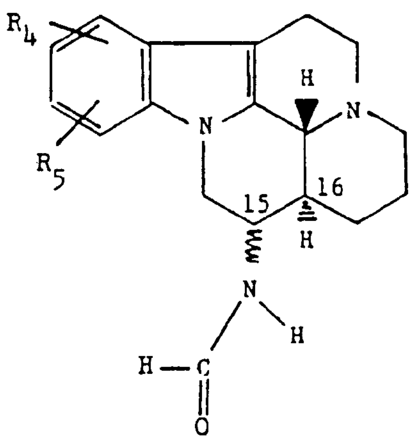

(VIII)

zu erhalten, die man reduziert, um eine Verbindung der Formeln $(I_{A_1})$ und $(I_{B_1})$ zu erhalten, worin der Rest $R'_1$ ein Methylrest ist, und
daß man gewünschtenfalls alle so erhaltenen Produkte der Formel (I) mit einer Mineral- oder organischen Säure behandelt, um daraus die Salze zu bilden.

2. Verfahren gemäß Anspruch 1, dadurch gekennzeichnet, daß das Ausgangsprodukt der Formel $(III_A)$ oder $(III_B)$ und die Reagentien, die man auf die Verbindungen der Formeln $(I_{A_1})$ und $(I_{B_1})$, $(I_{A_4})$, $(I_{B_4})$ zur Reaktion bringt, derart sind, daß man ein Produkt der Formel (I) erhält, worin $R_1$ und $R_2$, die identisch oder verschieden sind, einen Methyl- oder Ethylrest bedeuten, einen Rest

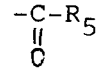

bedeuten, worin $R_5$ ein Methyl- oder Ethylrest, Phenyl, Benzyl, gegebenenfalls substituiert durch ein oder mehrere Chloratome, einen Rest $-CH_2-CH_2-OH$ oder
$R_1$ und $R_2$ bilden zusammen mit dem Stickstoffatom einen Pyrrolidinyl-, morpholinyl- oder Piperazinylrest, dessen Stickstoffatom durch einen Methyl- oder Phenylrest substituiert ist,
$R_4$ und $R_5$, die identisch oder verschieden sind, bedeuten ein Wasserstoffatom oder $R_4$ bedeutet einen

Nitrorest in 9- oder 11-Stellung und $R_5$ bedeutet ein Wasserstoffatom.

**3.** Verfahren gemäß Anspruch 1, dadurch gekennzeichnet, daß man herstellt:
[(±)(15alpha,16alpha]-14,15-Dihydro-N-methyl-20,21-dinoreburnamenin-15-amin,
[(±)(15alpha,16alpha)]-14,15-Dihydro-20,21-dinoreburnamenin-15-amin,
[(±)(15alpha,16alpha)]-2-[14,15-Dihydro-20,21-dinoreburnamenin-15-yl)-methylaminoethanol
und deren Additionssalze mit Säuren.

**4.** Verfahren zur Herstellung von pharmazeutischen Zusammensetzungen, dadurch gekennzeichnet, daß man als Wirkstoff wenigstens eine der Verbindungen der Formel (I), wie sie in Anspruch 1 definiert sind, oder wenigstens eines ihrer Additionssalze mit pharmazeutisch annehmbaren Mineral- oder organischen Säuren, in einer für diesen Verwendungszweck bestimmten Form einsetzt.

**5.** Verfahren gemäß Anspruch 4, dadurch gekennzeichnet, daß die Verbindung der Formel (I) ausgewählt ist unter:
[(±)(15alpha,16alpha]-14,15-Dihydro-N-methyl-20,21-dinoreburnamenin-15-amin,
[(±)(15alpha,16alpha)]-14,15-Dihydro-20,21-dinoreburnamenin-15-amin,
[(±)(15alpha,16alpha)]-2-[14,15-Dihydro-20,21-dinoreburnamenin-15-yl)-methylaminoethanol
und deren Addtionssalze mit Säuren.

**6.** Als neue industrielle Produkte die Verbindung der Formel (IV), (V), (VII), (VIII) und diejenigen der Formel (II) und (VI), worin $R_4$ und $R_5$ jeweils nicht ein Wasserstoffatom bedeuten.

## Claims

### Claims for the following Contracting States : AT, BE, CH, DE, FR, GB, IT, LI, LU, NL, SE

**1.** Compounds of formula (I):

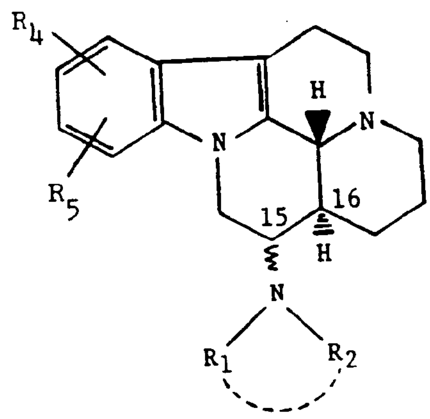

(I)

in which $R_1$ and $R_2$, identical or different, represent a hydrogen atom, an alkyl radical containing 1 to 5 carbon atoms, a $-(CH_2)_n-OH$ radical in which n is comprised between 2 and 5, an aryl or arylalkyl radical containing 7 to 12 carbon atoms, these two radicals being optionally substituted by 1, 2 or 3 radicals chosen from the group formed by halogen atoms, methyl, ethyl, methoxy, ethoxy, trifluoromethyl, amino and nitro radicals, a

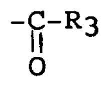

radical in which $R_3$ is an alkyl radical containing 1 to 5 carbon atoms, an aryl or arylalkyl radical containing 7 to 12 carbon atoms, these two radicals being optionally substituted by 1, 2 or 3 radicals chosen from the group formed by halogen atoms, methyl, ethyl, methoxy, ethoxy, trifluoromethyl, amino and nitro radicals, however, $R_1$ and $R_2$ cannot both represent an aryl radical, or $R_1$ and $R_2$ form together with the nitrogen atom to which they are linked a saturated or unsaturated heterocyclic radical which is able to contain a second heteroatom chosen from oxygen, sulphur and nitrogen atoms, the latter being optionally substituted by an alkyl radical containing 1 to 5 carbon atoms, an aryl radical, an arylalkyl radical containing 7 to 12 carbon atoms, these radicals being optionally substituted by 1, 2 or 3 radicals chosen from the group formed by halogen atoms, methyl, ethyl, methoxy, ethoxy, trifluoromethyl, amino and nitro radicals, $R_4$ and $R_5$, identical or different, represent a hydrogen atom, a halogen atom, an alkyl or alkoxy radical containing 1 to 5 carbon atoms, a hydroxy, trifluoromethyl or nitro radical, the said products of formula (I) being in all possible enantiomeric and diastereoisomeric forms and in the form of addition salts with acids.

**2.** Compounds of formula (I), as defined in claim 1, characterized in that $R_1$ and $R_2$, identical or different, represent a hydrogen atom, a methyl or ethyl radical, a

$-C-R_3$
O

radical in which $R_3$ is a methyl or ethyl, phenyl, benzyl radical optionally substituted by one or more chlorine atoms, a $CH_2$-$CH_2$-OH radical or $R_1$ and $R_2$ form together with the nitrogen atom a pyrrolidinyl, morpholinyl or piperazinyl radical the nitrogen atom of which is substituted by a methyl or phenyl radical, $R_4$ and $R_5$ represent a hydrogen atom or $R_4$ represents a nitro radical in position 9 or 11 and $R_5$ represents a hydrogen atom and their addition salts with acids.

**3.** [(±) (15alpha, 16alpha)]-14,15-dihydro-N-methyl-20,21-dinoreburnamenin-15-amine and its addition salts with acids.

**4.** [(±) (15alpha, 16alpha)]-14,15-dihydro-20,21-dinoreburnamenin-15-amine and its addition salts with acids.

**5.** [(±) (15alpha, 16alpha)]-2-[14,15-dihydro-20,21-dinoreburnamenin-15-yl)-methylamino]-ethanol and its addition salts with acids.

**6.** Preparation process for the compounds of formula (I) as defined in claim 1, characterized in that a compound of formula (II):

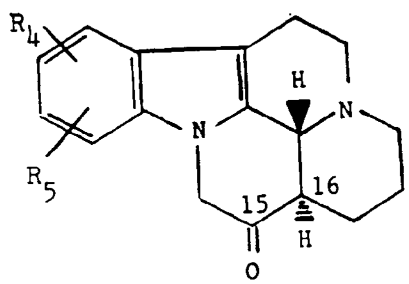

(II)

in which $R_4$ and $R_5$ have the meaning indicated previously, is subjected

- either to the action of an aliphatic amine of formula ($III_A$):

$H_2NR'_1$ ($III_A$)

in which $R'_1$ has the previous meaning of $R_1$, except for hydrogen, in order to obtain a compound of formula (IV):

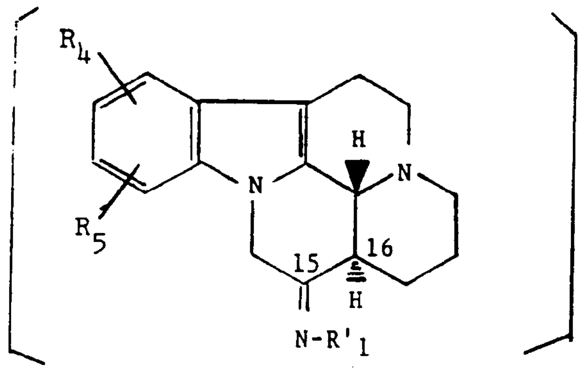

(IV)

which is reduced in order to obtain a compound of formula ($I_{A1}$):

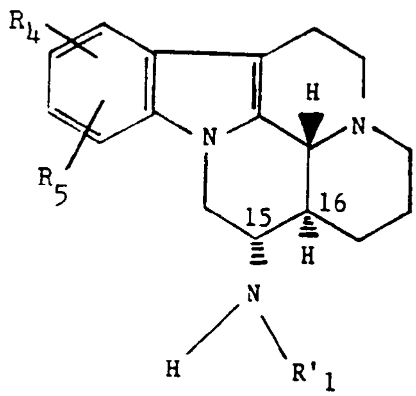

($I_{A1}$)

in which $R'_1$, $R_4$ and $R_5$ have the meaning already indicated,
- or to the action of a heterocyclic amine of formula ($III_B$):

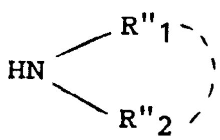

($III_B$)

in which $R''_1$ and $R''_2$ form together with the nitrogen atom to which they are linked the same heterocyclic amines as those given previously for $R_1$ and $R_2$, in order to obtain a compound of formula (V):

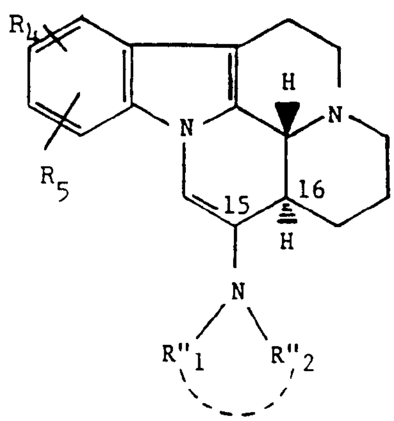

(V)

which is reduced in order to obtain a compound of formula ($I_{A2}$):

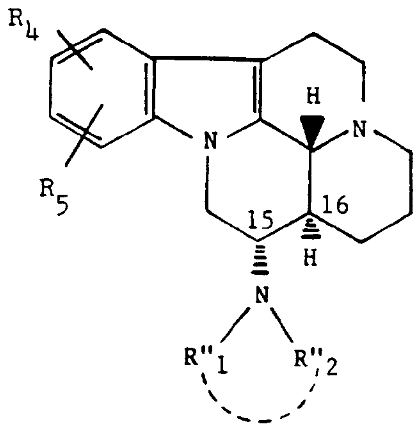

(IA2)

in which $R''_1$, $R''_2$, $R_4$ and $R_5$ have the values given previously,
- or to the action of a reducing agent in order to obtain a compound of formula (VI):

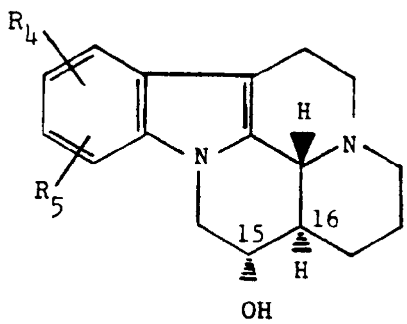

(VI)

the hydroxyl function of which is activated and which is subjected
- either to the action of an aliphatic amine ($III_A$):

$$H_2N\text{-}R'_1 \qquad (III_A)$$

$R'_1$ having the previous meaning, in order to obtain a compound of formula ($I_{B1}$):

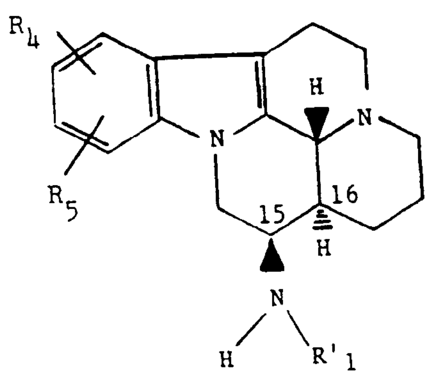

($I_{B1}$)

$R'_1$, $R_4$ and $R_5$ having the previous meaning,
- or to the action of a heterocyclic amine of formula ($III_B$):

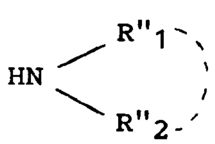

($III_B$)

$R''_1$ and $R''_2$ having the previous meanings, in order to obtain a compound of formula ($I_{B2}$):

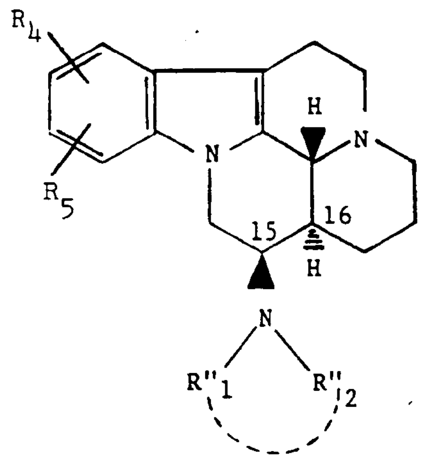

($I_{B2}$)

in which $R''_1$, $R''_2$, $R_4$ and $R_5$ have the meanings already indicated and that, if desired, the compounds of formulae ($I_{A1}$) and ($I_{B1}$) are subjected to the action of a reagent which allows the grafting of an $R'_2$ radical having the same values as given previously for $R_2B$, except for hydrogen, onto the amine in position 15, in order to obtain the compounds of formulae ($I_{A3}$) and ($I_{B3}$):

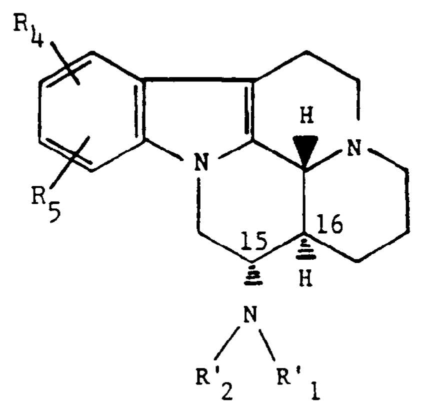

($I_{A3}$)

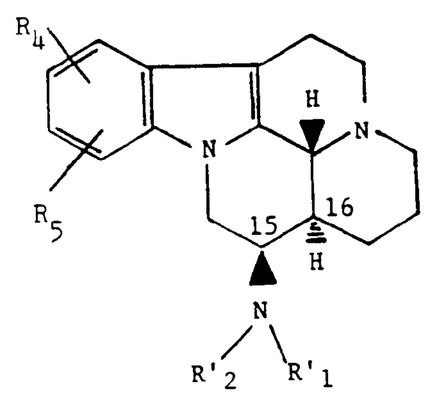

($I_{B3}$)

in which $R'_1$, $R'_2$, $R_4$ and $R_5$ have the previous meanings and that, if desired, in the case where the $R'_2$ radical to be introduced is a methyl radical, the compounds of formulae ($I_{A1}$) and ($I_{B1}$) are subjected to the action of a formate of formula:

$$HCO_2R$$

in which R is an alkyl radical containing 1 to 4 carbon atoms in order to obtain a compound of formula (VII):

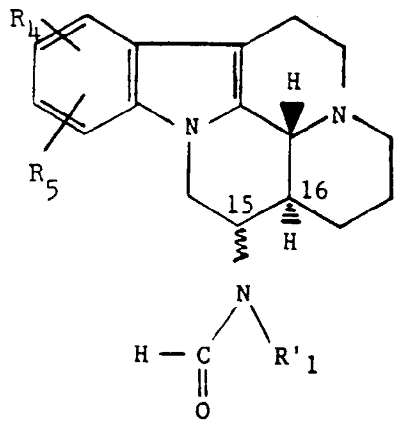

(VII)

which is reduced in order to obtain the compounds of formulae ($I_{A3}$) and ($I_{B3}$) in which the $R'_2$ radical is a methyl radical and that, if desired, the compounds of formulae ($I_{A1}$) and ($I_{B1}$) for which $R'_1$ is an optionally substituted arylalkyl radical are reduced in order to obtain the compounds of formulae ($I_{A4}$) and ($I_{B4}$):

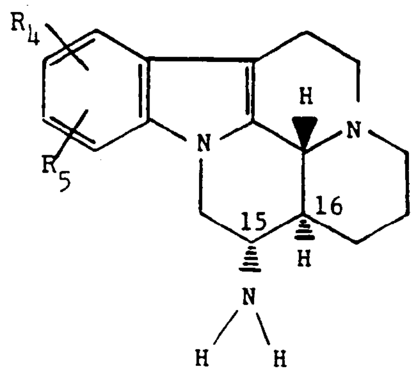

($I_{A4}$)

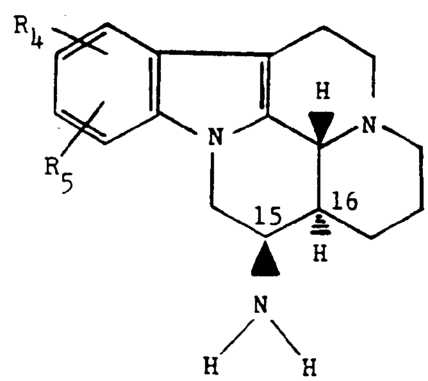

($I_{B4}$)

in which $R_4$ and $R_5$ have the meanings already indicated, and that, if desired, the compounds of formulae ($I_{A4}$) and ($I_{B4}$) are subjected,

- either to the action of a reagent which allows the $R'_1$ and $R'_2$ radicals to be grafted in order to obtain the compounds of formulae ($I_{A1}$), ($I_{B1}$), ($I_{A3}$) or ($I_{B3}$),
- or to the action of an $HCO_2R$ formate in order to obtain a compound of formula (VIII):

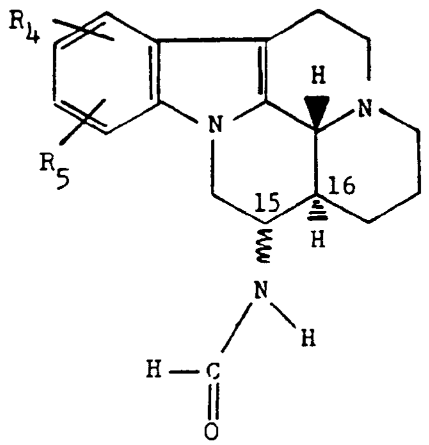

(VIII)

which is reduced in order to obtain a compound of formulae ($I_{A1}$) and ($I_{B1}$) in which the $R'_1$ radical is a methyl radical and, if desired, all the products of formula (I) thus obtained are treated with a mineral or organic acid, in order to form the salts.

7. As medicaments, the compounds as defined by formula (I) of claim 1, the said products of formula (I) being in all possible racemic or optically active isomer forms as well as the addition salts with pharmaceutically acceptable mineral or organic acids.

8. As medicaments, the compounds as defined in claim 2.

9. As medicaments, any one of the compounds of claims 3, 4 and 5.

10. The pharmaceutical compositions containing as active ingredient at least one of the medicaments as defined in any one of claims 7 to 9.

11. As new industrial products, the compounds of formulae (IV), (V), (VII), (VIII) and those of formulae (II) and (VI) in which $R_4$ and $R_5$ do not each represent a hydrogen atom.

**Claims for the following Contracting State : ES**

1. Process for the preparation of the compounds of formula (I):

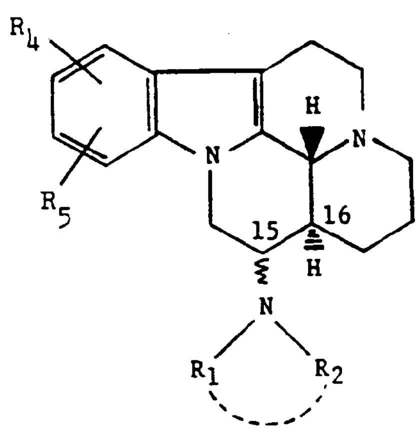

(I)

in which $R_1$ and $R_2$, identical or different, represent a hydrogen atom, an alkyl radical containing 1 to 5 carbon atoms, a $-(CH_2)_n-OH$ radical in which n is comprised between 2 and 5, an aryl or arylalkyl radical containing 7 to 12 carbon atoms, these two radicals being optionally substituted by 1, 2 or 3 radicals chosen from the group formed by halogen atoms, methyl, ethyl, methoxy, ethoxy, trifluoromethyl, amino and nitro radicals, a

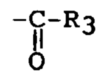

radical in which $R_3$ is an alkyl radical containing 1 to 5 carbon atoms, an aryl or arylalkyl radical containing 7 to 12 carbon atoms, these two radicals being optionally substituted by 1, 2 or 3 radicals chosen from the group formed by halogen atoms, methyl, ethyl, methoxy, ethoxy, trifluoromethyl, amino and nitro radicals, however, $R_1$ and $R_2$ cannot both represent an aryl radical, or $R_1$ and $R_2$ form together with the nitrogen atom to which they are linked a saturated or unsaturated heterocyclic radical which is able to contain a second heteroatom chosen from oxygen, sulphur and nitrogen atoms, the latter being optionally substituted by an alkyl radical containing 1 to 5 carbon atoms, an aryl radical, an arylalkyl radical containing 7 to 12 carbon atoms, these radicals being optionally substituted by 1, 2 or 3 radicals chosen from the group formed by halogen atoms, methyl, ethyl, methoxy, ethoxy, trifluoromethyl, amino and nitro radicals, $R_4$ and $R_5$, identical or different, represent a hydrogen atom, a halogen atom, an alkyl or alkoxy radical containing 1 to 5 carbon atoms, a hydroxy, trifluoromethyl or nitro radical, the said products of formula (I) being in all possible enantiomeric and diastereoisomeric forms and in the form of addition salts with acids, characterized in that a compound of formula (II):

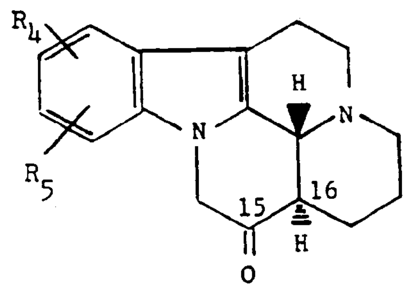

(II)

in which $R_4$ and $R_5$ have the meaning indicated previously, is subjected

- either to the action of an aliphatic amine of formula ($III_A$):

$$H_2NR'_1 \qquad (III_A)$$

in which $R'_1$ has the previous meaning of $R_1$, except for hydrogen, in order to obtain a compound of formula (IV):

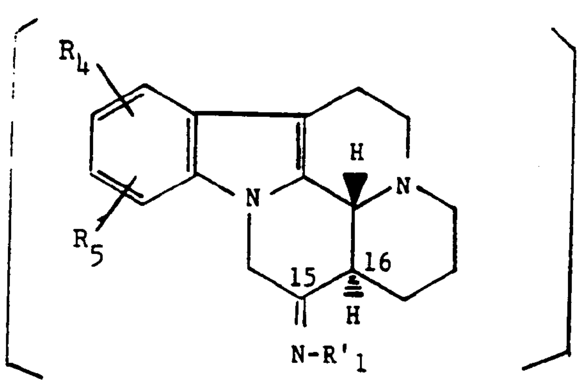

(IV)

which is reduced in order to obtain a compound of formula ($I_{A1}$):

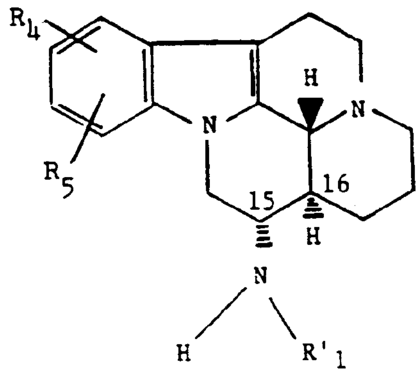

$(I_{A1})$

in which $R'_1$, $R_4$ and $R_5$ have the meaning already indicated,
- or to the action of a heterocyclic amine of formula $(III_B)$:

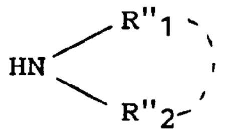

$(III_B)$

in which $R''_1$ and $R''_2$ form together with the nitrogen atom to which they are linked the same heterocyclic amines as those given previously for $R_1$ and $R_2$, in order to obtain a compound of formula (V):

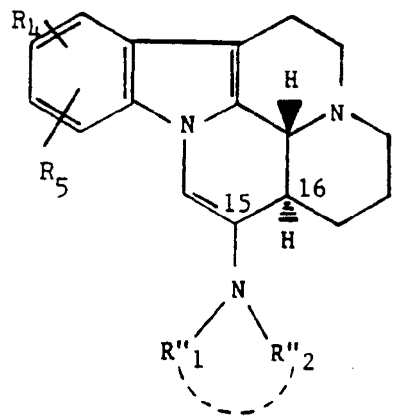

(V)

which is reduced in order to obtain a compound of formula $(I_{A2})$:

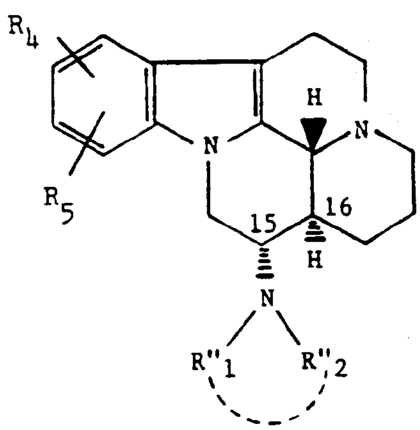

$(I_{A2})$

in which $R''_1$, $R''_2$, $R_4$ and $R_5$ have the values given previously,

- or to the action of a reducing agent in order to obtain a compound of formula (VI):

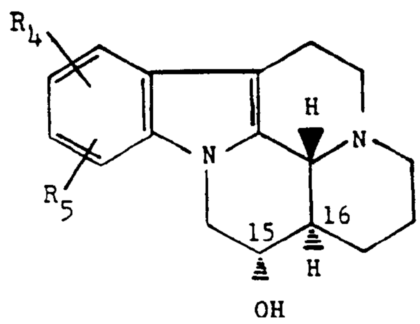

(VI)

the hydroxyl function of which is activated and which is subjected
- either to the action of an aliphatic amine ($III_A$):

$$H_2N\text{-}R'_1 \qquad (III_A)$$

$R'_1$ having the previous meaning, in order to obtain a compound of formula ($I_{B1}$):

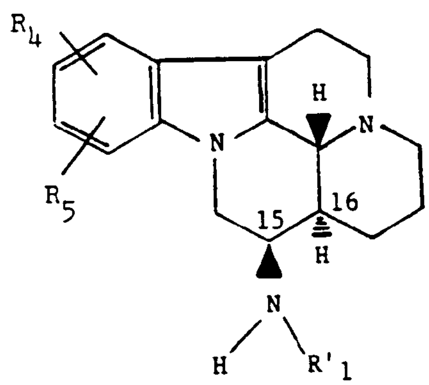

($I_{B1}$)

$R'_1$, $R_4$ and $R_5$ having the previous meaning,
- or to the action of a heterocyclic amine of formula ($III_B$):

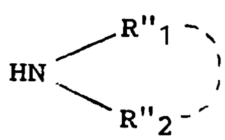

($III_B$)

$R''_1$ and $R''_2$ having the previous meanings, in order to obtain a compound of formula ($I_{B2}$):

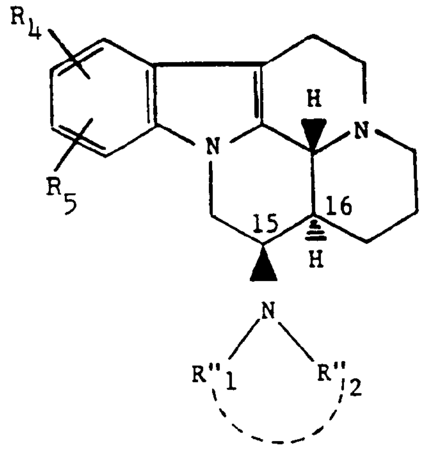

$(I_{B2})$

in which $R''_1$, $R''_2$, $R_4$ and $R_5$ have the meanings already indicated and that, if desired, the compounds of formulae $(I_{A1})$ and $(I_{B1})$ are subjected to the action of a reagent which allows the grafting of an $R'_2$ radical having the same values as given previously for $R_2$, except for hydrogen, onto the amine in position 15, in order to obtain the compounds of formulae $(I_{A3})$ and $(I_{B3})$:

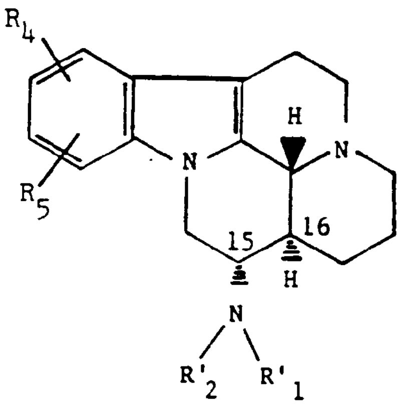

$(I_{A3})$

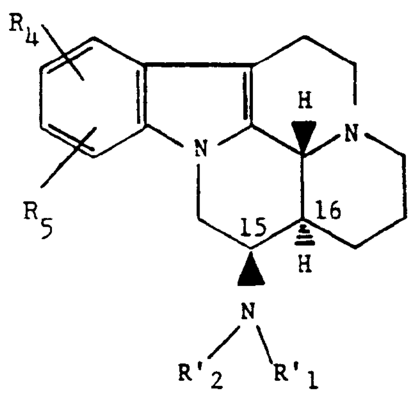

$(I_{B3})$

in which $R'_1$, $R'_2$, $R_4$ and $R_5$ have the previous meanings and that, if desired, in the case where the $R'_2$ radical to be introduced is a methyl radical, the compounds of formulae $(I_{A1})$ and $(I_{B1})$ are subjected to the action of a formate of formula:

$$HCO_2R$$

in which R is an alkyl radical containing 1 to 4 carbon atoms in order to obtain a compound of formula (VII):

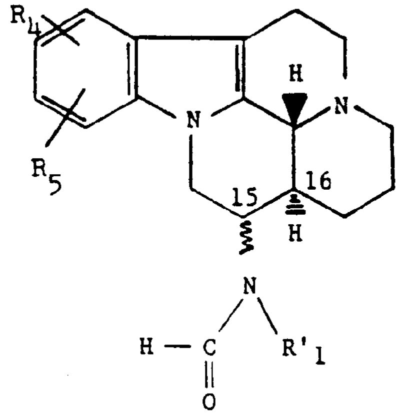

(VII)

which is reduced in order to obtain the compounds of formulae ($I_{A3}$) and ($I_{B3}$) in which the $R'_2$ radical is a methyl radical and that, if desired, the compounds of formulae ($I_{A1}$) and ($I_{B1}$) for which $R'_1$ is an optionally substituted arylalkyl radical are reduced in order to obtain the compounds of formulae ($I_{A4}$) and ($I_{B4}$):

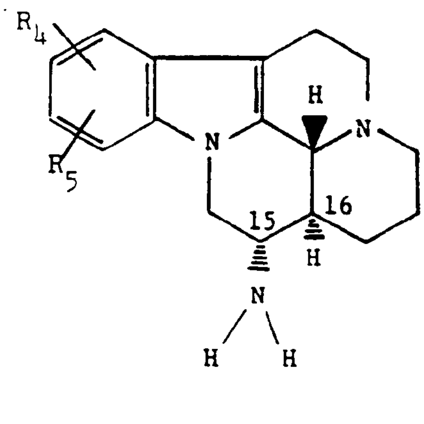

($I_{A4}$)

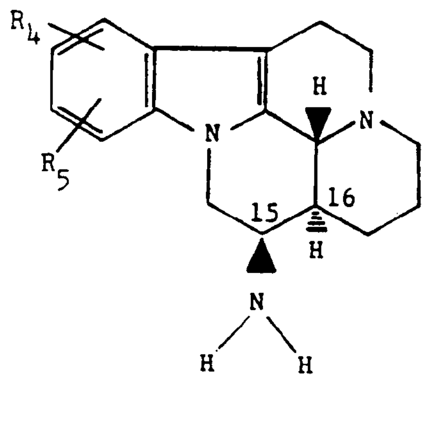

($I_{B4}$)

in which $R_4$ and $R_5$ have the meanings already indicated, and that, if desired, the compounds of formulae ($I_{A4}$) and ($I_{B4}$) are subjected,
- either to the action of a reagent which allows the $R'_1$ and $R'_2$ radicals to be grafted in order to obtain the compounds of formulae ($I_{A1}$), ($I_{B1}$), ($I_{A3}$) or ($I_{B3}$),
- or to the action of an $HCO_2R$ formate in order to obtain a compound of formula (VIII):

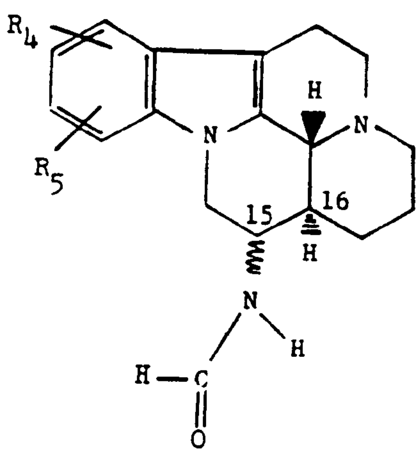

(VIII)

which is reduced in order to obtain a compound of formulae ($I_{A1}$) and ($I_{B1}$) in which the $R'_1$ radical is a methyl radical and, if desired, all the products of formula (I) thus obtained are treated with a mineral or organic acid, in order to form the salts.

2. Process according to claim 1, characterized in that the starting product of formula ($III_A$) or ($III_B$) and the reagents which are reacted on the compounds of formulae ($I_{A1}$), ($I_{B1}$), ($I_{A4}$), ($I_{B4}$) are such that a product of formula (I) is obtained in which $R_1$ and $R_2$, identical or different, represent a methyl or ethyl radical, a

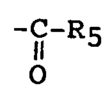

radical in which $R_5$ is a methyl or ethyl, phenyl, benzyl radical optionally substituted by one or more chlorine atoms, a $-CH_2-CH_2-OH$ radical or $R_1$ and $R_2$ form with the nitrogen atom a pyrrolidinyl, morpholinyl or piperazinyl radical the nitrogen atom of which is substituted by a methyl or phenyl radical, $R_4$ and $R_5$, identical or different, represent a hydrogen atom or $R_4$ represents a nitro radical in position 9 or 11 and $R_5$ represents

a hydrogen atom.

3. Process according to claim 1, characterized in that the following are prepared:
[(±) (15alpha, 16alpha)]-14,15-dihydro-N-methyl-20,21-dinoreburnamenin-15-amine,
[(±) (15alpha, 16alpha)]-14,15-dihydro-20,21-dinoreburnamenin-15-amine
[(±) (15alpha, 16alpha)]-2-[14,15-dihydro-20,21-dinoreburnamenin-15-yl)-methylamino]-ethanol
and their addition salts with acids.

**Claims for the following Contracting State : GR**

1. Process for the preparation of the compounds of formula (I):

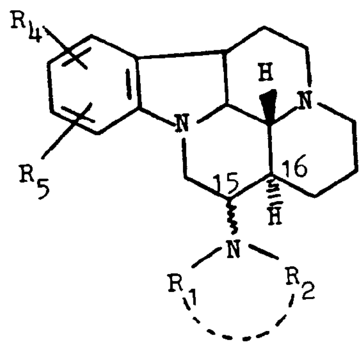

(I)

in which $R_1$ and $R_2$, identical or different, represent a hydrogen atom, an alkyl radical containing 1 to 5 carbon atoms, a $-(CH_2)_n-OH$ radical in which n is comprised between 2 and 5, an aryl or arylalkyl radical containing 7 to 12 carbon atoms, these two radicals being optionally substituted by 1, 2 or 3 radicals chosen from the group formed by halogen atoms, methyl, ethyl, methoxy, ethoxy, trifluoromethyl, amino and nitro radicals, a

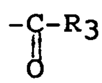

radical in which $R_3$ is an alkyl radical containing 1 to 5 carbon atoms, an aryl or arylalkyl radical containing 7 to 12 carbon atoms, these two radicals being optionally substituted by 1, 2 or 3 radicals chosen from the group formed by halogen atoms, methyl, ethyl, methoxy, ethoxy, trifluoromethyl, amino and nitro radicals, however, $R_1$ and $R_2$ cannot both represent an aryl radical, or $R_1$ and $R_2$ form together with the nitrogen atom to which they are linked a saturated or unsaturated heterocyclic radical which is able to contain a second heteroatom chosen from oxygen, sulphur and nitrogen atoms, the latter being optionally substituted by an alkyl radical containing 1 to 5 carbon atoms, an aryl radical, an arylalkyl radical containing 7 to 12 carbon atoms, these radicals being optionally substituted by 1, 2 or 3 radicals chosen from the group formed by halogen atoms, methyl, ethyl, methoxy, ethoxy, trifluoromethyl, amino and nitro radicals, $R_4$ and $R_5$, identical or different, represent a hydrogen atom, a halogen atom, an alkyl or alkoxy radical containing 1 to 5 carbon atoms, a hydroxy, trifluoromethyl or nitro radical, the said products of formula (I) being in all possible enantiomeric and diastereoisomeric forms and in the form of addition salts with acids, characterized in that a compound of formula (II):

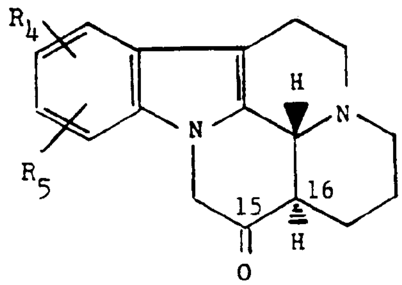

(II)

in which $R_4$ and $R_5$ have the meaning indicated previously, is subjected

- either to the action of an aliphatic amine of formula ($III_A$):

$$H_2NR'_1 \qquad (III_A)$$

in which $R'_1$ has the previous meaning of $R_1B$, except for hydrogen, in order to obtain a compound of formula (IV):

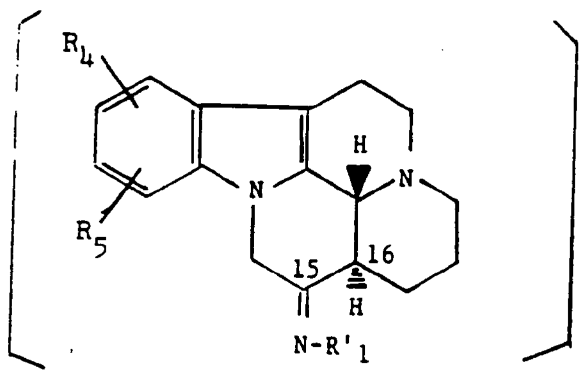

(IV)

which is reduced in order to obtain a compound of formula ($I_{A1}$):

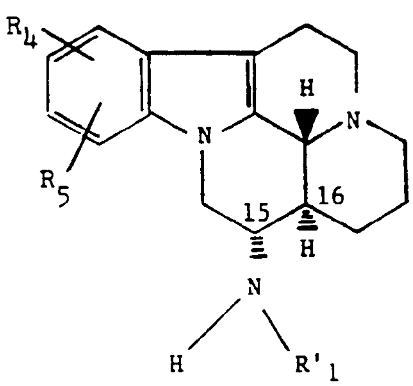

($I_{A1}$)

in which $R'_1$, $R_4$ and $R_5$ have the meaning already indicated,

- or to the action of a heterocyclic amine of formula ($III_B$):

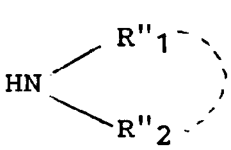

($III_B$)

in which $R''_1$ and $R''_2$ form together with the nitrogen atom to which they are linked the same heterocyclic amines as those given previously for $R_1$ and $R_2$, in order to obtain a compound of formula (V):

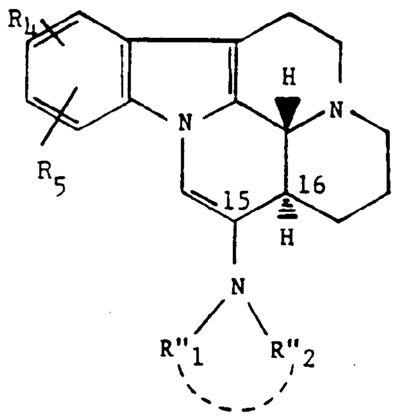

(V)

which is reduced in order to obtain a compound of formula ($I_{A2}$):

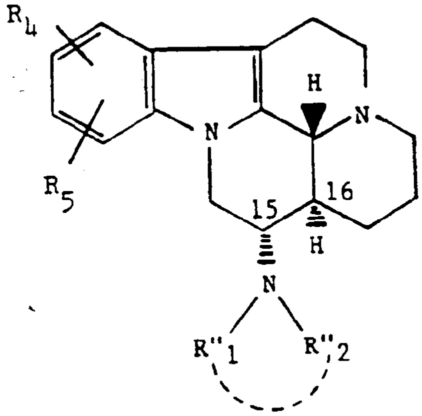

($I_{A2}$)

in which R″$_1$, R″$_2$, $R_4$ and $R_5$ have the values given previously,
- or to the action of a reducing agent in order to obtain a compound of formula (VI):

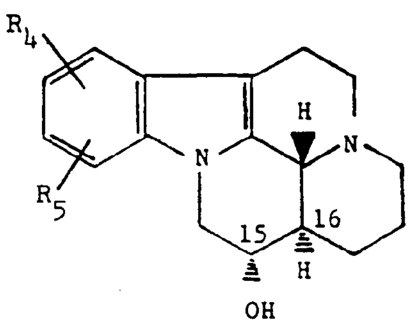

(VI)

the hydroxyl function of which is activated and which is subjected
- either to the action of an aliphatic amine ($III_A$):

$H_2N$-R′$_1$ ($III_A$)

R′$_1$ having the previous meaning, in order to obtain a compound of formula ($I_{B1}$):

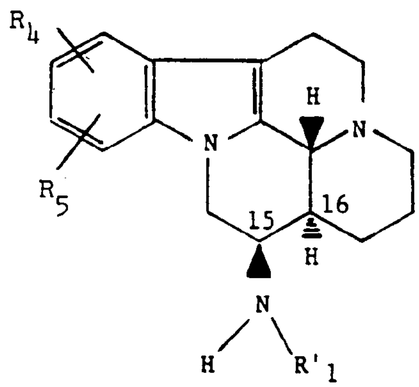

$(I_{B1})$

$R'_1$, $R_4$ and $R_5$ having the previous meaning,
- or to the action of a heterocyclic amine of formula $(III_B)$:

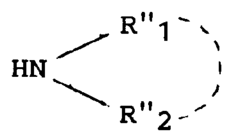

$(III_B)$

$R''_1$ and $R''_2$ having the previous meanings, in order to obtain a compound of formula $(I_{B2})$:

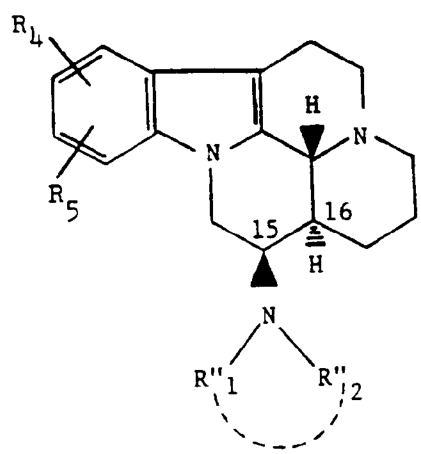

$(I_{B2})$

in which $R''_1$, $R''_2$, $R_4$ and $R_5$ have the meanings already indicated and that, if desired, the compounds of formulae $(I_{A1})$ and $(I_{B1})$ are subjected to the action of a reagent which allows the grafting of an $R'_2$ radical having the same values as given previously for $R_2$, except for hydrogen, onto the amine in position 15, in order to obtain the compounds of formulae $(I_{A3})$ and $(I_{B3})$:

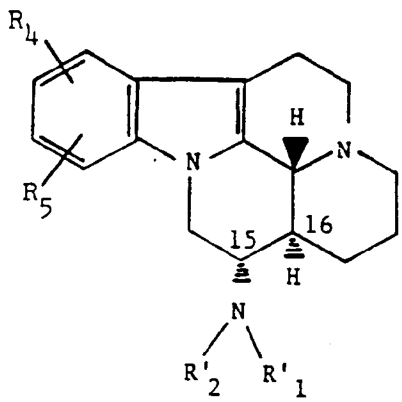

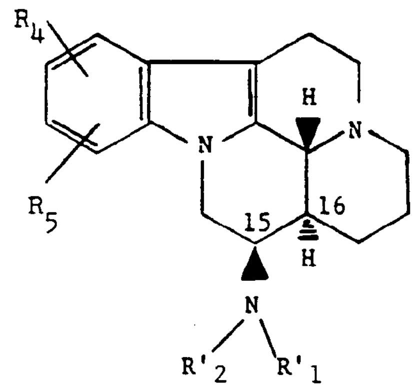

($I_{A3}$) ($I_{B3}$)

in which $R'_1$, $R'_2$, $R_4$ and $R_5$ have the previous meanings and that, if desired, in the case where the $R'_2$ radical to be introduced is a methyl radical, the compounds of formulae ($I_{A1}$) and ($I_{B1}$) are subjected to the action of a formate of formula:

$$HCO_2R$$

in which R is an alkyl radical containing 1 to 4 carbon atoms in order to obtain a compound of formula (VII):

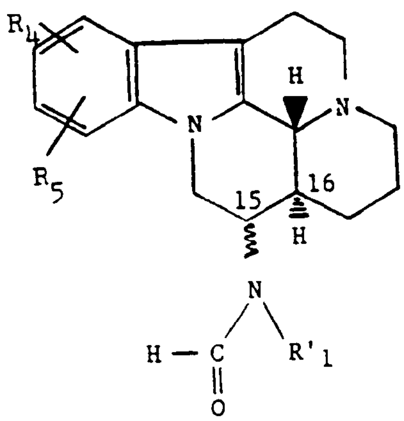

(VIII)

which is reduced in order to obtain the compounds of formulae ($I_{A3}$) and ($I_{B3}$) in which the $R'_2$ radical is a methyl radical and that, if desired, the compounds of formulae ($I_{A1}$) and ($I_{B1}$) for which $R'_1$ is an optionally substituted arylalkyl radical are reduced in order to obtain the compounds of formulae ($I_{A4}$) and ($I_{B4}$):

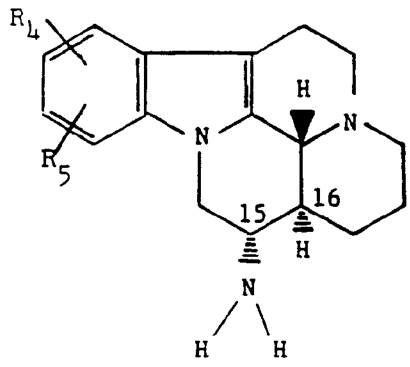 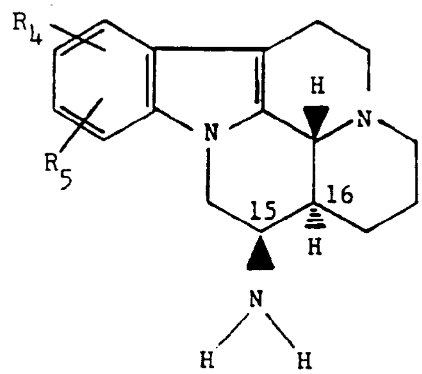

($I_{A4}$) ($I_{B4}$)

in which $R_4$ and $R_5$ have the meaning already indicated, and that, if desired, the compounds of formulae ($I_{A4}$) and ($I_{B4}$) are subjected,

- either to the action of a reagent which allows the $R'_1$ and $R'_2$ radicals to be grafted in order to obtain the compounds of formulae ($I_{A1}$), ($I_{B1}$), ($I_{A3}$) or ($I_{B3}$),
- or to the action of an $HCO_2R$ formate in order to obtain a compound of formula (VIII):

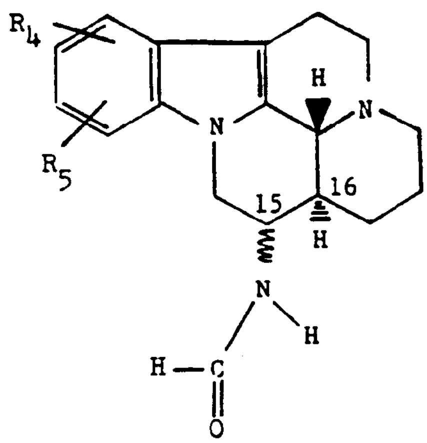

(VIII)

which is reduced in order to obtain a compound of formulae ($I_{A1}$) and ($I_{B1}$) in which the $R'_1$ radical is a methyl radical and, if desired, all the products of formula (I) thus obtained are treated with a mineral or organic acid, in order to form the salts.

2. Process according to claim 1, characterized in that the starting product of formula ($III_A$) or ($III_B$) and the reagents which are reacted on the compounds of formulae ($I_{A1}$), ($I_{B1}$), ($I_{A4}$), ($I_{B4}$) are such that a product of formula (I) is obtained in which $R_1$ and $R_2$, identical or different, represent a methyl or ethyl radical, a

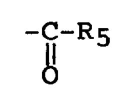

radical in which $R_5$ is a methyl or ethyl, phenyl, benzyl radical optionally substituted by one or more chlorine atoms, a $-CH_2-CH_2-OH$ radical or $R_1$ and $R_2$ form with the nitrogen atom a pyrrolidinyl, morpholinyl or piperazinyl radical the nitrogen atom of which is substituted by a methyl or phenyl radical, $R_4$ and $R_5$, identical or different, represent a hydrogen atom or $R_4$ represents a nitro radical in position 9 or 11 and $R_5$ represents a hydrogen atom.

3. Process according to claim 1, characterized in that the following are prepared:

[(+) (15alpha, 16alpha)]-14,15-dihydro-N-methyl-20,21-dinoreburnamenin-15-amine,
[(±) (15alpha, 16alpha)]-14,15-dihydro-20,21-dinoreburnamenin-15-amine
[(±) (15alpha, 16alpha)]-2-[14,15-dihydro-20,21-dinoreburnamenin-15-yl)-methylamino]-ethanol
and their addition salts with acids.

**4.** Preparation process for pharmaceutical compositions characterized in that at least one of the compounds of formula (I) as defined in claim 1 or at least one of their addition salts with pharmaceutically acceptable mineral or organic acids is used as active ingredient, in a form intended for this use.

**5.** Process according to claim 4, characterized in that the compound of formula (I) is chosen from:
[(±) (15alpha, 16alpha)]-14,15-dihydro-N-methyl-20,21-dinoreburnamenin-15-amine,
[(+) (15alpha, 16alpha)]-14,15-dihydro-20,21-dinoreburnamenin-15-amine
[(±) (15alpha, 16alpha)]-2-[14,15-dihydro-20,21-dinoreburnamenin-15-yl)-methylamino]-ethanol
and their addition salts with acids.

**6.** As new industrial products, the compounds of formulae (IV), (V), (VII), (VIII) and those of formulae (II) and (VI) in which $R_4$ and $R_5$ do not each represent a hydrogen atom.